(19) Europäisches Patentamt
European Patent Office
Office européen des brevets

(11) EP 1 169 470 B1

(12) EUROPEAN PATENT SPECIFICATION

(45) Date of publication and mention of the grant of the patent: **23.06.2004 Bulletin 2004/26**

(21) Application number: **00921974.2**

(22) Date of filing: **10.04.2000**

(51) Int Cl.[7]: **C12Q 1/00**, C12Q 1/37, C12Q 1/42, A61K 38/00, A61K 38/55

(86) International application number: **PCT/US2000/009497**

(87) International publication number: **WO 2000/061789 (19.10.2000 Gazette 2000/42)**

# (54) METHODS AND REAGENTS FOR DETERMINING ENZYME SUBSTRATE SPECIFICITY, AND USES RELATED THERETO

METHODEN UND REAGENTIEN ZUR BESTMMUNG DER ENZYM-SUBSTATSPEZIFITÄT UND DAMIT VERBUNDENE ANWENDUNGEN

PROCEDES ET REACTIFS SERVANT A DETERMINER LA SPECIFICITE DE SUBSTRATS ENZYMATIQUES ET UTILISATIONS CORRESPONDANTES

(84) Designated Contracting States:
**AT BE CH CY DE DK ES FI FR GB GR IE IT LI LU MC NL PT SE**

(30) Priority: **09.04.1999 US 128471 P**

(43) Date of publication of application: **09.01.2002 Bulletin 2002/02**

(73) Proprietor: **TRUSTEES OF TUFTS COLLEGE**
**Boston, MA 02111 (US)**

(72) Inventor: **BACHOVCHIN, William**
**Melrose, MA 02176 (US)**

(74) Representative: **Horner, Martin Grenville et al**
**Cruishank & Faiweather,**
**19 Roayl Exchange Square**
**Glasgow G1 3AE (GB)**

(56) References cited:
**WO-A-91/17762**
**WO-A-98/11251**
**US-A- 5 571 681**

- **CHEMICAL ABSTRACTS, vol. 129, no. 7, 17 August 1998 (1998-08-17) Columbus, Ohio, US; abstract no. 78349x, YIALLOUROS I ET AL: "Phosphinic peptides, the first potent inhibitors of astacin, behave as extremely slow-binding inhibitors" page 316; column 1; XP002901149 & BIOCHEM J, vol. 331, no. 2, 1998, pages 375-379,**
- **TSILIKOUNAS E ET AL: "11B NMR Spectroscopy of Peptide Boronic Acid Inhibitor Complexes of alpha-Lytic Protease. Direct Evidence for Tetrahedral Boron in both Boron-Histidine and Boron-Serine Adduct Complexes" BIOCHEMISTRY, vol. 32, no. 47, 1993, pages 12651-12655, XP002901150**
- **BURBAUM J J ET AL: "A paradigm for drug discovery employing encoded combinatorial libraries" PROC. NATL. ACAD. SCI. USA, vol. 92, no. 13, 20 June 1995 (1995-06-20), pages 6027-6031, XP002901151**

EP 1 169 470 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

## Background of the Invention

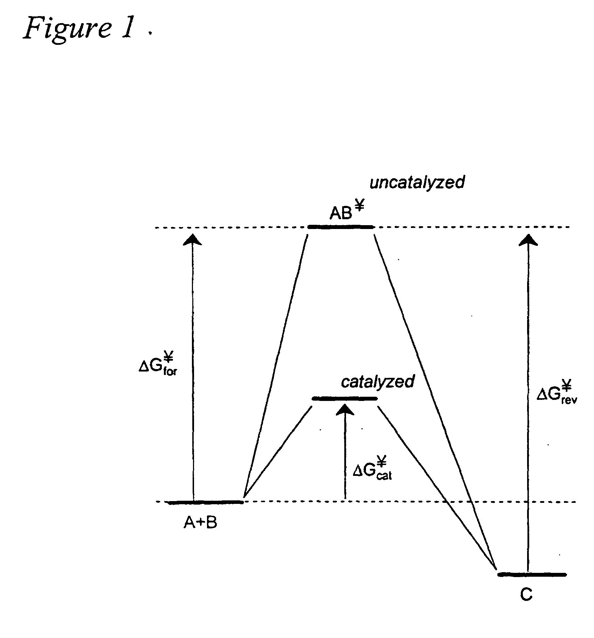

The mechanism by which enzymes catalyze a reaction, such as peptide hydrolysis, is often described in terms of transition state theory. In the application of transition state theory to a chemical reaction, the processes by which the reactants collide are ignored, and only the ground state of the reactants and most unstable species of the reaction pathway, the transition state, are considered. The transition state occurs at the peak in the reaction coordinate diagram. See Figure 1. In the reaction profile, the transition state represents the state of the reactant(s) when the chemical bonds are in the process of being broken or formed. From the difference in energies between the transition state and the ground state, the rate of reaction can be derived. The enhancement in rate achieved by enzyme catalysis of a reaction, relative to the spontaneous reaction rate, results from the ability of the enzyme to decrease the activation energy of the reaction

In this view, an enzyme is essentially a flexible molecular template, designed by evolution to be precisely complementary to the reactants in their activated transition-state geometry, as distinct from their ground-state geometry. Thus an enzyme strongly binds the transition state, greatly increasing its concentration and accelerating the reaction proportionately. This description of enzymic catalysis is now usually referred to as transition-state stabilization.

However, in its original form, the transition-state binding model did not provide an answer to an apparent paradox: many enzymes exhibit greatly increased activity toward extended substrates, for example, substrates with larger amino acid side chains, or with additional amino acid residues or sugar residues on either side of and at some distance from the peptide bond or glycosidic bond to be hydrolysed, as compared with smaller substrates. The puzzle was this: increased activity is usually manifested more by an increase in $k_{cat}$ than by a decrease in $K_m$, that is, by an increase in maximum rate rather than by an increase in substrate binding (as substrate binding affinity increases, $K_m$ tends to decrease). Consideration of the common occurrence of this phenomenon led to introduction of the "induced fit" concept, wherein distal portions of the enzyme contribute substantially to overall binding of the transition state but not of the ground state.

Recently, the Applicant and others have provided evidence of a modified induced fit mechanism wherein optimum binding of the transition state is a cooperative phenomenon. That is, the numerous individual binding interactions between transition state and enzyme are synergistic. The interaction of the specificity subsites of the enzyme with the substrate induces a conformational change in the active site, the new conformation being more suitable for stabilization of the transition state intermediate(s). This cooperativity can be seen as a strategy for amplifying enzyme specificity; a small perturbation in the chemical structure of a substrate can then cause a large decrease in binding of its transition state. See, for example, Tsilikounas et al. (1993) Biochemistry 32:12651; Tsilikounas et al. (1992) Biochemistry 31:12839; Bone et al. (1989) Biochemistry 28:7600; Farr-Jones et al. (1989) PNAS 86:6922; Smith et al. (1989) Science 244:961; Kettner et al. (1988) Biochemistry 27:7682; Bachovchin et al. (1988). Biochemistry 27:7689; and Bachovchin (1986) Biochemistry 25:7751.

WO9811251 discloses a method for identifying a substrate for a ligand-removing enzyme. In the disclosed method,a ligand capable of being removed by the ligand-removing enzyme is incorporated into a partially degenerate peptide library at a non-degenerate position. The peptide library is then treated with the ligand-removing enzyme to generate a mixture of peptides wherein the ligand is intact, and peptides wherein the ligand cleaved. The mixture is then treated with a proteolytic enzyme that preferentially cleaves ligand-free peptides in the mixture. These ligand-free peptides are then sequenced to identify optimum substrates for the ligand-removing enzyme.

WO 9117762 application discloses a peptide inhibitor of retroviral proteinase which is similar to the natural substrate of the proteinase (i.e., not degenerated) and is represented by the formula: $(Z)_nX\text{-}Y(Z)_m$ wherein X is any naturally occurring amino acid (such as tyrosine or phenylalanine) or modified amino acid; Y is an alpha-imino acid having a 4-, 6-, 7- or 8-membered ring structure; Z is an alpha-amino acid; and n and m are independently 0 to 7. Y can be further substituted by with functional groups which transforms the peptide into a tight-binding inhibitor or a covalent inhibitor. The application does not disclose libraries of said peptides, nor does it disclose screening for retroviral proteinase activity using a library of said peptides.

## Summary of the Invention

The invention provides a method for determining a substrate, such as a preferred amino acid sequence motif, which is preferentially bound by an enzyme active site and associated specificity subsites. Such a sequence motif can be derived by allowing an oriented degenerate library of peptides, peptide analogs, peptidomimetics, or other small molecules to interact with a given enzyme, determing which members of the library are preferred by the enzyme, and sequencing the preferred members to determine the preferences of the various specificity subsites of the enzyme. Exemplary enzymes considered for use in the present invention include proteases, phosphatases, and kinases, among

others.

## Description of the Drawings

Figure 1 illustrates a reaction coordinate scheme.

## Detailed Description of the Invention

*(A) Overview*

One aspect of the present invention pertains to methods and reagents for determining the substrate specificity of enzymes involved in protein/peptide processing, such as proteases, kinases, phosphatases and the like. In general, the invention provides a method that allows for the identification of an amino acid sequence motif defining the optimum substrate specificity of a specific enzyme without having to identify, isolate and compare native substrates of that enzyme.

The method of the invention is based upon selection of a particular peptidyl inhibitor(s) from a degenerate library of inhibitors. In particular, the subject assay utilizes libraries of inhibitors which each individually include: (i) a peptidyl portion which is degenerate in sequence, e.g., to provide variegation to the inhibitor library; and (ii) a functional group which can form a covalent adduct, or other tightly bound complex, with the enzyme in a manner or with an affinity which is dependent on the ability of the peptidyl portion of the inhibitor to interact with certain specificity subsites of the enzyme. A moiety bearing such a functional group is henceforth referred to as an active subunit. In preferred embodiments, the active subunit will be chosen such that the members of the library are slow-binding inhibitors of the enzyme. The inhibitor library is contacted with a target enzyme under conditions wherein enzyme-inhibitor complexes can form. Those members of the library which form tight complexes or covalent bonds with the enzyme are identified and/or isolated from the library. That is, the assay selects for inhibitors which cause a conformational change in the active site of the enzyme in a manner dependent upon the sequence of the peptidyl portion of the inhibitor. In preferred embodiments, the selected inhibitors have dissociation constants ($k_d$) of less than $10^{-6}$ M, and more preferably less than $10^{-7}$ M, $10^{-8}$ M, or even $10^{-9}$ M.

As described below, and by way of illustration, it has been shown that certain peptidyl boronic acids exhibit slow-binding inhibition kinetics for serine proteinases, among others, as defined by Morrison et al. (1988) Adv. Enzymol. Relat. Areas Mol. Biol. 61:202. This tendency correlates with whether or not the inhibitor is a substrate analog. So-called "good" substrate analogs, e.g.. those inhibitors whose peptidyl portion is complementary to the specificity subsites of the enzyme, tend to exhibit slow-binding kinetics. Moreover, these boronic acid inhibitors formed tetrahedral adducts with the active site serine, as would be expected based on a tetrahedral boronic acid-serine adduct being a good transition state analog of a tetrahedral amide-serine complex, the native substrate for the protease. However, simple alkyl and aryl boronic acids, for example, which would be considered "poor" substrate analogs in that they do not fill the specificity subsites, can also form covalent adducts with the protease. These adducts, e.g., with the active site histidine, are in fact distinct from the type of adducts formed by the good substrate analogs. That is, the mere presence of the transition state analog is not alone sufficient to engage the active site in a manner similar to the native substrate.

Additionally, it has been observed that many enzymes exhibit greatly increased activity toward extended substrates as compared with smaller substrates, manifested more by an increase in maximum rate ($k_{cat}$) rather than by an increase in substrate binding ($K_m$).

These observations and others have led the Applicant to propose an induced-distortion model for catalysis. See, for example, Bachovchin et al. (1988) Biochemistry 27:7689. In this model, the "induced-fit" concept - wherein distal portions of the enzyme contribute substantially to overall binding of the transition state but not of the ground state - is modified to recognize a synergism between the catalytic site of the enzyme and the specificity subsites. Briefly, rather than a mere lock-and-key fit, occupancy of the specificity subsites, e.g., by favorable contact with the peptide, induces the enzyme to adopt a structure around the catalytic site that is more complementary to the transition state. In this modified induced-fit model of enzyme-substrate binding, the shape of the active site of the unbound enzyme is not the exact complement of the substrate. After binding of the enzyme to the substrate is initiated, a conformational change in the shape of the active site results. Thus, contact between the peptide substrate and specificity subsites of the enzyme which are distal to the active site may nevertheless have profound effects on the catalytic activity of the enzyme towards the substrate.

For enzymes which operate via a mechanism requiring synergism between specificity subsites and the active site, the binding assays of the art which detect binding of individual peptides from a peptide library (see, for example,

Ladner, et al. U.S. Patent No. 5,223,409 and Roberts, et al. (1992) Gene 121:9-15) may miss optimal substrates. Because the synergism between the active site and the specificity subsites is largely accounted for by an increase in $K_{cat}$ rather than a decrease in $K_m$, a peptide library which does not engage the active site may not discern such substrate binding requirements, though these interactions can be critical to substrate and inhibitor design. The method of the present invention, on the other hand, is designed to determine the optimal substrate specificity by accounting for such synergism.

Slow-binding kinetics is a phenomenon which is not limited to proteases. Such kinetic behavior, requiring contact with a peptide substrate and induction of conformational changes to the active site, has been observed in a wide range of enzymes which modify proteins and other natural polymers such as nucleic acids and carbohydrates. Thus, it is specifically contemplated that the subject method can also be used to determine the substrate specificity of such enzymes as protein kinases and protein phosphatases.

In the method, the inhibitor library is contacted with the enzyme under conditions wherein adducts involving the enzyme can form if appropriate specificity subsites of the enzyme are productively filled. Those bound inhibitors having at least some threshold $K_i$ are separated from the unbound inhibitors, and from the bound inhibitors which do not have the minimum potency, thereby isolating the subpopulation of inhibitors having peptide sequences which are optimal substrates for the enzyme.

In certain embodiments, the inhibitor library is comprised of peptidyl inhibitors each represented in the general formula:

$$(Xaa)_n\text{-}Yaa\text{-}(Xaa)_m$$

wherein

Yaa is an active subunit capable of interacting with a portion of an active site of an enzyme,
Xaa is any amino acid or amino acid analog, and
n and m are integers from 0-10 inclusive, such that the sum of m and n is at least two, preferably at least four.

The library is degenerate at one or more amino acid positions to yield a variegated inhibitor library.

Yaa can be, for example, a transition state analog of a peptide backbone bond, or an amino acid moiety having a transition state analog group attached to the sidechain thereof. The transition state analog can be a functional group which, in the context of an appropriate interaction between the peptidyl portion of the inhibitor and the specificity subsites of the target enzyme, can form a covalent adduct or other tightly associated complex with the enzyme.

In preferred embodiments, the peptidyl portion of the library is 2-50 residues in length, more preferably 4-20, and even more preferably 4-10 residues in length. For instance, the peptidyl portion can be 4, 5, 6, 7, or 8 amino acid residues in length.

In certain embodiments, the inhibitors of the library are represented in one of the following formulas:

$$\text{(I)} \quad Xaa_1 - [-Xaa_1-]_n\, Xaa_1 - Yaa$$

$$\text{(II)} \quad Yaa - Xaa_2 - [-Xaa_2-]_m\, Xaa_2$$

$$\text{(III)} \quad Xaa_1 - [-Xaa_1-]_n\, Xaa_1 - Yaa - Xaa_2 - [-Xaa_2-]_m\, Xaa_2$$

wherein,

Yaa is as defined above, and

$Xaa_1$ and $Xaa_2$, independently for each occurence, represent an amino acid moiety or amino acid analog, and

n and m are each, independently, an integer from 1 to 10, such that the sum of m and n is at least two, preferably at least four.

After isolation of the subpopulation of inhibitors, the inhibitors are then sequenced and the relative abundance of each amino acid residue at each degenerate position of the peptides is determined. An amino acid sequence motif representing the enzyme's optimal substrate specificity can be determined from the most abundant amino acid residues at each degenerate position of the peptides. This method has the advantage that it can be used to determine a substrate specificity for any enzyme which alters proteins and peptides, regardless of whether native substrates for that enzyme have been identified.

In certain embodiments, the consensus sequence(s) identified for a given enzyme can be used to develop selective inhibitors of the enzyme, including peptide, peptidomimetic, and other small molecule inhibitors. Such inhibitors can be used *in vitro*, e.g., as cell culture additives or in other industrial processes, or *in vivo*, such as for human or animal therapy.

In other embodiments, based upon the amino acid sequence motif determined by the method of the invention, peptides can be made which are substrates for that particular enzyme, e.g., a particular protease. Peptides comprising the most preferred amino acid residues of a sequence motif represent optimal substrates for the enzyme. For example, as described below, the subject substrates can be used in research and diagnostic applications, such as to detect and quantitate a particular enzyme. In certain instances, the substrate may be used as a surrogate for the native substrate, particularly where the native substrate is not known, in assays generated for identifying, for example, small molecule inhibitors of the enzyme.

*(B) Definitions*

For convience, certain terms employed in the specfication, examples, and appended claims are collected here.

The International Union of Biochemistry and Molecular Biology (1984) has recommended to use the term "peptidase" for the subset of peptide bond hydrolases (Subclass E.C 3.4.). The widely used term protease is synonymous with peptidase. Peptidases comprise two groups of enzymes: the endopeptidases and the exopeptidases. Endopeptidases cleave peptide bonds at points within a protein, and exopeptidases remove amino acids sequentially from either the N- or C-terminus.

The term "proteinase" is also used as a synonym for endopeptidase. Proteinases are classified according to their catalytic mechanisms. Four mechanistic classes have been recognized by the International Union of Biochemistry and Molecular Biology: serine proteinases, cysteine proteinases, aspartic proteinases, and metalloproteinases.

This classification by catalytic types has been suggested to be extended by a classification by families based on the evolutionary relationships of proteases (Rawlings, N.D. and Barrett, A.J., (1993), Biochem. J., 290, 205-218). This classification is available in the SwissProt database.

In addition to these four mechanistic classes, there is a section of the enzyme nomenclature which is allocated for proteases of unidentified catalytic mechanism. This indicates that the catalytic mechanism has not been identified, and the possibility remains that novel types of proteases do exist.

The class "serine proteinases" comprises two distinct families: the chymotrypsin family which includes the mammalian enzymes such as chymotrypsin, trypsin or elastase or kallikrein, and the substilisin family which includes the bacterial enzymes such as subtilisin. The general three-dimensional structure is different in the two families but they have the same active site geometry and catalysis proceeds via the same mechanism. The serine proteinases exhibit different substrate specificities which are related to amino acid substitutions in the various enzyme subsites (see the nomenclature of Schechter and Berger) interacting with the substrate residues. Three residues which form the catalytic triad are essential in the catalytic process: His-57, Asp-102 and Ser-195 (chymotrypsinogen numbering).

The family of "cysteine proteinases" includes the plant proteases such as papain, actinidin or bromelain, several mammalian lysosomal cathepsins, the cytosolic calpains (calcium-activated), and several parasitic proteases (e.g., Trypanosoma, Schistosoma). Papain is the archetype and the best studied member of the family. Like the serine proteinases, catalysis proceeds through the formation of a covalent intermediate and involves a cysteine and a histidine residue. The essential Cys-25 and His-159 (papain numbering) play the same role as Ser-195 and His-57 respectively. The nucleophile is a thiolate ion rather than a hydroxyl group. The thiolate ion is stabilized through the formation of an ion pair with neighboring imidazolium group of His-159. The attacking nucleophile is the thiolate-imidazolium ion pair in both steps and then a water molecule is not required.

Most of the "aspartic proteinases" belong to the pepsin family. The pepsin family includes digestive enzymes

such as pepsin and chymosin as well as lysosomal cathepsins D, processing enzymes such as renin, and certain fungal proteases (penicillopepsin, rhizopuspepsin, endothiapepsin). A second family comprises viral proteinases such as the protease from the AIDS virus (HIV) also called retropepsin. In contrast to serine and cysteine proteinases, catalysis by aspartic proteinases does not involve a covalent intermediate, though a tetrahedral intermediate exists. The nucleophilic attack is achieved by two simultaneous proton transfers: one from a water molecule to the diad of the two carboxyl groups and a second one from the diad to the carbonyl oxygen of the substrate with the concurrent CO-NH bond cleavage. This general acid-base catalysis, which may be called a "push-pull" mechanism leads to the formation of a non-covalent neutral tetrahedral intermediate.

The "metalloproteinases" are found in bacteria, fungi as well as in higher organisms. They differ widely in their sequences and their structures but the great majority of enzymes contain a zinc atom which is catalytically active. In some cases, zinc may be replaced by another metal such as cobalt or nickel without loss of the activity. Bacterial thermolysin has been well characterized and its crystallographic structure indicates that zinc is bound by two histidines and one glutamic acid. Many enzymes contain the sequence HEXXH, which provides two histidine ligands for the zinc whereas the third ligand is either a glutamic acid (thermolysin, neprilysin, alanyl aminopeptidase) or a histidine (astacin). Other families exhibit a distinct mode of binding of the Zn atom. The catalytic mechanism leads to the formation of a non-covalent tetrahedral intermediate after the attack of a zinc-bound water molecule on the carbonyl group of the scissile bond. This intermediate is further decomposed by transfer of the glutamic acid proton to the leaving group.

In discussing the interactions of peptides with proteinases, e.g., serine and cysteine proteinases and the like, the present application utilizes the nomenclature of Schechter and Berger [(1967) Biochem. Biophys. Res. Commun. 27:157-162)]. The individual amino acid residues of a substrate or inhibitor are designated P1, P2, etc. and the corresponding subsites of the enzyme are designated S1, S2, etc. The scissile bond of the substrate is S1-S1'.

The binding site for a peptide substrate consists of a series of "specificity subsites" across the surface of the enzyme. The term "specificity subsite" refers to a pocket or other site on the enzyme capable of interacting with a portion of a substrate for the enzyme.

The term "substrate" refers to a substrate of an enzyme which is catalytically acted on and chemically converted by the enzyme to product(s).

The term "oxyanion hole" refers to one or more amino acid residues of an enzyme which stabilize an oxyanion species of a transition state intermediate. To illustrate, x-ray crystal structures and chemical data supports the existence of a tetrahedral transition-state intermediate in the catalytic mechanism of chymotrypsin. This is formed by the attack of the hydroxyl group of Ser-195 on the carbonyl carbon atom of the scissile bond. The C=O bond becomes a single bond, leaving a negative charge on the O atom (an oxyanion), while the fourth valency of the carbon atom is occupied by a bond with the serine $O\gamma$. The oxyanion forms hydrogen bonds to two main chain amides, of residues 193 (Gly) and 195 (Ser). This binding site in chymotrypsin is an example of an oxyanion hole.

The term "peptide" refers to an oligomer in which the monomers are amino acids (usually alpha-amino acids) joined together through amide bonds. Peptides are two or more amino acid monomers long, but more often are between 5 to 10 amino acid monomers long and can be even longer, i.e., up to 20 amino acids or more, and peptides longer than 20 amino acids are contemplated.

The term "protein" is well known in the art and usually refers to a very large polypeptide, or set of associated homologous or heterologous polypeptides, that has some biological function. For purposes of the present invention the terms "polypeptide" and "protein" are largely interchangeable.

The term "random peptide library" refers to a set of random or semi-random peptides, as well as sets of fusion proteins containing those random peptides (as applicable).

The term "synthetic" refers to production by *in vitro* chemical or enzymatic synthesis.

The phrases "individually selective manner" and "individually selective binding", with respect to binding of a test inhibitor with a target enzyme, refers to binding which is specific for, and dependent on, the molecular identity of the protein enzyme and the peptidyl portion of the inhibitor.

The terms "D-amino acid" and "L-amino acid" each denote an absolute configuration by convention relative to the possible stereoisomers of glyceraldehyde. Thus, all stereoisomers that are stereochemically related to L-glyceraldehyde are designated L-, and those related to D-glyceraldehyde are designated D-, regardless of the direction of the rotation of plane of polarized light by the given isomer. In the case of threonine and isoleucine, there are two stereochemical centers, i.e., the $C\alpha$ and the $C\beta$ atoms. The D-threonine and D-isoleucine employed herein preferably have stereochemistries at both chiral sites which are opposite (enantiomeric) to the stereochemistry of the L-enantiomers of those amino acids, e.g., they are complete mirror images. Glycine is the only commonly occurring achiral amino acid. Accordingly, when a peptide is designated herein as a D- or L- enantiomer, it is meant that essentially all of the chiral amino acid residues comprising such peptide have the indicated chirality. The presence of achiral amino acid residues such as glycine do not affect the designation of its chirality.

All chiral amino acids in protein found in nature, e.g., "naturally occurring", are L-amino acids.

The term "stereoisomers" refers to compounds which have identical chemical constitution, but differ with

regard to the arrangement of the atoms or groups in space. In particular, "enantiomers" refer to two stereoisomers of a compound which are non-superimposable mirror images of one another. "Diastereomers", on the other hand, refers to stereoisomers with two or more centers of asymmetry and whose molecules are not mirror images of one another. With respect to the nomenclature of a chiral center, terms "D" and "L" configuration are as defined by the IUPAC Recommendations. As to the use of the terms, diastereomer, racemate, and enantiomer will be used in their normal context to describe the stereochemistry of peptide preparations.

Similarly, the terms "enantiomerically enriched" and "non-racemic", as used interchangeably herein with reference to preparations of a molecule, refers to preparations of a chiral compound which substantially lacks one of the enantiomers. For instance, with reference to peptides, enantiomerically enriched refers to a preparation in which the L- or D-enantiomer sidechains are enriched at a given position in the peptide.

The term "degenerate peptide library" is intended to describe populations of peptides in which different amino acid residues are present at the same position in different peptides within the library. For example, a population of peptides of 10 amino acids in length in which the amino acid residue at position 5 of the peptides can be any one of the twenty amino acids would be a degenerate peptide library. A position within the peptides which is occupied by different amino acids in different peptides is referred to herein as a "degenerate position". A position within the peptides which is occupied by the same amino acid in different peptides is referred to herein as a "non-degenerate position". The "oriented degenerate peptide library" used in the method of the invention is composed of peptides which have a functional group which can form an adduct with a target enzyme at a fixed, non-degenerate position. This means that the peptides contained within the library all have the same functional group at the same position within the peptides.

As used herein, "variegated" refers to the fact that a population of peptides is characterized by having a peptide sequence which differ from one member of the library to the next. For example, in a given peptide library of n amino acids in length, the total number of different peptide sequences in the library is given by the product of $\nu_1$ x $\nu_2$ x ..... $\nu_{n-1}$ x $\nu_n$ where each $\nu_n$ represents the number different amino acid residues occurring at position n of the peptide. In a preferred embodiment of the present invention, the peptide display collectively produces a peptide library including at least 96 to $10^7$ different peptides, so that diverse peptides may be simultaneously assayed for the ability to interact with the target protein.

The term "alkyl" is recognized in the art and refers to saturated aliphatic groups having one to ten carbon atoms, including straight-chain alkyl groups, branched-chain alkyl groups, cycloalkyl (alicyclic) groups, alkyl substituted cycloalkyl groups, and cycloalkyl substituted alkyl groups. Such hydrocarbon moieties may be substituted on one or more carbons with, for example, a halogen, a hydroxyl, a thiol, an amino, or a nitro group. In the instance of the cycloalkyls, such substituents can further comprise an alkyl, an alkenyl, an alkoxy, an alkylthio, an alkylamino, an alkylcarboxyl, a nitro, a hydroxyl, $-CF_3$, -CN, or the like. Unless the number of carbons is otherwise specified, "lower alkyl" as used herein means an alkyl group, as defined above, but having from one to six carbon atoms, rather than from one to ten carbon atoms. Likewise, "lower alkenyl" and "lower alkynyl" have similar chain lengths.

Representative of such alkyl groups are methyl, ethyl, n-propyl, isopropyl, 2-chloropropyl, n-butyl, sec-butyl, 2-aminobutyl, isobutyl, tert-butyl, 3-thiopentyl, and the like. Other exemplary "alkyls" inlcude $CH_2Cl$, $CH_2F$, $CHF_2$, and $CF_3$. As used herein, the term "amino" means $-NH_2$; the term "nitro" means $-NO_2$; the terms "halogen" and "halo" designate - F, -Cl, -Br or -I; the term "thiol" means SH; and the term "hydroxyl" means -OH. Thus, the term "alkylamino" as used herein means an alkyl group, as defined above, having an amino group attached thereto. The term "alkylthio" refers to an alkyl group, as defined above, having a sulphydryl group attached thereto. The term "alkylcarboxyl" as used herein means an alkyl group, as defined above, having a carboxyl group attached thereto. The term "alkoxy" as used herein means an alkyl group, as defined above, having an oxygen atom attached thereto. Representative alkoxy groups include methoxy, ethoxy, propoxy, tert-butoxy and the like.

The terms "alkenyl" and "alkynyl" refer to unsaturated aliphatic groups analogous to alkyls, but which contain at least one double or triple bond respectively.

The term "aryl" as used herein includes 4-, 5- and 6-membered single-ring aromatic groups which may include from zero to four heteroatoms, for example, benzene, pyrrole, furan, thiophene, imidazole, oxazole, thiazole, triazole, pyrazole, pyrrolidine, pyridine, pyrazine, pyridazine and pyrimidine, and the like. The aromatic ring may be substituted at one or more ring positions with, for example, a halogen, a lower alkyl, a lower alkenyl, a lower alkoxy, a lower alkylthio, a lower alkylamino, a lower alkylcarboxyl, a nitro, a hydroxyl,$-CF_3$, -CN, or the like.

The term "heterocycle" or "heterocyclic group" refers to 4-, 5- and 6- membered single-ring alicyclic groups which include one to four heteroatoms.-Heterocyclic groups include pyrrolidine, oxolane, thiolane, imidazole, oxazole, piperidine, piperazine, morphaline. The alicyclic ring can be substituted at one or more ring positions with, for example, a halogen, a lower alkyl, a lower alkenyl, a lower alkoxy, a lower alkylthio, a lower alkylamino, a lower alkylcarboxyl, a nitro, a hydroxyl, $-CF_3$, -CN, or the like.

The term "heteroatom" as used herein means an atom of any element other than carbon or hydrogen. Preferred heteroatoms are nitrogen, oxygen, sulfur, and selenium.

By the terms "amino acid residue" and "peptide residue" is meant an amino acid or peptide molecule without

the -OH of its carboxyl group. In general the abbreviations used herein for designating the amino acids and the protective groups are based on recommendations of the IUPAC-IUB Commission on Biochemical Nomenclature (see Biochemistry (1972) 11:1726-1732). For instance Met, Ile, Leu, Ala and Gly represent "residues" of methionine, isoleucine, leucine, alanine and glycine, respectively. By the residue is meant a radical derived from the corresponding-$\alpha$-amino acid by eliminating the OH portion of the carboxyl group and the H portion of the $\alpha$-amino group. The term "amino acid side chain" is that part of an amino acid exclusive of the $-CH(NH_2)COOH$ portion, as defined by K. D. Kopple, "Peptides and Amino Acids", W. A. Benjamin Inc., New York and Amsterdam, 1966, pages 2 and 33; examples of such side chains of the common amino acids are $-CH_2CH_2SCH_3$ (the side chain of methionine), $-CH_2(CH_3)-CH_2CH_3$ (the side chain of isoleucine), $-CH_2CH(CH_3)_2$ (the side chain of leucine), or H-(the side chain of glycine).

For the most part, the amino acids used in the application of this invention are those naturally occurring amino acids found in proteins, or the naturally occurring anabolic or catabolic products of such amino acids which contain amino and carboxyl groups. Particularly suitable amino acid side chains include side chains selected from those of the following amino acids: glycine, alanine, valine, cysteine, leucine, isoleucine, serine, threonine, methionine, glutamic acid, aspartic acid, glutamine, asparagine, lysine, arginine, proline, histidine, phenylalanine, tyrosine, and tryptophan, and those amino acids and amino acid analogs which have been identified as constituents of peptidylglycan bacterial cell walls.

The term amino acid residue further includes analogs, derivatives and congeners of any specific amino acid referred to herein, as well as C-terminal or N-terminal protected amino acid derivatives (e.g. modified with an N-terminal or C-terminal protecting group). For example, the present invention contemplates the use of amino acid analogs wherein a side chain is lengthened or shortened while still providing a carboxyl, amino or other reactive precursor functional group for cyclization, as well as amino acid analogs having variant side chains with appropriate functional groups. For instance, the subject compound can include an amino acid analog such as, for example, cyanoalanine, canavanine, djenkolic acid, norleucine, 3-phosphoserine, homoserine, dihydroxy-phenylalanine, 5-hydroxytryptophan, 1-methylhistidine, 3-methylhistidine, diaminopimelic acid, omithine, or diaminobutyric acid. Other naturally occurring amino acid metabolites or precursors having side chains which are suitable herein will be recognized by those skilled in the art and are included in the scope of the present invention.

Also included are the (D) and (L) stereoisomers of such amino acids when the structure of the amino acid admits of stereoisomeric forms. The configuration of the amino acids and amino acid residues herein are designated by the appropriate symbols (D), (L) or (DL), furthermore when the configuration is not designated the amino acid or residue can have the configuration (D), (L) or (DL). It will be noted that the structure of some of the compounds of this invention includes asymmetric carbon atoms. It is to be understood accordingly that the isomers arising from such asymmetry are included within the scope of this invention. Such isomers can be obtained in substantially pure form by classical separation techniques and by stereochemically controlled synthesis. For the purposes of this application, unless expressly noted to the contrary, a named amino acid shall be construed to include both the (D) or (L) stereoisomers.

The phrases "protecting group" or "protective group" as used herein means substituents which protect the reactive functional group from undesirable chemical reactions. Examples of such protecting groups include esters of carboxylic acids and boronic acids, ethers of alcohols and acetals and ketals of aldehydes and ketones. For instance, the phrase "N-terminal protecting group" or "amino-protecting group" as used herein refers to various amino-protecting groups which can be employed to protect the N-terminus of an amino acid or peptide against undesirable reactions during synthetic procedures. Examples of suitable groups include acyl protecting groups such as, to illustrate, formyl, dansyl, acetyl, benzoyl, trifluoroacetyl, succinyl and methoxysuccinyl; aromatic urethane protecting groups as, for example, benzyloxycarbonyl (Cbz); and aliphatic urethane protecting groups such as t-butoxycarbonyl (Boc) or 9-fluorenylmethoxycarbonyl (FMOC).

Amino-derivative groups which can be present at the N-terminus of a peptide substrate without departing from .the spirit of this invention include epoxysuccinyl, cholesteryl, aryl, aralkyl and acyl derivatives. Carboxy-derivative groups which can be present at the C-terminus of a peptide substrate without departing from the spirit of this invention include alcohol, aldehyde, epoxysuccinate, acid halide, carbonyl, halomethane, and diazomethane derivatives.

As noted above, certain compounds of the present invention may exist in particular geometric or stereoisomeric forms. The present invention contemplates all such compounds, including cis- and trans-isomers, R- and S-enantiomers, diastereomers, (D)-isomers, (L)-isomers, the racemic mixtures thereof, and other mixtures thereof, as falling within the scope of the invention. Additional asymmetric carbon atoms may be present in a substituent such as an alkyl group. All such isomers, as well as mixtures thereof, are intended to be included in this invention.

If, for instance, a particular enantiomer of a compound of the present invention is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts are formed with an appropriate optically-active acid or base, followed by resolution of the diastereomers thus formed by fractional

crystallization or chromatographic means well known in the art, and subsequent recovery of the pure enantiomers. All of these procedures are well known in the art.

The term "boronic acid" is known in the art and refers to an organic molecule bearing the functionality -B$(OH)_2$. Boronic esters, which are analogs of boronic acids wherein one or both of the hydroxyl residues have been replaced by alkoxy groups, are readily hydrolyzed to liberate the corresponding boronic acid, and are therefore considered to be encompassed by the term boronic acid as used herein.

"Aldehyde" is an art-recognized term referring to an organic molecule bearing the functionality -CHO.

The term "trifluoromethyl ketone" as used herein encompasses any organic compound having the functionality $-C(=O)CF_3$, wherein this functionality is attached to a carbon atom of the molecule.

"Alpha-ketocarboxylic acid derviative" is an art-recognized term including organic compounds having the functionality -C(=O)C(=O)X, wherein this functionality is attached to a carbon atom of the molecule and X represents a substituted or unsubsituted heteroatom, including substituted and unsubstituted oxygen, nitrogen, and sulfur atoms.

The term "halomethyl ketone" includes structures having a group such as $-C(=O)CH_2X$, where X is a halogen as defined above. Structures having more than one halogen (and, correspondingly, fewer than two hydrogens) on this functional group are encompassed by the term "halomethyl ketone". For example, a "trihalomethyl ketone" is a moiety in which the methyl group is substituted by three halogen atoms, e.g., selected from Br, Cl, F, or I, which halogen atoms may be the same or different.

*(C) Exemplary Embodiments*

The method of the present invention can be used to determine the specificity of a variety of different enzymes, e.g., including proteinases, kinases, phosphatases, lipases, etc. The enzymes may be from animals, insects, plants or microbes. In certain preferred embodiments, the subject method is used determine the specificity of a peptidase, e. g., an enzyme which is designates by the International Union of Biochemistry and Molecular Biology (1984) as subclass E.C 3.4.-.-. For example, the subject method can be used to determine the specificity of an aminopeptidase (EC 3.4.11.-), a dipeptidase (EC 3.4.13.-), a dipeptidyl-peptidase or tripeptidyl peptidase (EC 3.4.14.-), a peptidyl-dipeptidase (EC 3.4.15.-), a serine-type carboxypeptidase (EC 3.4.16.-), a metallocarboxypeptidase (EC 3.4.17.-), a cysteine-type carboxypeptidase (EC 3.4.18.-), an omegapeptidase (EC 3.4.19.-), a serine proteinase (EC 3.4.21.-), a cysteine proteinase (EC 3.4.22.-), an aspartic proteinase (EC 3.4.23.-), a metallo proteinase (EC 3.4.24.-), or a proteinase of unknown mechanism (EC 3.4.99.-). Exemplary peptide hydrolyases include:

3.4.11.1 Leucyl aminopeptidase.
3.4.11.2 Membrane alanine aminopeptidase.
3.4.11.3 Cystinyl aminopeptidase.
3.4.11.4 Tripeptide aminopeptidase.
3.4.11.5 Prolyl aminopeptidase.
3.4.11.6 Aminopeptidase B.
3.4.11.7 Glutamyl aminopeptidase.
3.4.11.9 Xaa-Pro aminopeptidase.
3.4.11.10 Bacterial leucyl aminopeptidase.
3.4.11.13 Clostridial aminopeptidase.
3.4.11.14 Cytosol alanyl aminopeptidase.
3.4.11.15 Lysyl aminopeptidase.
3.4.11.16 Xaa-Trp aminopeptidase.
3.4.1.1.17 Tryptophanyl aminopeptidase.
3.4.11.18 Methionyl aminopeptidase.
3.4.11.19 D-stereospecific aminopeptidase.
3.4.11.20 Aminopeptidase Ey.
3.4.11.22 Vacuolar aminopeptidase I.
3.4.13.3 Xaa-His dipeptidase.
3.4.13.4 Xaa-Arg dipeptidase.
3.4.13.5 Xaa-methyl-His dipeptidase.
3.4.13.6 Cys-Gly dipeptidase.
3.4.13.7 Glu-Glu dipeptidase.
3.4.13.8 Pro-Xaa dipeptidase.
3.4.13.9 Xaa-Pro dipeptidase.
3.4.13.12 Met-Xaa dipeptidase.
3.4.13.17 Non-stereospecific dipeptidase.

3.4.13.18 Cytosol non-specific dipeptidase.
3.4.13.19 Membrane dipeptidase.
3.4.13.20 Beta-Ala-His dipeptidase.
3.4.14.1 Dipeptidyl-peptidase I.
3.4.14.2 Dipeptidyl-peptidase II.
3.4.14.4 Dipeptidyl-peptidase III.
3.4.14.5 Dipeptidyl-peptidase IV.
3.4.14.6 Dipeptidyl-dipeptidase.
3.4.14.9 Tripeptidyl-peptidase I.
3.4.14.10 Tripeptidyl-peptidase II.
3.4.14.11 Xaa-Pro dipeptidyl-peptidase.
3.4.15.1 Peptidyl-dipeptidase A.
3.4.15.4 Peptidyl-dipeptidase B.
3.4.15.5 Peptidyl-dipeptidase Dcp.
3.4.16.2 Lysosomal Pro-X carboxypeptidase.
3.4.16.4 Serine-type D-Ala-D-Ala carboxypeptidase.
3.4.16.5 Carboxypeptidase C.
3.4.16.6 Carboxypeptidase D.
3.4.17.1 Carboxypeptidase A.
3.4.17.2 Carboxypeptidase B.
3.4.17.3 Lysine(arginine) carboxypeptidase.
3.4.17.4 Gly-X carboxypeptidase.
3.4.17.6 Alanine carboxypeptidase.
3.4.17.7 Transferred entry: 3.4.19.10.
3.4.17.8 Muramoylpentapeptide carboxypeptidase.
3.4.17.10 Carboxypeptidase H.
3.4.17.11 Glutamate carboxypeptidase.
3.4.17.12 Carboxypeptidase M.
3.4.17.13 Muramoyltetrapeptide carboxypeptidase.
3.4.17.14 Zinc D-Ala-D-Ala carboxypeptidase.
3.4.17.15 Carboxypeptidase A2.
3.4.17.16 Membrane Pro-X carboxypeptidase.
3.4.17.17 Tubulinyl-Tyr carboxypeptidase.
3.4.17.18 Carboxypeptidase T.
3.4.17.19 Thermostable carboxypeptidase I.
3.4.17.20 Carboxypeptidase U.
3.4.17.21 Glutamate carboxypeptidase II.
3.4.17.22 Metallocarboxypeptidase D.
3.4.18.1 Cysteine-type carboxypeptidase.
3.4.19.1 Acylaminoacyl-peptidase.
3.4.19.2 Peptidyl-glyeinamidase.
3.4.19.3 Pyroglutamyl-peptidase I.
3.4.19.5 Beta-aspartyl-peptidase.
3.4.19.6 Pyroglutamyl-peptidase II.
3.4.19.7 N-formylmethionyl-peptidase.
3.4.19.8 Pteroylpoly-gamma-glutamate carboxypeptidase.
3.4.19.9 Gamma-glutamyl hydrolase.
3.4.19.11 Gamma-D-glutamyl-meso-diaminopimelate peptidase I.
3.4.21.1 Chymotrypsin.
3.4.21.2 Chymotrypsin C.
3.4.21.3 Metridin.
3.4.21.4 Trypsin.
3.4.21.5 Thrombin.
3.4.21.6 Coagulation factor Xa.
3.4.21.7 Plasmin.
3.4.21.8 Transferred entry: 3.4.31.34 and 3.4.21.35.
3.4.21.9 Enteropeptidase.
3.4.21.10 Acrosin.

3.4.21.11 Transferred entry: 3.4.21.36 and 3.4.21.37.
3.4.21.12 Alpha-lytic endopeptidase.
3.4.21.19 Glutamyl endopeptidase.
3.4.21.20 Cathepsin G.
3.4.21.21 Coagulation factor VIIa.
3.4.21.22 Coagulation factor IXa.
3.4.21.25 Cucumisin.
3.4.21.26 Prolyl oligopeptidase.
3.4.21.27 Coagulation factor XIa.
3.4.21.32 Brachyurin.
3.4.21.34 Plasma kallikrein.
3.4.21.35 Tissue kallikrein.
3.4.21.36 Pancreatic elastase.
3.4.21.37 Leukocyte elastase.
3.4.21.38 Coagulation factor XIIa.
3.4.21.39 Chymase.
3.4.21.41 Complement component C1r.
3.4.21.42 Complement component C1s.
3.4.21.43 Classical-complement pathway C3/C5 convertase.
3.4.21.45 Complement factor I.
3.4.21.46 Complement factor D.
3.4.21.47 Altemative-complement pathway C3/C5 convertase.
3.4.21.48 Cerevisin.
3.4.21.49 Hypodermin C.
3.4.21.50 Lysyl endopeptidase.
3.4.21.53 Endopeptidase La.
3.4.21.54 Gamma-renin.
3.4.21.55 Venombin AB.
3.4.21.57 Leucyl endopeptidase.
3.4.21.59 Tryptase.
3.4.21.60 Scutelarin.
3.4.21.61 Kexin.
3.4.21.62 Subtilisin.
3.4.21.63 Oryzin.
3.4.21.64 Proteinase K.
3.4.21.65 Thermomycolin.
3.4.21.66 Thermitase.
3.4.21.67 Endopeptidase So.
3.4.21.68 T-plasminogen activator.
3.4.21.69 Protein C (activated).
3.4.21.70 Pancreatic endopeptidase E.
3.4.21.71 Pancreatic elastase II.
3.4.21.72 IgA-specific serine endopeptidase.
3.4.21.73 U-plasminogen activator.
3.4.21.74 Venombin A.
3.4.21.75 Furin.
3.4.21.76 Myeloblastin.
3.4.21.77 Semenogelase.
3.4.21.78 Granzyme A.
3.4.21.79 Granzyme B.
3.4.21.80 Streptogrisin A.
3.4.21.81 Streptogrisin B.
3.4.21.82 Glutamyl endopeptidase II.
3.4.21.83 Olieopeptidase B.
3.4.21.84 Limulus clotting factor C.
3.4.21.85 Limulus clotting factor B.
3.4.21.86 Limulus clotting enzyme.
3.4.21.87 Omptin.

3.4.21.88 Repressor lexA.
3.4.21.89 Signal peptidase I.
3.4.21.90 Togavirin.
3.4.21.91 Flavirin.
3.4.21.92 Endopeptidase Clp.
3.4.21.93 Proprotein convertase 1.
3.4.21.94 Proprotein convertase 2.
3.4.21.95 Snake venom factor V activator.
3.4.21.96 Lactocepin.
3.4.22.1 Cathepsin B.
3.4.22.2 Papain.
3.4.22.3 Ficain.
3.4.22.6 Chymopapain.
3.4.22.7 Asclepain.
3.4.22.8 Clostripain.
3.4.22.10 Streptopain.
3.4.22.14 Actinidain.
3.4.22.15 Cathepsin L.
3.4.22.16 Cathepsin H.
3.4.22.17 Calpain.
3.4.22.24 Cathepsin T.
3.4.22.25 Glycyl endopeptidase.
3.4.22.26 Cancer procoagulant.
3.4.22.27 Cathepsin S.
3.4.22.28 Picomain 3C.
3.4.22.29 Picomain 2A.
3.4.22.30 Caricain.
3.4.22.31 Ananain.
3.4.22.32 Stem bromelain.
3.4.22.33 Fruit bromelain.
3.4.22.34 Legumain.
3.4.22.35 Histolysain.
3.4.22.36 Caspase-1.
3.4.22.37 Gingipain R.
3.4.22.38 Cathepsin K.
3.4.23.1 Pepsin A.
3.4.23.2 Pepsin B.
3.4.23.3 Gastricsin.
3.4.23.4 Chymosin.
3.4.23.5 Cathepsin D.
3.4.23.12 Neopenthesin.
3.4.23.15 Renin.
3.4.23.16 Retropepsin.
3.4.23.17 Pro-opiomelanocortin converting enzyme.
3.4.23.18 Aspergillopepsin I.
3.4.23.19 Aspergillopepsin II.
3.4.23.20 Penicillopepsin.
3.4.23.21 Rhizopuspepsin.
3.4.23.22 Endothiapepsin.
3.4.23.23 Mucoropepsin.
3.4.23.24 Candidapepsin.
3.4.23.25 Saccharopepsin.
3.4.23.26 Rhodotorulapepsin.
3.4.23.27 Physaropepsin.
3.4.23.28 Acrocylindropepsin.
3.4.23.29 Polyporopepsin.
3.4.23.30 Pycnoporopepsin.
3.4.23.31 Scytalidopepsin A.

3.4.23.32 Scytalidopepsin B.
3.4.23.33 Xanthomonapepsin.
3.4.23.34 Cathepsin E.
3.4.23.35 Barrierpepsin.
3.4.23.36 Signal peptidase II.
3.4.23.37 Pseudomonapepsin.
3.4.23.38 Plasmepsin I.
3.4.23.39 Plasmepsin II.
3.4.23.40 Phytepsin.
3.4.24.1 Atrolysin A.
3.4.24.3 Microbial collagenase.
3.4.24.6 Leucolysin.
3.4.24.7 Interstitial collagenase.
3.4.24.11 Neprilysin.
3.4.24.12 Envelysin.
3.4.24.13 IgA-specific metalloendopeptidase.
3.4.24.14 Procollagen N-endopeptidase.
3.4.24.15 Thimet oligopeptidase.
3.4.24.16 Neurolysin.
3.4.24.17 Stromelysin I.
3.4.24.18 Meprin A.
3.4.24.19 Procollagen C-endopeptidase.
3.4.24.20 Peptidyl-Lys metalloendopeptidase.
3.4.24.21 Astacin.
3.4.24.22 Stromelysin 2.
3.4.24.23 Matrilysin.
3.4.24.24 Gelatinase A.
3.4.24.25 Aeromonolysin.
3.4.24.26 Pseudolysin.
3.4.24.27 Thermolysin.
3.4.24.28 Bacillolysin.
3.4.24.29 Aureolysin.
3.4.24.30 Coccolysin.
3.4.24.31 Mycolysin.
3.4.24.32 Beta-lytic metalloendopeptidase.
3.4.24.33 Peptidyl-Asp metalloendopeptidase.
3.4.24.34 Neutrophil collagenase.
3.4.24.35 Gelatinase B.
3.4.24.36 Leishmanolysin.
3.4.24.37 Saccharolysin.
3.4.24.38 Autolysin.
3.4.24.39 Deuterolysin.
3.4.24.40 Serralysin.
3.4.24.41 Atrolysin B.
3.4.24.42 Atrolysin C.
3.4.24.43 Atroxase.
3.4.24.44 Atrolysin E.
3.4.24.45 Atrolysin F.
3.4.24.46 Adamalysin.
3.4.24.47 Horrilysin.
3.4.24.48 Ruberlysin.
3.4.24.49 Bothropasin.
3.4.24.50 Bothrolysin.
3.4.24.51 Ophiolysin.
3.4.24.52 Trimerelysin I.
3.4.24.53 Trimerelysin II.
3.4.24.54 Mucrolysin.
3.4.24.55 Pitrilysin.

3.4.24.56 Insulysin.
3.4.24.57 O-sialoglycoprotein endopeptidase.
3.4.24.58 Russellysin.
3.4.24.59 Mitochondrial intermediate peptidase.
3.4.24.60 Dactylysin.
3.4.24.61 Nardilysin.
3.4.24.62 Magnolysin.
3.4.24.63 Meprin B.
3.4.24.64 Mitochondrial processing peptidase.
3.4.24.65 Macrophage elastase.
3.4.24.66 Choriolysin L.
3.4.24.67 Choriolysin H.
3.4.24.68 Tentoxilysin.
3.4.24.69 Bontoxilysin.
3.4.24.70 Oligopeptidase A.
3.4.24.71 Endothelin-converting enzyme 1.
3.4.24.72 Fibrolase.
3.4.24.73 Jararhagin.
3.4.24.74 Fragilysin.
3.4.99.46 Multicatalytic endopeptidase complex.

*i) Synthetic Peptide Libraries*

Peptide libraries are systems which simultaneously display, in a form which permits interaction with a target protein, a highly diverse and numerous collection of peptides. In preferred embodiments, the combinatorial polypeptides are in the range of 3-100 amino acids in length, more preferably at least 5-50, and even more preferably at least 10, 13, 15, 20 or 25 amino acid residues in length. It will be understood that the length of the combinatorial peptide does not reflect any extraneous sequences which may be present in order to facilitate purification or the like.

A preferred type of oriented degenerate peptide library for use in the method of the invention is a soluble synthetic peptide library. The term "soluble synthetic peptide library" is intended to mean a population of peptides which are constructed by *in vitro* chemical synthesis, for example using an automated peptide synthesizer, and which are not connected to a solid support such as a bead or a cell. Standard techniques for *in vitro* chemical synthesis of peptides are known in the art. For example, peptides can be synthesized by (benzotriazolyloxy)tris(dimethylamino)-phosphonium hexafluorophosophate (BOP)/1-hydroxybenzotriazole coupling protocols. Automated peptide synthesizers are commercially available (e.g., Milligen/Biosearch 9600).

In contrast to recombinant methods, which may be employed for certain aspects of the present invention, *in vitro* chemical synthesis provides a method for generating libraries of compounds, without the use of living organisms, that can be screened for ability to bind to a target protein. Although *in vitro* methods have been used for quite some time in the pharmaceutical industry to identify potential drugs, recently developed methods have focused on rapidly and efficiently generating and screening large numbers of compounds and are particularly amenable to generating peptide libraries with non-natural functionality for use in the subject method. The various approaches to simultaneous preparation and analysis of large numbers of synthetic peptides (herein "multiple peptide synthesis" or "MPS") each rely on the fundamental concept of synthesis on a solid support introduced by Merrifield in 1963 (Merrifield, R.B. (1963) *J Am Chem Soc* 85:2149-2154; and references cited in section I above). Generally, these techniques are not dependent on the protecting group or activation chemistry employed, although most workers today avoid Merrifield's original tBoc/Bzl strategy in favor of the more mild Fmoc/$^{t}$Bu chemistry and efficient hydroxybenzotriazole-based coupling agents. Many types of solid matrices have been successfully used in MPS, and yields of individual peptides synthesized vary widely with the technique adopted (e.g., nanomoles to millimoles).

*a) Multipin Synthesis*

One form that the peptide library of the subject method can take is the multipin library format. Briefly, Geysen and co-workers (Geysen et al. (1984) *PNAS* 81:3998-4002) introduced a method for generating peptides by parallel synthesis on polyacrylic acid-grated polyethylene pins arrayed in the microtitre plate format. In the original experiments, about 50 nmol of a single peptide sequence was covalently linked to the spherical head of each pin, and interactions of each peptide with a receptor or antibody could be determined in a direct binding assay. The Geysen technique can be used to synthesize and screen thousands of peptides per week using the multipin method, and the tethered peptides may be reused in many assays. In subsequent work, the level of peptide loading on individual pins has been increased

to as much as 2 µmol/pin by grafting greater amounts of functionalized acrylate derivatives to detachable pin heads, and the size of the peptide library has been increased (Valerio et al. (1993) *Int J Pept Protein Res* 42:1-9). Appropriate linker moieties have also been appended to the pins so that the peptides may be cleaved from the supports after synthesis for assessment of purity and evaluation in competition binding or functional bioassays (Bray et al. (1990) *Tetrahedron Lett* 31:5811-5814; Valerio et al. (1991) *Anal Biochem* 197:168-177; Bray et al. (1991) *Tetrahedron Lett* 32:6163-6166).

More recent applications of the multipin method of MPS have taken advantage of the cleavable linker strategy to prepare soluble peptides (Maeji et al. (1990) *J Immunol Methods* 134:23-33; Gammon et al. (1991) *J Exp Med* 173: 609-617; Mutch et al. (1991) *Pept Res* 4:132-137).

*b) Divide-Couple-Recombine*

In yet another embodiment, a variegated library of peptides can provide on a set of beads utilizing.the strategy of divide-couple-recombine (see, for example, Houghten (1985) *PNAS* 82:5131-5135; and U.S. Patents 4,631,211, 5,440,016, and 5,480,971). Briefly, as the name implies, at each synthesis step where degeneracy is introduced into the library, the beads are divided into as many separate groups to correspond to the number of different amino acid residues to be added that position, the different residues coupled in separate reactions, and the beads recombined into one pool for the next step.

In one embodiment, the divide-couple-recombine strategy can be carried out using the so-called "tea bag" MPS method first developed by Houghten, peptide synthesis occurs on resin that is sealed inside porous polypropylene bags (Houghten et al. (1986) *PNAS* 82:5131-5135). Amino acids are coupled to the resins by placing the bags in solutions of the appropriate individual activated monomers, while all common steps such as resin washing and $\alpha$-amino group deprotection are performed simultaneously in one reaction vessel. At the end of the synthesis, each bag contains a single peptide sequence, and the peptides may be liberated from the resins using a multiple cleavage apparatus (Houghten et al. (1986) *Int J Pept Protein Res* 27:673-678). This technique offers advantages of considerable synthetic flexibility and has been partially automated (Beck-Sickinger et al. (1991) *Pept Res* 4:88-94). Moreover, soluble peptides of greater than 15 amino acids in length can be produced in sufficient quantities (>.500 µmol) for purification and complete characterization if desired.

Multiple peptide synthesis using the tea-bag approach is useful for the production of a peptide library, albeit of limited size, for screenings of the present method, as is illustrated by its use in a range of molecular recognition problems including antibody epitope analysis (Houghten et al. (1986) *PNAS* 82:5131-5135), peptide hormone structure-function studies (Beck-Sickinger et al. (1990) *Int J Pept Protein Res* 36:522-530; Beck-Sickinger et al. (1990) *Eur J Biochem* 194:449-456), and protein conformational mapping (Zimmerman et al. (1991) *Eur J Biochem* 200:519-528).

An exemplary synthesis of a set of mixed peptides having equimolar amounts of the twenty natural amino acid residues is as follows. Aliquots of five grams (4.65mmols) of p-methylbenzhydrylamine hydrochloride resin (MBHA) are placed into twenty porous polypropylene bags. These bags are placed into a common container and washed with 1.0 liter of $CH_2Cl_2$ three times (three minutes each time), then again washed three times (three minutes each time) with 1.0 liter of 5 percent DIEA/$CH_2Cl_2$ (DIEA = diisopropylethylamine; $CH_2Cl_2$ = DCM). The bags are then rinsed with DCM and placed into separate reaction vessels each containing 50 ml (0.56M) of the respective t-BOC-amino acid/ DCM. N,N-Diisopropylcarbodiimide (DIPCDI; 25 ml; 1.12M) is added to each container as a coupling agent. Twenty amino acid derivatives are separately coupled to the resin in 50/50 (v/v) DMF/DCM. After one hour of vigorous shaking, Gisen's picric acid test (Gisen (1972) *Anal. Chem. Acta* 58:248-249) is performed to determine the completeness of the coupling reaction. On confirming completeness of reaction, all of the resin packets are then washed with 1.5 liters of DMF and washed two more times with 1.5 liters of $CH_2Cl_2$. After rinsing, the resins are removed from their separate packets and admixed together to form a pool in a common bag. The resulting resin mixture is then dried and weighed, divided again into 20 equal portions (aliquots), and placed into 20 further polypropylene bags (enclosed).

In a common reaction vessel the following steps are carried out: (1) deprotection is carried out on the enclosed aliquots for thirty minutes with 1.5 liters of 55 percent TFA/DCM; and 2) neutralization is carried out with three washes of 1.5 liters each of 5 percent DIEA/DCM. Each bag is placed in a separate solution of activated t-BOC-amino acid-derivative and the coupling reaction carried out to completion as before. All coupling reactions are monitored using the above quantitative picric acid assay.

Next, the bags are opened and the resulting t-BOC-protected dipeptide resins are mixed together to form a pool, aliquots are made from the pool, the aliquots are enclosed, deprotected and further reactions are carried out. This process can be repeated any number of times yielding at each step an equimolar representation of the desired number of amino acid residues in the peptide chain. The principal process steps are conveniently referred to as a divide-couple-recombine synthesis.

After a desired number of such couplings and mixings are carried out, the polypropylene bags are kept separated to provide twenty sets having the amino-terminal residue as the single, predetermined residue, with, for example,

positions 2-4 being occupied by equimolar amounts of the twenty residues. To prepare sets having the single, predetermined amino acid residue at other than the amino-terminus, the contents of the bags are not mixed after adding a residue at the desired, predetermined position. Rather, the contents of each of the twenty bags are separated into 20 aliquots, deprotected and then separately reacted with the twenty amino acid derivatives. The contents of each set of twenty bags thus produced are thereafter mixed and treated as before-described until the desired oligopeptide length is achieved.

*c) Multiple Peptide Synthesis through Coupling of Amino Acid Mixtures*

Simultaneous coupling of mixtures of activated amino acids to a single resin support has been used as a multiple peptide synthesis strategy on several occasions (Geysen et al. (1986) *Mol Immunol* 23:709-715; Tjoeng et al. (1990) *Int J Pept Protein Res* 35:141-146; Rutter et al. (1991) U.S. Patent No. 5,010,175; Birkett et al. (1991) *Anal Biochem* 196:137-143; Petithory et al. (1991) *PNAS* 88:11510-11514) and can have applications in the subject method. For example, four to seven analogs of the magainin 2 and angiotensinogen peptides were successfully synthesized and resolved in one HPLC purification after coupling a mixture of amino acids at a single position in each sequence (Tjoeng et al. (1990) *Int J Pept Protein Res* 35:141-146). This approach has also been used to prepare degenerate peptide mixtures for defining the substrate specificity of endoproteolytic enzymes (Birkett. et al. (1991) *Anal Biochem* 196:137-143; Petithory et al. (1991) *PNAS* 88:11510-11514). In these experiments, a series of amino acids was substituted at a single position within the substrate sequence. After proteolysis, Edman degradation was used to quantitate the yield of each amino acid component in the hydrolysis product and hence to evaluate the relative $k_{cat}/K_m$ values for each substrate in the mixture.

However, it is noted that the operational simplicity of synthesizing many peptides by coupling monomer mixtures is offset by the difficulty in controlling the composition of the products. The product distribution reflects the individual rate constants for the competing coupling reactions, with activated derivatives of sterically hindered residues such as valine or isoleucine adding at a significantly slower rate than activated derivatives of glycine or alanine, for example. The nature of the resin-bound component of the acylation reaction also influences the addition rate, and the relative rate constants for the formation of 400 dipeptides form the 20 genetically coded amino acids have been determined by Rutter and Santi (Rutter et al. (1991) U.S. Patent No. 5,010,175). These reaction rates can be used to guide the selection of appropriate relative concentrations of amino acids in the mixture to favor more closely equimolar coupling yields.

*d) Multiple Peptide Synthesis on Nontraditional Solid Supports*

The search for innovative methods of multiple peptide synthesis has led to the investigation of polymeric supports alternative to the polystyrene-divinylbenzene matrix originally popularized by Merrifield. Cellulose, either in the form of paper disks (Blankemeyer-Menge et al. (1988) *Tetrahedron Lett* 29-5871-5874; Frank et al. (1988) *Tetrahedron* 44:6031-6040; Eichler et al. (1989) *Collect Czech Chem Commun* 54:1746-1752; Frank, R. (1993) *Bioorg Med Chem Lett* 3:425-430) or cotton fragments (Eichler et al. (1991) *Pept Res* 4:296-307; Schmidt et al. (1993) *Bioorg Med Chem Lett* 3:441-446), has been successfully functionalized for peptide synthesis. Typical loadings attained with cellulose paper range from 1 to 3 $\mu mol/cm^2$, and HPLC analysis of material cleaved from these supports indicates a reasonable quality for the synthesized peptides. Alternatively, peptides may be synthesized on cellulose sheets via non-cleavable linkers and then used in ELISA-based binding studies (Frank, R. (1992) *Tetrahedron* 48:9217-9232). The porous, polar nature of this support may help suppress unwanted nonspecific protein binding effects. By controlling the volume of activated amino acids and other reagents spotted on the paper, the number of peptides synthesized at discrete locations on the support can be readily varied. In one convenient configuration spots are made in an 8 x 12 microtiter plate format. Frank has used this technique to map the dominant epitopes of an antiserum raised against a human cytomegalovirus protein, following the overlapping peptide screening (Pepscan) strategy of Geysen (Frank, R. (1992) *Tetrahedron* 48:9217-9232). Other membrane-like supports that may be used for multiple solid-phase synthesis include polystyrene-grafted polyethylene films (Berg et al. (1989) *J Am Chem Soc* 111:8024-8026).

*e) Combinatorial Libraries by Light-Directed, Spatially Addressable Parallel Chemical Synthesis*

A scheme of combinatorial synthesis in which the identity of a compound is given.by its locations on a synthesis substrate is termed a spatially-addressable synthesis. In one embodiment, the combinatorial process is carried out by controlling the addition of a chemical reagent to specific locations on a solid support (Dower et al. (1991) *Annu Rep Med Chem* 26:271-280; Fodor, S.P.A. (1991) *Science* 251:767; Pirrung et al. (1992) U.S. Patent No. 5,143,854; Jacobs et al. (1994) *Trends Biotechnol* 12:19-26). The technique combines two well-developed technologies: solid-phase peptide synthesis chemistry and photolithography. The high coupling yields of Merrifield chemistry allow efficient peptide

synthesis, and the spatial resolution of photolithography affords miniaturization. The merging of these two technologies is done through the use of photolabile amino protecting groups in the Merrifield synthetic procedure.

The key points of this technology are illustrated in Gallop et al. (1994) *J Med Chem* 37:1233-1251. A synthesis substrate is prepared for amino acid coupling through the covalent attachment of photolabile nitroveratryloxycarbonyl (NVOC) protected amino linkers. Light is used to selectively activate a specified region of the synthesis support for coupling. Removal of the photolabile protecting groups by lights (deprotection) results in activation of selected areas. After activation, the first of a set of amino acids, each bearing a photolabile protecting group on the amino terminus, is exposed to the entire surface. Amino acid coupling only occurs in regions that were addressed by light in the preceding step. The solution of amino acid is removed, and the substrate is again illuminated through a second mask, activating a different region for reaction with a second protected building block. The pattern of masks and the sequence of reactants define the products and their locations. Since this process utilizes photolithography techniques, the number of compounds that can be synthesized is limited only by the number of synthesis sites that can be addressed with appropriate resolution. The position of each compound is precisely known; hence, its interactions with other molecules can be directly assessed. The target protein can be labeled with a fluorescent reporter group to facilitate the identification of specific interactions with individual members of the matrix.

In a light-directed chemical synthesis, the products depend on the pattern of illumination and on the order of addition of reactants. By varying the lithographic patterns, many different sets of test peptides can be synthesized in the same number of steps. This leads to the generation of many different masking strategies.

*f) Encoded Combinatorial Libraries*

In yet another embodiment, the subject method utilizes a peptide library provided with an encoded tagging system. A recent improvement in the identification of active compounds from combinatorial libraries employs chemical indexing systems using tags that uniquely encode the reaction steps a given bead has undergone and, by inference, the structure it carries. Conceptually, this approach mimics phage display libraries above, where activity derives from expressed peptides, but the structures of the active peptides are deduced from the corresponding genomic DNA sequence. The first encoding of synthetic combinatorial libraries employed DNA as the code. Two forms of encoding have been reported: encoding with sequenceable bio-oligomers (e.g., oligonucleotides and peptides), and binary encoding with non-sequenceable tags.

*1) Tagging with sequenceable bio-oligomers*

The principle of using oligonucleotides to encode combinatorial synthetic libraries was described in 1992 (Brenner et al. (1992) *PNAS* 89:5381-5383), and an example of such a library appeared the following year (Needles et al. (1993) *PNAS* 90:10700-10704). A combinatorial library of nominally $7^7$ (= 823,543) peptides composed of all combinations of Arg, Gln, Phe, Lys, Val, D-Val and Thr (three-letter amino acid code), each of which was encoded by a specific dinucleotide (TA, TC, CT, AT, TT, CA and AC, respectively), was prepared by a series of alternating rounds of peptide and oligonucleotide synthesis on solid support. In this work, the amine-linking functionality on the bead was specifically differentiated toward peptide or oligonucleotide synthesis by simultaneously preincubating the beads with reagents that generate protected OH groups for oligonucleotide synthesis and protected $NH_2$ groups for peptide synthesis (here, in a ratio of 1:20). When complete, the tags each consisted of 69-mers, 14 units of which carried the code. The bead-bound library was incubated with a fluorescently labeled antibody, and beads containing bound antibody that fluoresced strongly were harvested by fluorescence-activated cell sorting (FACS). The DNA tags were amplified by PCR and sequenced, and the predicted peptides were synthesized. Following the such techniques, the peptide libraries can be derived for use in the subject method and screened using the D-enantiomer of the target protein.

It is noted that an alternative approach useful for generating nucleotide-encoded synthetic peptide libraries employs a branched linker containing selectively protected OH and $NH_2$ groups (Nielsen et al. (1993) *J Am Chem Soc* 115:9812-9813; and Nielsen et al. (1994) *Methods Compar Methods Enzymol* 6:361-371). This approach requires that equimolar quantities of test peptide and tag co-exist, though this may be a potential complication in assessing biological activity, especially with nucleic acid based targets.

The use of oligonucleotide tags permits exquisitely sensitive tag analysis. Even so, the method requires careful choice of orthogonal sets of protecting groups required for alternating co-synthesis of the tag and the library member. Furthermore, the chemical lability of the tag, particularly the phosphate and sugar anomeric linkages, may limit the choice of reagents and conditions that can be employed for the synthesis on non-oligomeric libraries. In preferred embodiments, the libraries employ linkers permitting selective detachment of the test peptide library member for bioassay, in part (as described *infra*) because assays employing beads limit the choice of targets, and in part because the tags are potentially susceptible to biodegradation.

Peptides themselves have been employed as tagging molecules for combinatorial libraries. Two exemplary

approaches are described in the art, both of which employ branched linkers to solid phase upon which coding and ligand strands are alternately elaborated. In the first approach (Kerr JM et al. (1993) *J Am Chem Soc* 115:2529-2531), orthogonality in synthesis is achieved by employing acid-labile protection for the coding strand and base-labile protection for the ligand strand.

In an alternative approach (Nikolaiev et al. (1993) *Pept Res* 6:161-170), branched linkers are employed so that the coding unit and the test peptide are both attached to the same functional group on the resin. In one embodiment, a linker can be placed between the branch point and the bead so that cleavage releases a molecule containing both code and ligand (Ptek et al. (1991) *Tetrahedron Lett* 32:3891-3894). In another embodiment, the linker can be placed so that the test peptide can be selectively separated from the bead, leaving the code behind. This last construct is particularly valuable because it permits screening of the test peptide without potential interference, or biodegradation, of the coding groups. Examples in the art of independent cleavage and sequencing of peptide library members and their corresponding tags has confirmed that the tags can accurately predict the peptide structure.

It is noted that peptide tags are more resistant to decomposition during ligand synthesis than are oligonucleotide tags, but they must be employed in molar ratios nearly equal to those of the ligand on typical 130 μm beads in order to be successfully sequenced. As with oligonucleotide encoding, the use of peptides as tags requires complex protection/deprotection chemistries.

*2) Non-sequenceable tagging: binary encoding*

An alternative form of encoding the test peptide library employs a set of non-sequenceable electrophoric tagging molecules that are used as a binary code (Ohlmeyer et al. (1993) *PNAS* 90:10922-10926). Exemplary tags are haloaromatic alkyl ethers that are detectable as their tetramethylsilyl ethers at less than femtomolar levels by electron capture gas chromatography (ECGC). Variations in the length of the alkyl chain, as well as the nature and position of the aromatic halide substituents, permit the synthesis of at least 40 such tags, which in principle can encode $2^{40}$ (e.g., upwards of $10^{12}$) different molecules. In the original report (Ohlmeyer et al., *supra*), the tags were bound to about 1% of the available amine groups of a peptide library via a photocleavable O-nitrobenzyl linker. This approach is convenient when preparing combinatorial libraries of peptides or other amine-containing molecules. A more versatile system has, however, been developed that permits encoding of essentially any combinatorial library. Here, the ligand is attached to the solid support via the photocleavable linker and the tag is attached through a catechol ether linker via carbene insertion into the bead matrix (Nestler et al. (1994) *J Org Chem* 59:4723-4724). This orthogonal attachment strategy permits the selective detachment of library members for bioassay in solution and subsequent decoding by ECGC after oxidative detachment of the tag sets.

Binary encoding with electrophoric tags has been particularly useful in defining selective interactions of substrates with synthetic receptors (Borchardt et al. (1994) *J Am Chem Soc* 116:373-374), and model systems for understanding the binding and catalysis of biomolecules. Even using detailed molecular modeling, the identification of the selectivity preferences for synthetic receptors has required the manual synthesis of dozens of potential substrates. The use of encoded libraries makes it possible to rapidly examine all the members of a potential binding set. The use of binary-encoded libraries has made the determination of binding selectivities so facile that structural selectivity has been reported for four novel synthetic macrobicyclic and tricyclic receptors in a single communication (Wennemers et al. (1995) *J Org Chem* 60:1108-1109; and Yoon et al. (1994) *Tetrahedron Lett* 35:8557-8560) using the encoded library mentioned above. Similar facility in defining specificity of interaction would be expected for many other biomolecules.

Although the several amide-linked libraries in the art employ binary encoding with the electrophoric tags attached to amine groups, attaching these tags directly to the bead matrix provides far greater versatility in the structures that can be prepared in encoded combinatorial libraries. Attached in this way, the tags and their linker are nearly as unreactive as the bead matrix itself. Two binary-encoded combinatorial libraries have been reported where the electrophoric tags are attached directly to the solid phase (Ohlmeyer et al. (1995) *PNAS* 92:6027-6031) and provide guidance for generating the subject peptide library. Both libraries were constructed using an orthogonal attachment strategy in which the library member was linked to the solid support by a photolabile linker and the tags were attached through a linker cleavable only by vigorous oxidation. Because the library members can be repetitively partially photoeluted from the solid support, library members can be utilized in multiple assays. Successive photoelution also permits a very high throughput iterative screening strategy: first, multiple beads are placed in 96-well microtiter plates; second, ligands are partially detached and transferred to assay plates; third, a bioassay identifies the active wells; fourth, the corresponding beads are rearrayed singly into new microtiter plates; fifth, single active compounds are identified; and sixth, the structures are decoded.

The above approach was employed in screening for carbonic anhydrase (CA) binding and identified compounds which exhibited nanomolar affinities for CA. Unlike sequenceable tagging, a large number of structures can be rapidly decoded from binary-encoded libraries (a single ECGC apparatus can decode 50 structures per day). Thus, binary-encoded libraries can be used for the rapid analysis of structure-activity relationships and optimization of both

potency and selectivity of an active series. The synthesis and screening of large unbiased binary encoded peptide libraries for lead identification, followed by preparation and analysis of smaller focused libraries for lead optimization, offers a particularly powerful approach to drug discovery using the subject method.

It will be apparent to one of skill in the art that the above methods are directed towards the synthesis of highly diverse peptide libraries. Incorporation of an active subunit designed to mimic a transition state species that interacts with the active site of an enzyme may require including a subunit which is not a common amino acid. The above methods can be readily adapted to this end.

For instance, in the case where a boronic acid-bearing subunit is desired to be incorporated into a peptide library, a peptide library can be prepared according to any of the above methods, the C-terminus can be liberated to reveal the carboxylic acid, and an active subunit of, for example, the general formula $H_2N\text{-}CH(R)\text{-}B(OH)_2$, where R is any functional group, desired to be incorporated into this subunit, including substituted and unsubstituted alkyl and aryl residues, can be coupled to it by a standard method of peptide coupling.

Another possibility is to attach a first amino acid to a resin by the N-terminus and synthesize the peptide library in the N-to-C direction. Although peptide synthesis in this direction is more problematic and less commonplace than synthesis in the C-to-N direction, this method uses the same basic methodology and allows for a peptide library to be synthesized and then uniformly or degenerately be coupled to one or more boron-containing subunits, e.g., an active subunit having the formula set forth in the preceding paragraph, or an active subunit having any other functionality which may be desired. Resins appropriate for attachment via the N-terminus are known in the art.

A third option is to attach a boronic acid-bearing subunit according to the general formula set forth above to a resin, e.g., PerSeptive's backbone amide linker (BAL) resin, by means of the nitrogen functionality. The remaining residues of the peptide library may then be attached to the N-terminus of the boronic acid-bearing subunit according to the methods described above, and the library may be cleaved from the resin, e.g., by using 85% TFA in the case where the resin is BAL resin. More generally, such a nitrogen-based linkage may be formed by treating an aldehyde-bearing resin with a nitrogen-containing subunit in the presence of a hydride reducing agent (e.g. $NaBH_4$ and variants thereof) under conditions for reductively aminating the resin. Such reductive aminations are well known in the art.

Alternatively, a boronic acid-containing residue can be directly attached to the resin through the boronic acid functionality. For example, a resin containing a vicinal diol functionality (HO-C-C-OH) can be used to form a cyclic boronic ester with the boronic acid of the selected subunit. This has the dual benefits of protecting the boronic acid functionality during the remainder of the library synthesis, while also providing a cleavable link to the solid support. In this manner, the peptide library, when liberated from the resin, e.g., by acid hydrolysis, has the boronic acid residue present on all members of the library. Furthermore, by attaching a variety of boronic acid subunits with different sidechains to the resin, this method allows the boronic-acid bearing position to be degenerate as well. A similar strategy may be employed for aldehyde-containing active subunits, which may be attached to a diol-containing resin by forming an acetal linkage.

It has been estimated that approximately 9 to 12 amino acids of a substrate peptide are likely to contact the active site and associated substrate specificity subsites of an enzyme, based upon mutagenesis studies of known consensus motifs for phosphorylation sites of certain kinases and from X-ray crystallographic studies of peptides bound to kinases. Accordingly, an oriented degenerate peptide library can be used in which the degenerate residues encompass the region likely to contact the active site and specificity subsites of the chosen enzyme. The number of amino acids residues surrounding the residue of a substrate which enters the active site that influence the substrate specificity of a enzyme is likely to differ for different enzymes. Thus, it is expected that oriented degenerate peptide libraries which will be useful in the method of the invention may have as few as two degenerate amino acid residues covalently attached to the active subunit which acts as a transition-state analog. For some enzymes it will be preferred that both amino acids be bound to the same side of the subunit, and for other enzymes, it will be preferred that one amino acid be bound to each side of the active subunit, depending on the characteristics of the enzyme. A preferred number of degenerate residues to be attached to the active subunit is at least two, preferably at least four. There may be an equal number of degenerate residues on either side of the active subunit, or there may be unequal numbers of degenerate residues on either side of the active subunit (e.g., 1 on one side and 3 on the other, etc.). The diversity of the peptide library (e.g., the number of different peptides contained within the library) is a function of the number of degenerate residues; the greater the number of degenerate residues the greater the diversity. For example, a library in which only 2 positions are degenerate and any amino acid can be at these degenerate positions would represent 400 unique peptides ($20^2$) whereas a library in which 8 positions are degenerate and any amino acid can be at these positions would represent approximately $2.5 \times 10^{10}$ unique peptides ($20^8$).

In one embodiment of the invention, the oriented degenerate peptide library is composed of peptides having a formula:

$$(Xaa)_n\text{-}Yaa\text{-}(Xaa)_m \qquad \text{(SEQ ID NO: 1)}$$

wherein Yaa is an active subunit selected to interact, e.g., by forming a covalent bond, with a portion of an enzyme active site, Xaa is any amino acid or amino acid analog, and n and m are integers from 0-10 inclusive, such that the sum of m and n is at least two, preferably at least four.

In a preferred embodiment of the invention, the active subunit at the fixed non-degenerate position is the only residue in the peptide that can, for example, covalently interact with the active site of the enzyme. Peptide libraries can be constructed in which the type of amino acid residue(s) likely to interact with the active site of the chosen enzyme are omitted at the degenerate position(s). Similarly, certain amino acids bearing functionality likely to interact with the active subunit, e.g., serine when the active subunit is a boronic acid, may similarly be omitted from some or all of the degenerate positions. Accordingly the invention provides oriented degenerate peptide libraries composed of peptides having a formula: $(Xaa)_n$-Yaa-$(Xaa)_m$ that have certain amino acids omitted from the degenerate position(s).

In certain embodiments, it may be desirable to add additional amino acid residues to one or both ends of the degenerate region(s) of the peptides. Non-degenerate amino acid residues N-terminal to the degenerate region of the peptides can serve to verify that peptides from the peptide mixture are being sequenced properly and can be used to quantitate the amount of peptides present. Similarly, non-degenerate amino acid residues C-terminal to the degenerate region of the peptides can be used for quantification purposes. The addition of a poly-lysine tail to the C-terminal end of the peptides of the library can prevent wash-out of peptides during automated sequencing and improve the solubility of the peptide mixture.

Another embodiment of the peptide library of the invention specifically allows for the addition of non-degenerate amino acids at the N-terminal and/or C-terminal ends of the degenerate region of the peptides. In this embodiment, the oriented degenerate peptide library comprises peptides comprising a formula:

$$Z_1\text{-}(Xaa)_n\text{-}Yaa\text{-}(Xaa)_m\text{-}Z_2 \qquad \text{(SEQ ID NO: 2)}$$

wherein

Yaa is a non-degenerate active subunit as defined above,

Xaa is any amino acid or amino acid analog,

n and m are integers from 0-10 inclusive, such that the sum of m and n is at least two, preferably at least four,

$Z_1$ is hydrogen or a peptide having a formula $(Xaa)_a$ wherein Xaa is any non-degenerate amino acid or amino acid analog and a is an integer from 1-15 inclusive, and

$Z_2$ is hydrogen or a peptide having a formula $(Xaa)_b$ wherein Xaa is any non-degenerate amino acid or amino acid analog and b is an integer from 1-15 inclusive.

This library encompasses peptides up to a length of 51 amino acids. Alternatively, a and b can be integers from 1-10 inclusive. This library encompasses peptides up to a length of 41 amino acids. Alternatively, a and b can be integers from 1-5 inclusive. This library encompasses peptides up to a length of 31 amino acids.

In yet another embodiment, the invention provides an oriented degenerate peptide library comprising peptides comprising a formula

(SEQ ID NO:3):

Met-Ala-Xaa1-Xaa2-Xaa3-Xaa4-Yaa-Xaa5-Xaa6-Xaa7-Xaa8-Ala-Lys-Lys-Lys

wherein Xaa1, Xaa2, Xaa3, Xaa4, Xaa5, Xaa6, Xaa7, and Xaa8 are any amino acid residue and Yaa is an active subunit as described above.

The active subunit may be selected to include a functionality that permits members of the peptide library to function as slow-binding inhibitors of the target enzyme. For instance, when the target enzyme is a serine protease, boronic acids, aldehydes, trifluoromethyl ketones, alpha-keto carboxylic acid derivatives such as alpha-keto amides, perhaloalkyls or perhaloalkyls or perfluoroalkyls such as pentafluoroethyl, and phosphinic functional groups, among others, have been shown to exhibit slow-binding inhibition of serine proteinases. A degenerate peptide library designed to determine a preferred amino acid sequence motif for a serine protease may be designed to include one of these functional groups on the active subunit. The active subunit may be located at the C-terminus, the N-terminus, or in the interior of the members of the peptide library. Slow binding inhibitors of many other classes are known, and incorporation of similar active subunits into the degenerate peptide libraries of this invention is within the purview of one skilled in the art.

To select from a degenerate peptide library as described above those sequences which bind the active site and associated sequence specificity subsites with high affinity, the peptide library is treated with a quantity of an enzyme, such that there are substantially fewer enzyme molecules than peptide molecules. For instance, there may be no more than 20%-as many molecules of enzyme as peptides, possibly less than 10%, 5%, 1%, or even less than 1%. Following a period of time appropriate for allowing the enzyme to bind members of the peptide library and preferably to reach equilibrium, the enzyme is washed free of umbound peptide. Then, the bound peptides may be liberated from the enzyme and collected, and their sequences determined, e.g., by a chemical sequencing technique, detection or characterization of a label or tag, etc. Liberation of the bound peptides may be facilitated by changing the pH, salt concentration, temperature, dilution, or other attributes of the medium containing the enzyme/peptide complexes.

An enzyme used in the method of the invention can be a purified native enzyme, for example purified from a biological source. Some purified enzymes are commercially available (e.g., from Sigma Chemical Co.). Alternatively, an enzyme used in the method of the invention can be a recombinantly produced enzyme. Many enzymes have been molecularly cloned and characterized and thus can be expressed recombinantly by standard techniques. A recombinantly produced enzyme which maintains proper enzymatic function can be used in the method of the invention. If the recombinant enzyme to be examined is a eukaryotic enzyme, it is preferable that the enzyme be recombinantly expressed in a eukaryotic expression system to ensure proper post-translational modification of the enzyme. Many eukaryotic expression systems (e.g., baculovirus and yeast expression systems) are known in the art and standard procedures can be used to express an enzyme recombinantly. A recombinantly produced enzyme can also be a fusion protein (i.e., composed of the enzyme and a second protein or peptide, for example a protease fused to glutathione-S-transferase (GST)) as long as the fusion protein retains the catalytic activity of the non-fused form of the enzyme. Furthermore, the term "enzyme" is intended to include portions of native enzymes which retain catalytic activity. For example, a subunit of a multisubunit protease which contains the catalytic domain of the protease can be used in the method of the invention.

After the bound peptides are separated from the enzymes, the amino acid sequences of the peptides may be determined. Preferably, the oriented degenerate peptide library used is a soluble synthetic peptide library and the peptides are sequenced as a bulk population using an automated peptide sequencer (alternatively, manual sequencing could be performed, e.g., using Edman-based techniques). This approach provides information on the abundance of each amino acid residue at a given cycle in the sequence of the peptide mixture, most importantly at the degenerate positions. For each degenerate position in the peptides of the library, a relative abundance value can then be calculated by dividing the abundance of a particular amino acid residue at that position after library screening (i.e., after peptide binding, release, and separation) by the abundance of the same amino acid residue at that position in the starting library. Thus, the relative abundance (RA) of an amino acid residue Xaa at a degenerate position in a peptide can be defined as:

$$RA = \frac{\text{amount of Xaa in the population of bound peptides}}{\text{amount of Xaa in the oriented degenerate peptide library}}$$

The relative abundance value may be corrected for background contamination using techniques well known in the art.

Amino acid residues which are neither enriched for nor selected against in the population of peptides which bind preferentially to the enzyme (i.e., the preferred peptides) will have a relative abundance of 1.0. Those amino acid residues which are preferred at a particular degenerate position (i.e., residues which are enriched at that position in the preferred peptides) will have a relative abundance greater than 1.0. Those amino acid residues which are not preferred (i.e., residues which are selected against at that position in the preferred peptides) will have a relative abundance less than 1.0. Based upon the relative abundance values for each amino acid residue at a degenerate position, preferred amino acid residues, i.e., amino acid residues with a relative abundance greater than 1.0, can be identified at that position.

As discussed above, if a solid-support bound bead-type library is used, each isolate must be sequenced individually. This requires sequencing of large numbers of isolates and would likely provide only an estimation of the relative abundance of each amino acid residue at a degenerate position. Alternatively, if a method employing a spatially addressable peptide library, such as that described in item 'e' above, is used, the enzyme may be marked with a detectable marker, the library may be treated with a quantity of the enzyme, and then the library may be examined to determine which of the bound peptides are preferred by the enzyme. For example, if the marker is fluorescent, the library may be examined under conditions which cause the marker to fluoresce. By determining the relative luminosity of regions known to display particular peptide sequences, the relative abundance of a particular amino acid an any position of the peptide sequence may be approximated. The approximation will only be as accurate as the measurement of the intensity of the marker, and thus a fluorescence-based technique as set forth here will be subject to a certain amount of qualitative assessment. It will be noted that by using this method, the peptide library may include active subunits which bind *irreversibly* to the enzyme, e.g., halomethyl ketones, sulfonyl fluorides, etc., provided that the peptide library consists of slow-binding inhibitors.

Other methods for assaying the relative binding affinities of an enzyme to a library of compounds are well known in the art and could easily be adapted for use in this invention without departing from the spirit of this invention.

Sequencing peptide sequences having an active subunit which is not an amino acid may require additional manipulations in preparation for sequencing. For example, when the peptide terminates in an aldehyde, it may be desirable to convert the aldehyde to a carboxylic acid function, a function native to peptides, by any of the many methods known in the art, e.g., treatment with sodium chlorite. A method should preferably be chosen that will not oxidize sulfur atoms that may be present in an amino acid at a degenerate position. When the peptide terminates in a boronic acid, the boronic acid may be converted to a carboxylic acid, or may be converted to an aldehyde and further manipulated as set forth above (Brown, H.C. et al. *Pure and Appl. Chem.* **1991**, *63,* 307-316). If a boronic functional group occurs in the middle of a chain, e.g., in place of a carbonyl function of a natural peptide, such functional groups may easily be hydrolyzed to provide two fragments: a C-terminal peptide fragment, and an N-terminal boronic acid fragment which may be processed as set forth above. For the purposes of this invention, it is not necessary to be able to correlate a specific C-terminal peptide fragment to the N-terminal fragment with which it was associated during the assay. It is sufficient simply to know the relative abundance of amino acids at particular positions. In this example, the N-terminal and C-terminal fragments can be differentiated on the basis that the N-terminal set will include boronic acid residues that the C-terminal fragments lack. Other methods of converting boronic acid groups to functionalities suitable for peptide sequencing are known in the art.

Depending on the nature and location of the active subunit, certain methods for preparing the sequence for automatic sequencing may be utilized. For example, as in the case above wherein a boronic acid residue replaces a carboxyl group towards the middle of a peptide chain, hydrolysis may be used to cleave the peptides at this linkage, the N-terminal boronic acid portions may be separated from the C-terminal peptide portions, e.g., based on their differing acidities or their differing affinities for a chromatographic substrate, and the two portions of the sample may be sequenced by an automatic sequencer.

In cases where the active subunit is non-degenerate, it may not be necessary to sequence the active subunit itself; only sequences for the other residues may be needed. Edman degradation, commonly employed in automatic peptide sequencers, is capable of sequencing peptides from the N-terminus (N-terminal sequencing) independent of functionality at the C-terminus. Thus, peptides with an active subunit, for example, including aldehyde or boronic acid functionality, at the C-terminus can still be sequenced using an automatic sequencer. Methods for C-terminal sequencing are known in the art (e.g., U.S. Pat. No. 5,432,092 to Bailey, et al. and U.S. Pat. No. 5,049,507 to Hawke, et al.) and have been incorporated into automatic peptide sequencers. Thus, peptides having a non-degenerate active subunit at the N-terminus can still be sequenced using an automatic peptide sequencer. Furthermore, automatic peptide sequencers are known capable of performing both C- and N-terminal peptide sequencing on the same-sample (e.g., Hewlett Packard's HP 1100 Series LC-based N- and C-terminal protein sequencer). Thus, a peptide library having an active subunit in the interior of the peptide can be sequenced from both ends to determine the relative abundances of the residues at particular positions thereof.

Another method useful for sequencing peptides is mass spectrometry. Mass spectrometry, particularly elecrospray mass spectrometry, can be used to determine fragmentation patterns for peptide molecules. Because fragmentation occurs predominantly at a peptide bond, a fragmentation pattern typically provides atomic masses of fragments wherein fragments of decreasing mass are indicative of the successive loss of amino acid residues in a peptide sequence. In order to differentiate C-terminal cleavage from N-terminal cleavage, it is particularly useful for the members of a peptide library to have the amino acid at either the C-terminus, the N-terminus, or both be labelled with a distinctive isotope pattern. A distinctive isotope pattern can be a pattern indicative of the presence of an element which occurs naturally as at least two isomers in a ratio of between 1:1 and 1:10, preferably 1:1 and 1:5. Elements which exhibit this quality include chlorine, bromine, boron, and selenium. Thus, boronic acid-type peptide libraries where the boronic acid is present at one of the termini are well-suited to this type of analysis. Alternatively or additionally, one of the atoms in a terminal residue of peptides in the library may be enriched substantially completely or, preferably, partially, so that peptide fragments which include the enriched terminus exhibit a distinctive isotope pattern in a mass spectrum. Thus, for example, the terminal carboxy group of a peptide library may be enriched with $^{13}C$, the alpha proton may be enriched with $^{2}H$, etc. As a result, the mass spectra for such compounds will exhibit a series of fragments, each with the readily identifiable, distinctive isotope pattern. By determining the mass difference between successive peaks, the amino acid lost with each fragmentation can be determined, permitting minute quantities of a peptide to be sequenced. An example of peptide sequencing using this technique is presented in Geysen M., Abstracts of Papers of the American Chemical Society 218:144-Anyl , Part 1 Aug. 22 1999. Furthermore, mass spectrometry techniques can be applied to enzyme-bound substrates as well as to substrates which have been separated from the bound enzyme.

Furthermore, LC/MS can be used to permit chromatographic separation of a library of peptides, followed by mass spectrometry to sequence each peptide as it elutes, as described above. Alternatively, a mass spectrometer can be configured, as is well known in the art, to determine a combined mass spectrum for a mixture of compounds, separate the molecules by mass, and determine fragmentation patterns for each of the separate entities. In this way, individual peptide sequences, rather than just relative abundances for each position in a set of peptide sequences, can be determined.

Based upon the relative abundance of different amino acid residues at each degenerate position within the population of bound and sequenced peptides, a preferred amino acid sequence motif for an active site of the enzyme can be determined. The amino acid sequence motif encompasses the degenerate region of the peptides. The particular amino acid residues chosen for the motif at each degenerate position are those which are most abundant at each position. Thus, an amino acid residue(s) with a relative abundance value greater than 1.0 at a particular position can be chosen as the amino acid residue(s) at that position within the amino acid sequence motif. Alternatively, a higher relative abundance value can be used as the basis for inclusion of an amino acid residue to create an even more preferred amino acid sequence motif for an enzyme. For example, an amino acid residue(s) with a relative abundance value equal to or greater than 1.5 at a particular position can be chosen as the amino acid residue(s) at that position within the amino acid sequence motif.

Peptides composed of amino acid residues corresponding to one of the possible residues prescribed by the amino acid sequence motif of an enzyme can be synthesized to create a peptide substrate for the enzyme. Peptides composed of the most preferred amino acid residues of the motif (i.e, amino acid residues having the highest relative abundance at each degenerate position) can be synthesized as optimal peptide substrates for the enzyme.

It should be appreciated that it may be possible to make amino acid substitutions in a substrate peptide of the invention without substantially changing the affinity of the peptide for an enzyme. For example, amino acid substitutions are likely to be possible at a position within the peptide which is relatively insensitive to variations in amino acid residues at that position. Similarly, amino acid substitutions are likely to be possible at a position which can broadly accommodate particular types of amino acids (e.g., hydrophobic or hydrophilic amino acids). The oriented degenerate peptide library used to determine an amino acid sequence motif of the invention represents peptides having every possible amino acid substitution at each degenerate position and the sequences determined from the library screening provide information regarding the effect of substituting a particular amino acid at each degenerate position surrounding an active site. Thus, positions at which substitutions from the defined motif may be possible are readily identifiable from the data obtained from the sequenced bound peptides of the library (i.e., from the relative abundance values for each amino acid residue at each degenerate position). For example, a position which is relatively insensitive to variations in amino acid residues will have many amino acid residues at that position with relative abundance values around 1.0 (i.e., neither substantially selected for or against in the library screening). While one or more amino acid residues at that position may have an RA greater than 1.0 (the value set for inclusion in an amino acid sequence motif of the invention), it may be possible to substitute this residue with an amino acid residue having an RA of 1.0 or slightly less than one at that position without substantially affecting the affinity of the peptide substrate for the protease. For example, for src family kinases, the position at -4 relative to the phosphorylated site (at position 0) can display many amino acids with relative abundance values around 1.0, indicating that this position can accommodate many different amino acids. Likewise, a position which can broadly accommodate, for example, a hydrophobic residue, will have many hydrophobic residues at that position with RA values around 1.0, although only one or a few residues may have the preferred RA value of greater than 1.0.

Accordingly, the invention encompasses a peptide substrate for an enzyme having substantially equal affinity

for the enzyme as a peptide substrate comprising a preferred amino acid sequence motif determined by the methods presented herein. The term "substantially equal affinity" is intended to mean that the peptide has an affinity for the enzyme such that it can function in substantially the same way as a peptide having a preferred amino acid sequence motif presented herein (e.g., the peptide can competitively inhibit the activity of an enzyme against another substrate approximately equivalently to a peptide having a preferred amino acid sequence motif as determined by the methods presented herein). An affinity of a peptide for an enzyme can be defined as a $K_i$ value (i.e., the concentration of peptide needed to inhibit activity toward another substrate by 50%) by competition with other substrates. Preferably, a peptide having substantially equal affinity for an enzyme as a peptide substrate comprising a preferred amino acid sequence motif presented herein has a $K_i$ value no more than 2-fold greater than the $K_i$ of the peptide having the amino acid sequence motif. Other methods for determining the affinities of substrates for enzymes are well known in the literature and may be applied to the invention without departing from the scope thereof.

The peptide substrates of the invention can be incorporated into a larger protein to create a preferred binding site for an enzyme within the protein. Incorporation of the preferred amino acid sequence motif for an enzyme into a protein may be expected to provide a site on the protein for high-affinity binding of that enzyme. For example, when the enzyme is a protease, a preferred amino acid sequence motif for that protease may be incorporated into a protein. This preferred amino acid sequence motif provides a site on the protein which can be expected to be readily and preferentially cleaved by that protease.

The preferred amino acid sequence motif of the specificity subsites of an enzyme, determined by the method of the invention, can also be used to make pseudosubstrates for the enzyme. In certain cases, the pseudosubstrate will combine the active subunit with the preferred amino acid sequence motif. In the particular case of proteases, the pseudosubstrates may comprise peptides having amino acid sequences corresponding to the amino acid sequence motif but in which the scissile bond, ordinarily cleaved by the protease, is replaced by a non-cleavable linkage. Alterations which may render the scissile bond non-cleavable include methylating the nitrogen of the amide, replacing the carbonyl of the amide with $-CH_2$, replacing the nitrogen with $-CH_2-$, and other techniques well known in the art. Analogous alterations suitable for use with other classes of enzymes are well known in the art an may be applied to the present invention without departing from the spirit and scope of this disclosure.

Peptide analogs, peptidomimetics, and other small molecules which can be classified as pseudosubstrates can also be designed based upon the amino acid sequence motifs provided by the invention. The amino acid sequence motifs provided by the invention can be used to design such analogs of the substrates of the invention. It has generally been found that when peptides are added to intact cells or administered *in vivo*, their uptake is inefficient and/or they are degraded. Thus, in situations in which one wants to modulate a signal transduction pathway (for example, for therapeutic purposes *in vivo*), it is desirable to use a peptide analog rather than a peptide. The term "peptide analog" as used herein is intended to include molecules which mimic the chemical structure of a peptide and retain biological properties of the peptide but which are cell membrane permeable. The term "peptide analog" is also intended to include molecules which mimic the chemical structure of a peptide and retain biological properties of the peptide but which are more stable (e.g., are less susceptible to degradation either in the circulation *in vivo* or intracellularly) than the peptide. In the case of peptide analogs of the peptide substrates of the invention, the peptide analog retains the biological property of being able to bind to the enzyme (i.e., occupy the catalytic site of the enzyme). Preferably, the peptide analog is not altered by the enzyme, although a alterable peptide analog may also be useful. Approaches to designing peptide analogs are known in the art. For example, see Farmer, P.S. in Drug Design (E.J. Ariens, ed.) Academic Press, New York, 1980, vol. 10, pp. 119-143; Ball. J.B. and Alewood, P.F. (1990) *J. Mol. Recognition* 3:55; Morgan, B.A. and Gainor, J.A. (1989) *Ann. Rep. Med. Chem.* 24:243; and Freidinger, R.M. (1989) *Trends Pharmacol. Sci.* 10:270.

One type of peptide analog of the peptide substrates of the invention which can be made is a peptide which has methylated amide linkages. This modification of the peptide has been found to increase the membrane permeability of the peptide and increase its stability. Additionally, the rigidity of the peptide may be increased, which may increase the affinity of the peptide analog for the catalytic site of the enzyme relative to the unmodified peptide substrate.

Alternatively, retro-inverso peptides may be useful in the methods disclosed herein. Retro-inverso peptides according to the invention may be prepared in analogy to the following example. If the preferred amino acid sequence motif is $H_2N$-(L)Arg-(L)Ala-(L)Lys-$CO_2$-, a corresponding retro-inverso peptide is -$O_2C$-(D)Arg-(D)Ala-(D)Lys-$NH_2$-. Conceptually, such sequences are the reverse of the parent sequence (retro) and use amino acids enantiomeric (inverso) to those of the parent sequence. One advantage of retro-inverso peptides is that they are resistant to enzymatic activity, although they bind to enzymes with affinities similar to those of the parent sequences. This characteristic makes them useful as inhibitors of enzymes to which they bind. Furthermore, such retro-inverso peptides are useful in pharmaceutical preparations because they are resistant to the enzymatic degradation which gives standard peptides short half-lives in the body.

Non-peptidyl small molecules may also be useful as inhibitors according to the invention. Such small molecules may be designed rationally based upon the known structure of a preferred amino acid binding motif and tailoring the structural characteristics of the small molecule to be analogous to that the structural characteristics of that motif.

Alternatively, such small molecules may be generated combinatorially and subjected to such assays as those disclosed above for peptide substrates. Other methods which may be used to determine small molecule substrates useful for the invention are intended to be encompassed by the scope of this invention.

The peptide substrates and pseudosubstrates of the invention can be used to inhibit the activity of an enzyme. The binding of a substrate by a particular enzyme can be inhibited by contacting the enzyme with an inhibitory amount of a peptide substrate or pseudosubstrate for the enzyme, as provided by the invention. Inhibition of an enzyme may be useful in *in vitro* studies or in *in vivo* therapeutic treatments. Enzymes play a role in virtually all signal transduction pathways, including those involved in cell cycle control, immunological responses, transcriptional activation and cell development. Moreover, enzymes play a role in cell transformation. The ability to inhibit the activity of enzymes thus provides a means by which to modulate a wide variety of cellular responses. Novel therapeutic agents composed of or based upon the peptide substrates, pseudosubstrates and peptide analogs of the invention may thus have applications in a wide variety of disease situations. In some cases, it may be desirable to inhibit the activity of an enzyme to modulate a signal transduction pathway. As an example, VanX is a D-Ala-D-Ala dipeptidase that is essential for vancomycin resistance in *Enterococcus faecium*. Inhibition of this dipeptidase may prove useful in circumventing vancomycin resistance in bacteria that might otherwise render an infection immune to known anti-bacterial pharmaceuticals.

The peptide substrates of the invention are useful for detecting and quantitating the activity of enzymes *in vitro*. For example, a solution can be contacted with a substrate of the invention to detect the presence of a particular enzyme in the solution. For example, the solution can be a cell extract or a column fraction of a partially purified enzyme. For *in vitro* assays, the peptide substrate can be immobilized on a solid support (e.g., a bead) to allow for easy separation of the peptide from other reaction components (e.g., [$\gamma$-$^{32}$P]-ATP).

For example, the ability to rapidly detect and inhibit proteases is important in maintaining the stability of protein solutions. If contaminating proteases are not detected and inhibited, proteolysis can lead to decreased yields throughout purification and instability during long-term storage. Very minute quantities of protease are sufficient to cause degradation.

In one aspect, the library of peptidyl inhibitors includes compounds represented by the general Formula I:

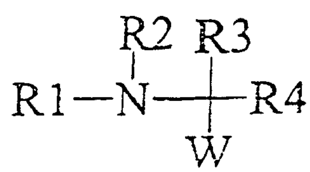

wherein

W represents $BY_1Y_2$, perhaloalkyl (e.g., perfluoroalkyl), or $C(=O)R_5$
$R_1$ represents a C-terminally linked amino acid residue or amino acid analog, or a C-terminally linked peptide or peptide analog, or

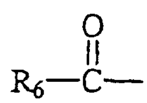, 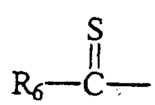, 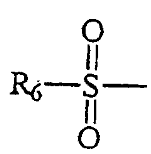;

$R_2$ represents hydrogen, an alkyl, an alkenyl, an alkynyl, $-(CH_2)m-R_7$, $-(CH_2)m-OH$, - $(CH_2)m$-O-alkyl, $-(CH_2)m$-O-alkenyl, $-(CH_2)m$-O-alkynyl, $-(CH_2)m-O-C(=O)$-alkyl,$-(CH_2)m-O-C(=O)$-alkenyl, $-(CH_2)m-O-C(=O)$-alkynyl $-(CH_2)m-O-C(=O)-(CH_2)m-R_7$;
$R_3$ and $R_4$ each represent a hydrogen, an alkyl, an alkenyl, an alkynyl, $-(CH_2)m-R_7$,$-(CH_2)n-OH$, $-(CH_2)n$-O-alkyl, $-(CH_2)n$-O-alkenyl, $-(CH_2)n$-O-alkynyl, $-(CH_2)n-O-(CH_2)_m-R_7$, $-(CH_2)n-SH$, $-(CH_2)n$-S-alkyl, $-(CH_2)n$-S-alkenyl, $-(CH_2)n$-S-alkynyl, $-(CH_2)n-S-(CH_2)m-R_7$, an $\alpha$-carbon linked side chain of an amino acid or an amino acid analog.

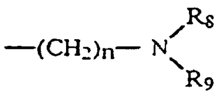, 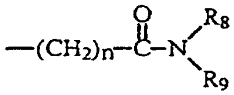, 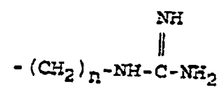, 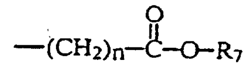

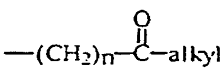, 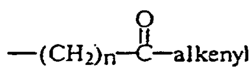, 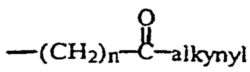, or 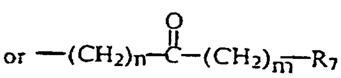

or, $R_2$ and $R_3$ taken together can complete a ring having from 4 to 8 atoms in the ring structure,
or, provided $R_2$ and $R_3$ are not taken together forming a ring, $R_3$ and $R_4$ taken together can complete a ring having from 3 to 8 atoms in the ring structure,
$Y_1$ and $Y_2$ can independently or together be hydroxyl, or a group capable of being hydrolyzed to a hydroxyl group, including cyclic derivatives where $Y_1$ and $Y_2$ are connected via a ring having from 5 to 8 atoms in the ring structure,
$R_5$ represents H, halomethyl, trifluoromethyl, amido, ester, ketone, carboxyl, an alkyl, an alkenyl, an alkynyl, $-(CH_2)m-R_7$, $-(CH_2)n-OH$, $-(CH_2)n$-O-alkyl, $-(CH_2)n$-O-alkenyl,$-(CH_2)n$-O-alkynyl, $-(CH_2)n-O-(CH_2)m-R_7$, $-(CH_2)n-SH$, $-(CH_2)n$-S-alkyl, $-(CH_2)n$-S-alkenyl, $-(CH_2)n$-S-alkynyl, $-(CH_2)n-S-(CH_2)m-R_7$, $-CH_2O-R_{10}$,
$R_6$ represents hydrogen, a halogen, a alkyl, a alkenyl, a alkynyl, an aryl, $-(CH_2)_m-R_7$,$-(CH_2)_m-OH$, $-(CH_2)_m$-O-alkyl, $-(CH_2)_m$-O-alkenyl, $-(CH_2)_m$-O-alkynyl, $-(CH_2)_m-O-(CH_2)_m-R_7$, $-(CH_2)_m-SH$, $-(CH_2)_m$-S-alkyl, $-(CH_2)_m$-S-alkenyl, $-(CH_2)_m$-S-alkynyl, $-(CH_2)_m-S-(CH_2)_m-R_7$,

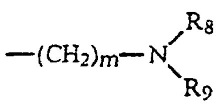, 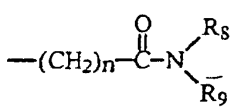, 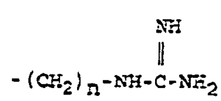, 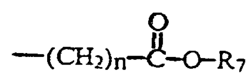

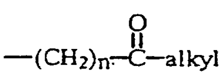, 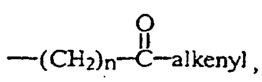, 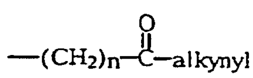, or 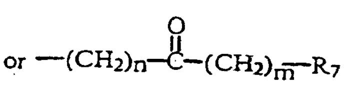

$R_7$ represents an aryl, a cycloalkyl a cycloalkenyl, or a heterocycle;
$R_8$ and $R_9$ each independently represent hydrogen, alkyl, alkenyl, $-(CH_2)_m-R_7$,-C(=O)-alkyl, -C(=O)-alkenyl, -C(=O)-alkynyl, $-C(=O)-(CH_2)_m-R_7$,
or $R_8$ and $R_9$ taken together with the N atom to which they are attached complete a heterocyclic ring having from 4 to 8 atoms in the ring structure;
$R_{10}$ represents represents a C-terminally linked amino acid residue or amino acid analog, or a C-terminally linked peptide or peptide analog,

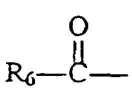, 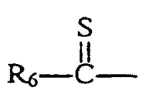, or 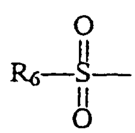;

m is zero or an integer in the range of 1 to 8; and
n is an integer in the range of 1 to 8.

In certain embodiments, W represents a boronic acid (including boronic acid esters), an aldehyde, a perhaloalkyl or perfluoroalkyl (e.g., trifluoromethyl, pentafluoroethyl), a trifluoromethylketone, a halomethylketone (e.g., $-C(=O)CX_3$, $-C(=O)CHX_2$, or $-C(=O)CH_2X$, wherein X, independently for each occurrence, is F, Cl, or Br), an alpha-ketoester, an alpha-diketone, an alpha-ketoamide, or an alpha-ketoacid.

In a certain preferred embodiment, $R_2$ and $R_4$ each represent H, and $R_3$, independently for each occurrence in the compound library, represents a naturally occuring amino acid sidechain.

Also deemed as equivalents are any compounds which can be hydrolytically converted into any of the aforementioned compounds including boronic acid esters and halides, and carbonyl equivalents including acetals, hemiacetals, ketals, and hemiketals, and cyclic dipeptide analogs.

In certain other embodiments, the subject inhibitors are boronic acid analogs of amino acids. Exemplary boronic acid derived libraries for use in the method include a plurality of inhibitors represented in the general Formula II:

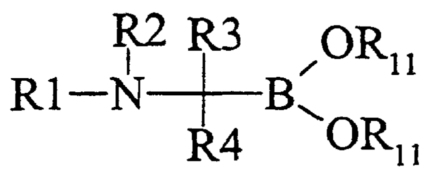

wherein

$R_1$, $R_2$, $R_3$ and $R_4$ are as defined above; and
$R_{11}$, independently for each occurrence, represents hydrogen, a alkyl, or a pharmaceutically acceptable salt, or both $R_{11}$ taken together with the O-B-O atoms to which they are attached complete a heterocyclic ring having from 5 to 8 atoms in the ring structure;

In other embodiments, the subject inhibitor library includes aldehyde analogs. Exemplary aldehyde-derived inhibitors of the present invention are represented by the general Formula III:

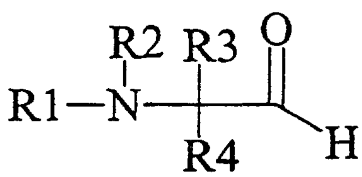

wherein

$R_1$, $R_2$, $R_3$ and $R_4$ are as defined above.

In certain embodiments, the library of subject inhibitors includes inhibitors represented by the general Formula IIIa:

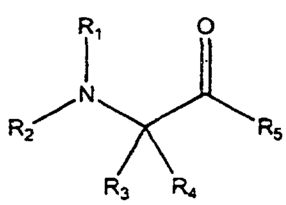

wherein

$R_1$ represents a C-terminally linked amino acid residue or amino acid analog, or a C-terminally linked peptide or peptide analog, or

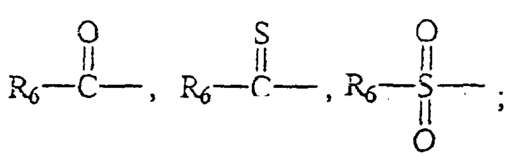

$R_2$ represents hydrogen, an alkyl, an alkenyl, an alkynyl, $-(CH_2)m-R_7$, $-(CH_2)m-OH$, - $(CH_2)m-O$-alkyl, $-(CH_2)m-O$-alkenyl, $-(CH_2)m-O$-alkynyl, $-(CH_2)m-O-C(=O)$-alkyl,$-(CH_2)m-O-C(=O)$-alkenyl, $-(CH_2)m-O-C(=O)$-alkynyl $-(CH_2)m-O-C(=O)-(CH_2)m-R_7$;

$R_3$ and $R_4$ each represent a hydrogen, an alkyl, an alkenyl, an alkynyl, $-(CH_2)m-R_7$,$-(CH_2)n-OH$, $-(CH_2)n-O$-alkyl, $-(CH_2)n-O$-alkenyl, $-(CH_2)n-O$-alkynyl, $-(CH_2)n-O-(CH_2)m-R_7$, $-(CH_2)n-SH$, $-(CH_2)n-S$-alkyl, $-(CH_2)n-S$-alkenyl, $-(CH_2)n-S$-alkynyl, $-(CH_2)n-S-(CH_2)m-R_7$, an $\alpha$-carbon linked side chain of an amino acid or an amino acid analog,

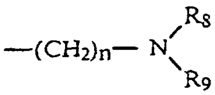 , 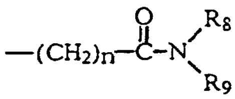 , 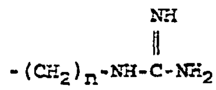 , 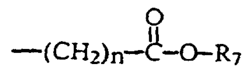

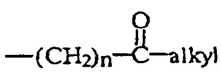 , 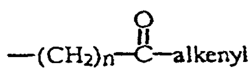 , 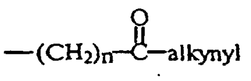 , or 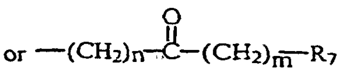

or, $R_2$ and $R_3$ taken together can complete a ring having from 4 to 8 atoms in the ring structure,

or, provided $R_2$ and $R_3$ are not taken together to form a ring, $R_3$ and $R_4$ taken together can complete a ring having from 3 to 8 atoms in the ring structure; and

$R_5$ represents halomethyl, trifluoromethyl, amido, ester, ketone, or carboxyl.

In yet further embodiments, the subject inhibitor library includes halo-methyl ketone analogs. Exemplary inhibitors of this class include compounds represented by the general Formula IV:

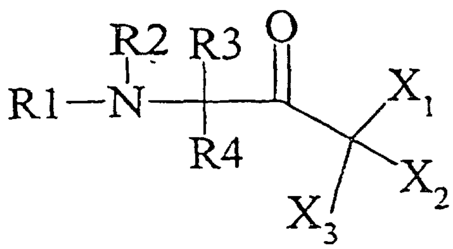

wherein

$R_1$, $R_2$, $R_3$ and $R_4$ are as defined above; and

$X_1$, $X_2$ and $X_3$ each represent a hydrogen or a halogen.

In yet other embodiments, subject analogs include a modified tyrosine residue. Such analogs are particularly useful as kinase and phosphatase inhibitors. Accordingly, another aspect of the present invention provides a boronophenylalanine moiety in a wide range of compounds, e.g., as described in U.S. Patents No. 5,776,902 and 5,580,979. In one embodiment, the analog is a boronophenylalanine (boronoF) or a phosphonophenylalanine (phosphonoF) represented by the general Formula V:

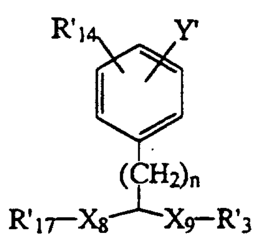

wherein

Y' represents a substitution at one of the meta, ortho or para positions of the phenyl moiety, Y' being a borono given by the general formula

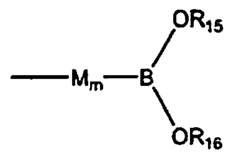

or a phosphono, given by the general formula

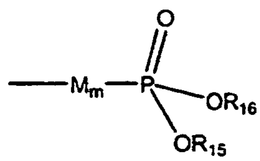

$R_{15}$ and $R_{16}$ each independently represent hydrogen, a lower alkyl, or a pharmaceutically acceptable salt, or $R_{15}$ and $R_{16}$ taken together with the O-B-O or O-P-O atoms to which they are attached complete a heterocyclic ring having from 5 to 8 atoms in the ring structure;
$R'_{14}$ is absent or represents one or more substituents at remaining ring positions, which substituents are selected from halogens, lower alkyls, lower alkoxys, a hydroxyl, amino, nitro, thiol, amines, imines, amides, carbonyls, carboxyls, silyls, ethers, thioethers, sulfonyls, selenoethers, ketones, aldehydes, esters, or $-(CH_2)_m-R_7$, $-CF_3$, -CN, or the like;
M, independently for each occurrence, represents a substituted or unsubsituted methylene group, e.g., $CH_2$, CHOH, CHF, CHMe, $CHNH_2$, etc., preferably $CH_2$ or CHOH;
$X_8$ and $X_9$ each, independently, represent a methylene, an ethylene, an acetylene, an amine, a carbonyl, a phosphonyl, a sulfur, an oxygen, or a selenium;
$R'_{17}$ is absent, or represents hydrogen, halogens, alkyls, alkenyls, alkynyls, hydroxyl, amino, nitro, thiol, amines, imines, amides, phosphoryls, phosphonates, phosphines, carbonyls, carboxyls, silyls, ethers, thioethers, sulfonyls, selenoethers, ketones, aldehydes, esters, or $-(CH_2)_m-R_7$ or $R'_{17}$ represents an amino acid residue or peptide condensed with $X_8$;
$R'_3$ is absent, or represents hydrogen, halogens, alkyls, alkenyls, alkynyls, hydroxyl, amino, nitro, thiol, amines, imines, amides, phosphoryls, phosphonates, phosphines, carbonyls, carboxyls, silyls, ethers, thioethers, sulfonyls, selenoethers, ketones, aldehydes, esters, or $-(CH_2)_m-R_7$ or $R'_3$ represents an amino acid residue or peptide condensed with $X_9$;
$R_7$ represents an aryl, a cycloalkyl, a cycloalkenyl, a heterocycle or a polycycle;
m is zero or an integer in the range of 1 to 8; and
n is 1, 2 or 3.

In certain embodiments, the compounds of formula V will have at least one methylene between the phenyl ring and the moiety Y', e.g., m=1, or m=2. In preferred embodiments, n=1. In certain embodiments, Y' is attached to the para position of the phenyl ring.

As above, the substitutions of formula V can chosen so as to provide a cross-linking agent for covalently or non-covalently immobilizing the mimetic on an insoluble matrix, e.g., to purify protein kinases and phosphatases, e.g., tyrosine kinases, tyrosine phosphatase, etc.. The substituents can also provide a detectable label, such as radiolabel or fluorescent label, or biotin, streptavidin or the like, for detecting the presence of the mimetic.

In preferred embodiments, the compound of formula V is provided as a peptide or peptide analog which is equivalent in size to a dipeptide or larger, e.g., the compound is a peptide of peptide analog of 2 or greated amino acids or amino acid analogs. Preferably, length of the peptide or peptide analog is in the range of 2 to 30 amino acid residues, more preferably from 2 to 20 or 4 to 20 residues in length, and even more preferably from 4 to 10 residues in length.

As is apparent from the present disclosure, non-hydrolyzable peptide analogs can be generated which incoporate the compound of formula V. For illustrative purposes, peptide analogs of the present invention can be generated using, in addition to the benzodiazepines described above, substituted gama lactam rings (Garvey et al. in *Peptides: Chemistry and Bioloy*, G.R. Marshall ed., ESCOM Publisher: Leiden, Netherlands, 1988, p123), C-7 mimics (Huffman

et al. in *Peptides: Chemistry and Biologyy*, G.R. Marshall ed., ESCOM Publisher: Leiden, Netherlands, 1988, p. 105), keto-methylene pseudopeptides (Ewenson et al. (1986) *J Med Chem* 29:295; and Ewenson et al. in *Peptides: Structure and Function* (Proceedings of the 9th American Peptide Symposium) Pierce Chemical Co. Rockland, IL, 1985), β-turn dipeptide cores (Nagai et al. (1985) *Tetrahedron Lett* 26:647; and Sato et al. (1986) *J Chem Soc Perkin Trans* 1:1231), β-aminoalcohols (Gordon et al. (1985) *Biochem Biophys Res Commun* 126:419; and Dann et al. (1986) *Biochem Biophys Res Commun* 134:71), diaminoketones (Natarajan et al. (1984) *Biochem Biophys Res Commun* 124:141), and methyleneamino-modifed (Roark et al. in *Peptides: Chemistry and Biology*, G.R. Marshall ed., ESCOM Publisher: Leiden, Netherlands, 1988, p134). Also, see generally, Session III: Analytic and synthetic methods, in *Peptides: Chemistry and Biology*, G.R. Marshall ed., ESCOM Publisher: Leiden, Netherlands, 1988).

Analogously, phosphatases and kinases that naturally modify serine or threonine residues can be targeted by including in a peptide analog a borono or phosphono-modified serine or threonine analog, such as a residue of Formula VI:

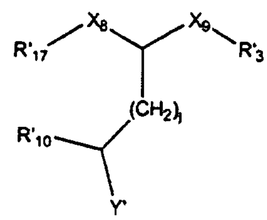

wherein

$X_8$, $X_9$, $R'_3$, $R'_{17}$, Y', m, M, and n are defined as above,
j is an integer from 0-3, and
$R'_{10}$ is H, OH, $NH_2$, halogen, or lower alkyl, preferably H or Me.

In certain embodiments, m is 1 or 2, preferably 1. In certain embodiments, j is 0 or 1, preferably 0.

In an exemplary embodiment, a peptidomimetic can be a retro-inverso peptide. To illustrate, a boronoF-EEI peptide can be generated as a retro-inverso analog:

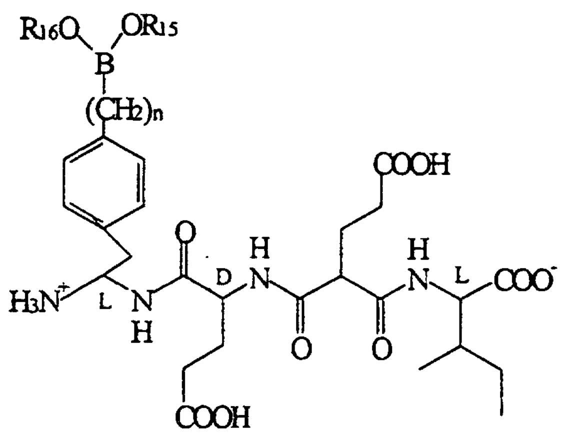

Such retro-inverso analogs can be made according to the methods known in the art, such as that described by the Sisto et al. U.S. Patent 4,522,752. For example, the illustrated retro-inverso analog can be generated as follows. The geminal diamine corresponding to the boronophenylalanine analog is synthesized by treating a borono-protected (e.g. as the 2,6-dichlorobenzyl ether) N-Boc-L-boronoPhe with ammonia under HOBT-DCC coupling conditions to yield N-Boc-L-boronophenylalanineamide, and then effecting a Hofmann-type rearrangement with I,I-bis-(trifluoroacetoxy) iodobenzene (TIB), as described in Radhakrishna et al. (1979) *J. Org. Chem.* 44:1746. The product amine salt is then coupled to a side-chain protected (e.g., as the benzyl ester) N-Fmoc D-Glu residue under standard conditions to yield the pseudodipeptide. The Fmoc (fluorenylmethoxycarbonyl) group is removed with piperidine in dimethylformamide, and the resulting amine is trimethylsilylated with bistrimethylsilylacetamide (BSA) before condensation with suitably alkylated, side-chain protected derivative of Meldrum's acid, as described in U.S. Patent 5,061,811 to Pinori et al., to yield the retro-inverso tripeptide analog. The pseudotripeptide is then coupled with L-Ile under standard conditions to

give the protected tetrapeptide analog. The protecting groups are removed to release the final product, which is purified by HPLC.

In another illustrative embodiment, the peptidomimetic can be derived as a retro-enatio analog of the peptide, such as the exemplary retro-enantio peptide analog:

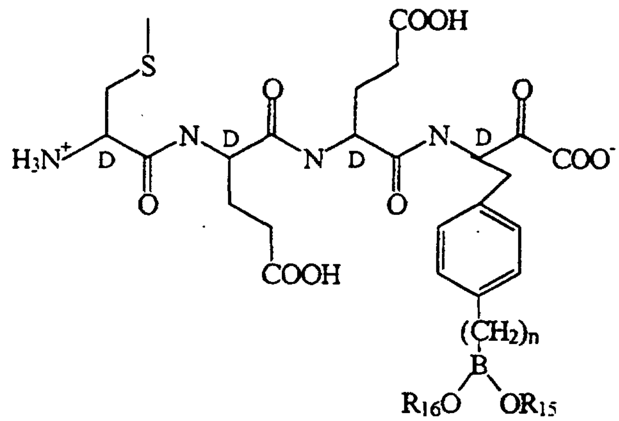

Retro-enantio analogs such as this can be synthesized using D-enatiomers of the subject borono-analogs or phosphono-analogs and commercially available D-amino acids and standard solid- or solution-phase peptide-synthesis techniques. For example, in a preferred solid-phase synthesis method, a suitably amino-protected (t-butyloxycarbonyl, Boc) D-boronophenylalanine residue is covalently bound to a solid support such as chloromethyl resin. The boronyl can be protected, for example, as described in the examples below. The resin is washed with dichloromethane (DCM), and the BOC protecting group removed by treatment with TFA in DCM. The resin is washed and neutralized, and the next Boc-protected D-amino acid (D-Glu; the side -chain carboxylate is protected as, for example, the benzyl ester) is introduced by coupling with diisopropylcarbodiimide. The resin is again washed, and the cycle repeated for each of the remaining amino acids in turn (D-Glu, D-Met). When synthesis of the protected retro-enantio peptide is complete, the protecting groups are removed and the peptide cleaved from the solid support by treatment with hydrofluoric acid/anisole/dimethyl sulfide/thioanisole. The final product is purified by HPLC to yield the pure retro-enantio analog.

In still another illustrative embodiment, trans-olefin derivatives can be made with the subject analogs, e.g., by incorporating phosphonoF or boronoF moieties. For example, an exemplary olefin analog is:

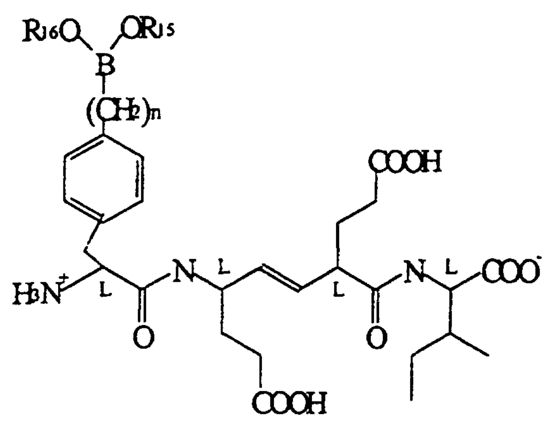

The trans-olefin analog of a boronophenylalanine-containing peptide can be synthesized according to the method of Y.K. Shue et al. (1987) *Tetrahedron Letters* 28:3225, which scheme is shown in Figure 8 of U.S. Patent No. 5,776,902. Variations in the procedure may be necessary according to the nature of the reagents used and the structure of the target compound, but any such variations will be routine and obvious to one of skill in the art.

It is further possible couple the peptide analogs synthesized by the above method to other peptide analogs, to make peptide analogs with several olefinic functionalities in place of amide functionalities. For example, pseudodipeptides corresponding to pTyr-Glu and Glu-Ile could be made and then coupled together by standard techniques to yield an analog of the tetrapeptide pYEEI which has two olefinic bonds between residues.

Still another class of peptidomimetic boronophenylalanine and phosphonophenylalanine derivatives include the phosphonate derivatives, such as:

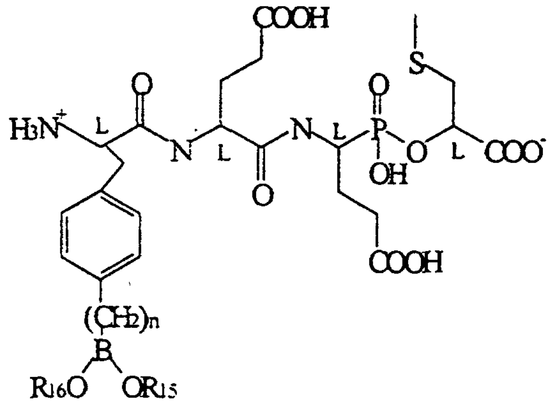

The synthesis of such phosphonate derivatives can be adapted from known synthesis schemes. See, for example, Loots et al. in *Peptides: Chemistry and Biology*, (Escom Science Publishers, Leiden, 1988, p. 118); Petrillo et al. in *Peptides: Structure and Function* (Proceedings of the 9th American Peptide Symposium, Pierce Chemical Co. Rockland, IL, 1985).

As used herein, the definition of each expression, e.g., alkyl, m, n, etc., when it occurs more than once in any structure, is intended to be independent of its definition elsewhere in the same structure. The invention is not intended to by limited by substitution of compounds listed above when such subsitution includes functional groups known in the art suitable for incorporation into the compounds listed above.

In another aspect, the present invention provides pharmaceutically acceptable compositions which comprise a therapeutically-effective amount of one or more enzyme inhibitors, such as identified by the method described above, or peptidomimetics thereof, formulated together with one or more pharmaceutically acceptable carriers (additives) and/or diluents for use in the treatment of a medical condition. As described in detail below, the pharmaceutical compositions of the present invention may be specially formulated for administration in solid or liquid form, including those adapted for the following: (1) oral administration, for example, drenches (aqueous or non-aqueous solutions or suspensions), tablets, boluses, powders, granules, pastes for application to the tongue; (2) parenteral administration, for example, by subcutaneous, intramuscular or intravenous injection as, for example, a sterile solution or suspension; (3) topical application, for example, as a cream, ointment or spray applied to the skin; or (4) intravaginally or intravectally, for example, as a pessary, cream or foam.

The phrase "therapeutically effective amount" as used herein means that amount of a compound, material, or composition comprising an enzyme inhibitor according to the present invention which is effective for producing some desired therapeutic effect by inhibiting the action of a particular enzyme.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable carrier" as used herein means a pharmaceutically acceptable material, composition or vehicle, such as a liquid or solid filler, diluent, excipient, solvent or encapsulating material, involved in carrying or transporting the subject peptidomimetic agent from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

As set out above, certain embodiments of the present enzyme inhibitors may contain a basic functional group, such as amino or alkylamino, and are thus capable of forming pharmaceutically acceptable salts with pharmaceutically acceptable acids. The term "pharmaceutically acceptable salts" in this respect, refers to the relatively non-toxic, inorganic and organic acid addition salts of enzyme inhibitors. These salts can be prepared *in situ* during the final isolation and purification of the peptides, peptidomimetics, or other small molecules of the invention, or by separately reacting a purified peptide, peptidomimetic, or other small molecule of the invention in its free base form with a suitable organic or inorganic acid, and isolating the salt thus formed. Representative salts include the hydrobromide, hydrochloride, sulfate, bisulfate, phosphate, nitrate, acetate, valerate, oleate, palmitate, stearate, laurate, benzoate, lactate, tosylate, citrate, maleate, fumarate, succinate, tartrate, napthylate, mesylate, glucoheptonate, lactobionate, and laurylsulphonate salts and the like. (See, for example, Berge et al. (1977) "Pharmaceutical Salts", *J. Pharm. Sci*. 66:1-19)

In other cases, the compounds of the present invention may contain one or more acidic functional groups and, thus, are capable of forming pharmaceutically acceptable salts with pharmaceutically acceptable bases. The term "pharmaceutically acceptable salts" in these instances refers to the relatively non-toxic, inorganic and organic base addition salts of an enzyme inhibitor. These salts can likewise be prepared *in situ* during the final isolation and purification of the peptides or peptidomimetics, or by separately reacting the purified compound in its free acid form with a suitable base, such as the hydroxide, carbonate or bicarbonate of a pharmaceutically acceptable metal cation, with ammonia, or with a pharmaceutically acceptable organic primary, secondary or tertiary amine. Representative alkali or alkaline earth salts include the lithium, sodium, potassium, calcium, magnesium, and aluminum salts and the like. Representative organic amines useful for the formation of base addition salts include ethylamine, diethylamine, ethylenediamine, ethanolamine, diethanolamine, piperazine and the like. (See, for example, Berge et al., *supra*)

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Formulations of the present invention include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient which can be combined with a carrier material to produce a single dosage form will generally be that amount of the enzyme inhibitor which produces a therapeutic effect. Generally, out of one hundred percent, this amount will range from about 1 percent to about ninety-nine percent of active ingredient, preferably from about 5 percent to about 70 percent, most preferably from about 10 percent to about 30 percent.

Methods of preparing these formulations or compositions include the step of bringing into association a compound of the present invention with the carrier and, optionally, one or more accessory ingredients. In general, the formulations are prepared by uniformly and intimately bringing into association a peptide or peptidomimetic of the present invention with liquid carriers, or finely divided solid carriers, or both, and then, if necessary, shaping the product.

Formulations of the invention suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A peptide, peptidomimetic, or small molecule of the present invention may also be administered as a bolus, electuary or paste.

In solid dosage forms of the invention for oral administration (capsules, tablets, pills, dragees, powders, granules and the like), the active ingredient is mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered peptide, peptidomimetic, or small molecule moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active GGPTase inhibitor(s), may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions of the invention for rectal or vaginal administration may be presented as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active inhibitor.

Formulations of the present invention which are suitable for vaginal administration also include pessaries, tampons, creams, gels, pastes, foams or spray formulations containing such carriers as are known in the art to be appropriate.

Dosage forms for the topical or transdermal administration of a peptide, peptidomimetic, or small molecule of this invention include powders, sprays, ointments, pastes, creams, lotions, gels, solutions, patches and inhalants. The active compound may be mixed under sterile conditions with a pharmaceutically-acceptable carrier, and with any preservatives, buffers, or propellants which may be required.

The ointments, pastes, creams and gels may contain, in addition to an active GGPTase inhibitor, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this invention, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of a compound of the present invention to the body. Such dosage forms can be made by dissolving or dispersing the peptide, peptidomimetic, or small molecule in the proper medium. Absorption enhancers can also be used to increase the flux of the drug across the skin. The rate of such flux can be controlled by either providing a rate controlling membrane or dispersing the peptide, peptidomimetic, or small molecule in a polymer matrix or gel.

Opthalmic formulations, eye ointments, powders, solutions and the like, are also contemplated as being within the scope of this invention.

Pharmaceutical compositions of this invention suitable for parenteral administration comprise one or more peptides, peptidomimetics, or small molecules of the invention in combination with one or more pharmaceutically acceptable sterile isotonic aqueous or nonaqueous solutions, dispersions, suspensions or emulsions, or sterile powders

which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and other antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming microencapsuled matrices of the subject peptides, peptidomimetics, or small molecule in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissue.

When the compounds of the present invention are administered as pharmaceuticals, to humans and animals, they can be given per se or as a pharmaceutical composition containing, for example, 0.1 to 99.5% (more preferably, 0.5 to 90%) of active ingredient in combination with a pharmaceutically acceptable carrier.

The preparations of the present- invention may be given orally, parenterally, topically, or rectally. They are of course given by forms suitable for each administration route. For example, they are administered in tablets or capsule form, by injection, inhalation, eye lotion, ointment, suppository, etc. administration by injection, infusion or inhalation; topical by lotion or ointment; and rectal by suppositories. Oral administration is preferred.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticulare, subcapsular, subarachnoid, intraspinal and intrastemal injection and infusion.

The phrases "systemic administration," "administered systemically," "peripheral administration" and "administered peripherally" as used herein mean the administration of a compound, drug or other material other than directly into the central nervous system, such that it enters the patient's system and, thus, is subject to metabolism and other like processes, for example, subcutaneous administration.

Regardless of the route of administration selected, the enzyme inhibitors useful in the subject method may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of this invention may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular enzyme inhibitor employed, or the ester, salt or amide thereof, the route of administration, the time of administration, the rate of excretion of the particular compound being employed, the duration of the treatment, other drugs, compounds and/or materials used in combination with the particular inhibitor employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved.

In general, a suitable daily dose of a potent enzyme inhibitor, e.g., having an $EC_{50}$ in the range of 1 mM to sub-nanomolar, will be that amount of the compound which is the lowest dose effective to produce a therapeutic effect. Such an effective dose will generally depend upon the factors described above. Generally, intravenous, intracerebroventricular and subcutaneous doses of the compounds of this invention for a patient, when used for the desired effects, will range from about 0.0001 to about 1000 mg per kilogram of body weight per day, though preferably 0.5 to 300 mg per kilogram.

If desired, the effective daily dose of the active inhibitor may be administered as two, three, four, five, six or more sub-doses administered separately at appropriate intervals throughout the day, optionally, in unit dosage forms.

In a preferred embodiment, the agent is formulated for oral administration, as for example in the form of a solid tablet, pill, capsule, caplet or the like (collectively hereinafter "tablet") or an aqueous solution or suspension. In a preferred embodiment of the tablet form of the agent, the tablets are preferably formulated such that the amount of agent (or agents) provided in 20 tablets, if taken together, would provide a dose of at least the median effective dose (ED50), e.g., the dose at which at least 50% of individuals exhibited the quantal effect of inhibition of enzyme action (e.g., a statistically significant reduction in activity). More preferably, the tablets are formulated such that the total amount of agent (or agents) provided in 10, 5, 2 or 1 tablets would provide at least an ED50 dose to a patient (human or non-human mammal). In other embodiments, the amount of agent (or agents) provided in 20, 10, 5 or 2 tablets taken in a 24 hour time period would provide a dosage regimen providing, on average, a mean plasma level of the agent(s) of at least the ED50 concentration (the concentration for 50% of maximal effect of, e.g., inhibiting cell growth), though preferably less than 100 times the ED50, and even more preferably less than 10 or 5 times the ED50. In preferred embodiments, a single dose of tablets (1-20 tablets) provides about 0.25 mg to 1250 mg of an agent(s).

Likewise, the agents can be formulated for parenteral administration, as for example, for subcutaneous, intramuscular or intravenous injection, e.g., the agent can be provided in a sterile solution or suspension (collectively hereinafter "injectable solution"). The injectable solution is preferably formulated such that the amount of agent (or agents) provided in a 200cc bolus injection would provide a dose of at least the median effective dose, though preferably less than 100 times the ED50, and even more preferably less than 10 or 5 times the ED50. More preferably, the injectable solution is formulated such that the total amount of agent (or agents) provided in 100, 50, 25, 10, 5, 2.5, or 1 cc injections would provide an ED50 dose to a patient, and preferably less than 100 times the ED50, and even more preferably less than 10 or 5 times the ED50. In other embodiments, the amount of agent (or agents) provided in a total volume of 100cc, 50, 25, 5 or 2 cc to be injected at least twice in a 24 hour time period would provide a dosage regimen providing, on average, a mean plasma level of the agent(s) of at least the ED50 concentration, though preferably less than 100 times the ED50, and even more preferably less than 10 or 5 times the ED50. In preferred embodiments, a single dose injection provides about .25 mg to 1250 mg of agent.

For continuous intravenous infusion, e.g., drip or push, the agent can be provided in a sterile dilute solution or suspension (collectively hereinafter "i.v. injectable solution"). The i.v. injectable solution is preferably formulated such that the amount of agent (or agents) provided in a 1L solution would provide a dose, if administered over 15 minutes or less, of at least the median effective dose, though preferably less than 100 times the ED50, and even more preferably less than 10 or 5 times the ED50. More preferably, the i.v. injectable solution is formulated such that the total amount of agent (or agents) provided in 1L solution administered over 60, 90, 120 or 240 minutes would provide an ED50 dose to a patient, though preferably less than 100 times the ED50, and even more preferably less than 10 or 5 times the ED50. In preferred embodiments, a single i.v. "bag" provides about 0.25 mg to 5000 mg of agent per liter i.v. solution, more preferably 0.25 mg to 2500 mg, and even more preferably 0.25 mg to 1250 mg.

As discussed above, the preferred agent pharmaceutical preparation, whether for injection or oral delivery (or other route of administration), would provide a dose less than the ED50 for modulation of enzyme activity in the host, more preferably at least 1 order of magnitude less, more preferably at least 2, 3 or 4 orders magnitude less.

An ED50 dose, for a human, is based on a body weight of from 10 lbs to 250 lbs, though more preferably for an adult in the range of 100 to 250 lbs.

## Exemplification

The present invention will now be illustrated by reference to the following example which set forth particularly advantageous embodiments. However, it should be noted tha these embodiments are illustrative and are not to be construed as restricting the invention in any way.

*Preparation of Soluble Peptide Boronic Acid Libraries for LC-MS Analysis*

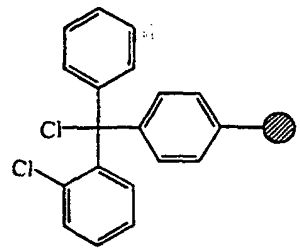

2-Chlorotrityl chloride resin

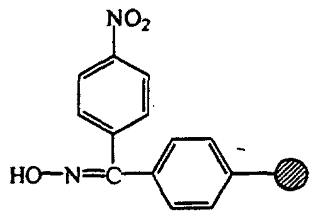

Oxime resin

**X-boroPro**

Step 1: BocX(boc)----{oxime resin}
Step 2: BocX(boc)-boroPro (*released by boroPro*)
Step 3: Remove all protection groups => **X-boroPro**

**X-X-boroPro**

Step 1: Fmoc-X(boc)----{2-ClTrt resin}
Step 2: BocX(boc)-X(boc)----{2-ClTrt resin}
Step 3: BocX(boc)-X(boc) (1%TFA *cleavage*)
Step 4: BocX(boc)-X(boc)----{oxime resin}
Step 5: BocX(boc)-X(boc)-boroPro (*released by boroPro*)
Step 6: Remove all protection groups => **X-X-boroPro**

**X-X-X-boroPro**

Step 1: Fmoc-X(boc)----{2-ClTrt resin}
Step 2: FmocX(boc)-X(boc)----{2-ClTrt resin}
Step 3: BocX(boc)-X(boc)-X(boc)----{2-ClTrt resin}
Step 4: BocX(boc)-X(boc)-X(boc) (1%TFA *cleavage*)
Step 5: BocX(boc)-X(boc)-X(boc)----{oxime resin}
Step 6: BocX(boc)-X(boc)-X(boc)-boroPro (*released by boroPro*)
Step 7: Remove all protection groups => **X-X-X-boroPro**

**X-X-X-X-boroPro**

Step 1: Fmoc-X(boc)----{2-ClTrt resin}
Step 2: FmocX(boc)-X(boc)----{2-ClTrt resin}
Step 3: FmocX(boc)-X(boc)-X(boc)----{2-ClTrt resin}
Step 4: BocX(boc)-X(boc)-X(boc)-X(boc)----{2-ClTrt resin}

Step 5: BocX(boc)-X(boc)-X(boc)-X(boc) (*cleavage*)
Step 6: BocX(boc)-X(boc)-X(boc)-X(boc)----{oxime resin}
Step 7: BocX(boc)-X(boc)-X(boc)-X(boc)-boroPro (*released by boroPro*)
Step 8: Remove all protection groups => **X-X-X-X-boroPro**

The embodiments described above are intended to be exemplary, and are not intended to limit the scope of the invention thereto.

## Claims

1. A method for determining a preferred amino acid sequence motif for an active site of an enzyme, comprising:

   a) contacting the enzyme with an oriented degenerate peptide library comprising an active subunit at a fixed non-degenerate position under conditions which permit the active subunit to interact with the active site of the enzyme;
   b) allowing the enzyme to bind peptides within the oriented degenerate peptide library;
   c) separating the population of unbound peptides from peptide/enzyme complexes;
   d) releasing the bound peptides from the enzyme;
   e) determining the amino acid sequences of the population of bound peptides; and
   f) determining a preferred amino acid sequence motif for an active site of the enzyme based upon the relative abundance of different amino acid residues at each degenerate position within the population of bound peptides.

2. The method of claim 1, wherein the oriented degenerate peptide library is a soluble synthetic peptide library.

3. The method of claim 1, wherein the oriented degenerate peptide library is a solid support-bound library.

4. The method of claim 1, wherein the oriented degenerate peptide library comprises peptides comprising a formula:

   $$(Xaa)_n\text{-}Yaa\text{-}(Xaa)_m$$

   wherein

   Yaa is an active subunit capable of forming one of a covalent adduct or a tightly bound adduct with a portion of an active site of an enzyme;
   Xaa is any amino acid or amino acid analog; and
   n and m are integers from 0-10 inclusive, such that the sum of m and n is at least two.

5. The method of claim 1, wherein the active subunit at the fixed non-degenerate position of the peptides of the oriented degenerate peptide library is the only active subunit in the peptides.

6. The method of claim 1, wherein the enzyme is a protease and the oriented degenerate peptide library comprises peptides comprising a formula:

   $$(Xaa)_n\text{-}Yaa\text{-}(Xaa)_m$$

   wherein

   Yaa is an active subunit comprising a functional group capable of forming one of a covalent adduct or a tightly bound adduct with a nucleophile;
   Xaa is any amino acid or amino acid analog; and
   n and m are integers from 0-10 inclusive, such that the sum of n and m is at least two.

7. The method of claim 6, wherein Yaa comprises a functional group selected from the group consisting of aldehyde, boronic acid, halomethyl ketone, trifluoromethyl ketone, alpha-keto carboxylic acid derivative, and phosphonic functional groups

8. The method of claim 4, wherein the enzyme is a protease.

9. The method of claim 1, wherein the oriented degenerate peptide library comprises peptides comprising a formula:

$$Z_1 - (Xaa)_n\text{-}Yaa\text{-}(Xaa)_m\text{-}Z_2$$

wherein

Yaa is an active subunit capable of forming one of a covalent adduct or a tightly bound adduct with a portion of an active site of an enzyme;
Xaa is any amino acid or amino acid analog;
n and m are integers from 0-10 inclusive, such that the sum of n and m is at least 2;
$Z_1$ is hydrogen or a peptide having a formula $(Xaa)_a$ wherein Xaa is any non-degenerate amino acid or amino acid analog and a is an integer from 1-15 inclusive; and
$Z_2$ is hydrogen or a peptide having a formula $(Xaa)_b$ wherein Xaa is any non-degenerate amino acid or amino acid analog and b is an integer from 1-15 inclusive.

**10.** The method of claim 9, wherein a and b are integers from 1-10 inclusive.

**11.** The method of claim 9, wherein a and b are integers from 1-5 inclusive.

**12.** The method of claim 1, wherein the oriented degenerate peptide library comprises peptides comprising a formula:

$$Xaa_1\text{-}[Xaa_1]_n\text{-}Xaa_1\text{-}Yaa$$

wherein

$Xaa_1$, independently for each occurrence, represents any amino acid or amino acid analog;
n is an integer from 0 to 20; and
Yaa is an active subunit capable of forming one of a covalent adduct or a tightly bound adduct with a portion of an active site of an enzyme.

**13.** The method of claim 12, wherein n is an integer between 0 and 10.

**14.** The method of claim 1, wherein the active subunit forms at least one of a covalent bond or a tightly bound complex with a portion of the active site of the enzyme.

**15.** The method of claim 1, wherein the enzyme is a protein phosphatase.

**16.** The method of claim 1, wherein the preferred amino acid sequence motif for the active site of the enzyme is determined by calculating a relative abundance (RA) value for each amino acid residue (Xaa) at each degenerate position, wherein RA is determined by a formula:

$$RA = \frac{\text{amount of Xaa in the population of selected peptides}}{\text{amount of Xaa in the oriented degenerate peptide library}}$$

and selecting amino acid residues that have a relative abundance value of greater than 1.0 at a degenerate position for inclusion at a position corresponding to the degenerate position in the preferred amino acid sequence motif.

**17.** The method of claim 1, further comprising, preparing a peptidomimetic corresponding to said determined preferred amino acid sequence motif.

**18.** The method of claim 1, wherein the library comprises an active subunit having a structure of Formula I:

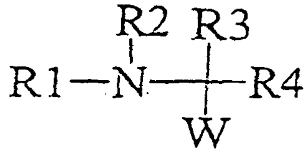

wherein

W represents $BY_1Y_2$, perhaloalkyl, or $C(=O)R_5$;

$R_1$ represents a C-terminally linked amino acid residue, or a C terminally linked peptide or peptide analog, or

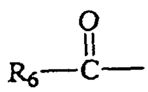 , 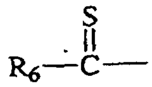 , 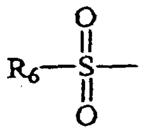 ;

$R_2$ represents hydrogen, an alkyl, an alkenyl, an alkynyl, $-(CH_2)_m-R_7$, $-(CH_2)_m-OH$, $-(CH_2)_m$-O-alkyl, $-(CH_2)_m$-O-alkenyl, $-(CH_2)_m$-O-alkynyl, $-(CH_2)_m$-O-C(=O)-alkyl, $-(CH_2)_m$-O-C(=O)-alkenyl, $-(CH_2)_m$-O-C(=O)-alkynyl $-(CH_2)_m-O-C(=O)-(CH_2)_m-R_7$;

$R_3$ and $R_4$ each represent a hydrogen, an alkyl, an alkenyl, an alkynyl, $-(CH_2)_m-R_7$, $-(CH_2)_n-OH$, $-(CH_2)_n$-O-alkyl,$-(CH_2)_n$-O-alkenyl, $-(CH_2)_n$-O-alkynyl, $-(CH_2)_n-O-(CH_2)_m-R_7$,$-(CH_2)_n-SH$, - $(CH_2)_n$-S-alkyl, $-(CH_2)_n$-S-alkenyl, $-(CH_2)_n$-S-alkynyl, $-(CH_2)_n-S-(CH_2)_m-R_7$, an $\alpha$-carbon linked side chain of an amino acid or an amino acid analog,

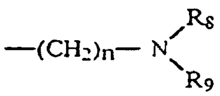 , 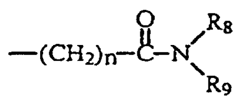 , 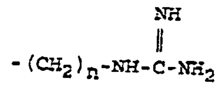 , 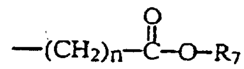

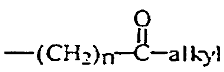 , 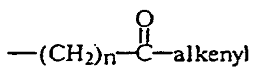 , 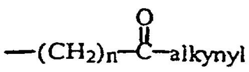 , or 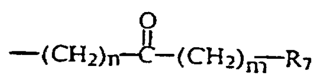

or, $R_2$ and $R_3$ taken together can complete a ring having from 4 to 8 atoms in the ring structure,

or, provided $R_2$ and $R_3$ are not taken together to form a ring, $R_3$ and $R_4$ taken together can complete a ring having from 3 to 8 atoms in the ring structure,

$Y_1$ and $Y_2$ independently or together are hydroxyl or a group capable of being hydrolyzed to a hydroxyl group, including cyclic derivatives where $Y_1$ and $Y_2$ are connected via a ring having from 5 to 8 atoms in the ring structure, or one or $Y_1$ and $Y_2$ represents a bond to an amino group of an amino acid residue,

$R_5$ represents H, halomethyl, trifluoromethyl, amido, ester, ketone, carboxyl, an alkyl, an alkenyl, an alkynyl, $-(CH_2)_m-R_7$, $-(CH_2)_n-OH$, $-(CH_2)_n$-O-alkyl, $-(CH_2)_n$-O-alkenyl, $-(CH_2)_n$-O-alkynyl, $-(CH_2)_n-O-(CH_2)_m-R_7$, $-(CH_2)_n-SH$, - $(CH_2)_n$-S-alkyl, $-(CH_2)_n$-S-alkenyl, $-(CH_2)_n$-S-alkynyl,$-(CH_2)_n-S-(CH_2)_m-R_7$, $-CH_2O-R_{10}$,

$R_6$ represents hydrogen, a halogen, a alkyl, a alkenyl, a alkynyl, an aryl, $-(CH_2)_m-R_7$, $-(CH_2)_m-OH$, $-(CH_2)_m$-O-alkyl,$-(CH_2)_m$-O-alkenyl, $-(CH_2)_m$-O-alkynyl, $-(CH_2)_m-O-(CH_2)_m-R_7$,$-(CH_2)_m-SH$, $-(CH_2)_m$-S-alkyl, $-(CH_2)_m$-S-alkenyl, $-(CH_2)_m$-S-alkynyl, $-(CH_2)_m-S-(CH_2)_m-R_7$,

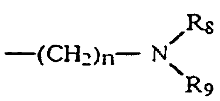 , 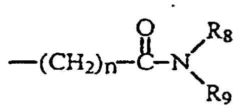 , 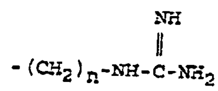 , 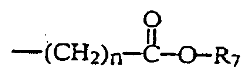

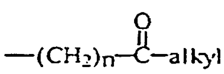 , 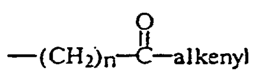 , 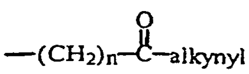 , or 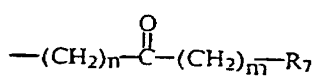

$R_7$ represents an aryl, a cycloalkyl, a cycloalkenyl, or a heterocycle;

$R_8$ and $R_9$ each independently represent hydrogen, alkyl, alkenyl, $-(CH_2)_m-R_7$, -C(=O)-alkyl, -C(=O)-alkenyl, -C(=O)-alkynyl, $-C(=O)-(CH_2)_m-R_7$,

or $R_8$ and $R_9$ taken together with the N atom to which they are attached complete a heterocyclic ring having from 4 to 8 atoms in the ring structure;

$R_{10}$ represents a C-terminally linked amino acid residue or amino acid analog, or a C-terminally linked peptide or peptide analog, or ;

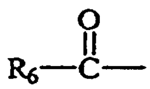 , 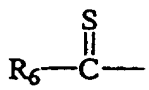 , 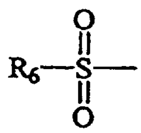

m is zero or an integer in the range of 1 to 8; and

n is an integer in the range of 1 to 8.

**19.** The method of claim 18, wherein W represents a boronic acid, an aldehyde, a perhaloalkyl or perfluoroalkyl, a trifluoromethylketone, a halomethylketone, an alpha-ketoester, an alpha-diketone, an alpha-ketoamide, or an alpha-ketoacid.

**20.** The method of claim 18, wherein $R_2$ and $R_4$ both represent H, and $R_3$, independently for each occurrence in the compound library, represents a naturally occurring amino acid sidechain.

**21.** The method of claim 18, wherein W represents $BY_1Y_2$, and $Y_1$ and $Y_2$ independently or together are hydroxyl, or a group capable of being hydrolyzed to a hydroxyl group, including cyclic derivatives where $Y_1$ and $Y_2$ are connected via a ring having from 5 to 8 atoms in the ring structure.

**22.** The method of claim 18, wherein $R_1$ represents a C-terminally linked amino acid residue or amino acid analog, and $R_2$ represents H.

**23.** The method of claim 1, wherein the library comprises a plurality of peptides represented in the general Formula II:

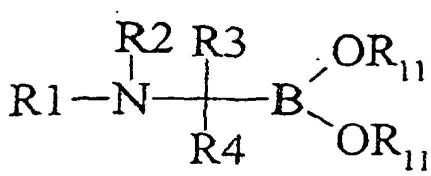

wherein

$R_1$ represents a C-terminally linked amino acid residue or amino acid analog, or a C terminally linked peptide or peptide analog, or

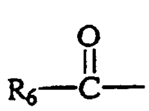 , 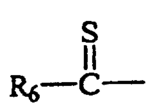 , 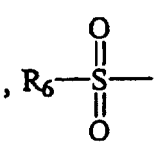

$R_2$ represents hydrogen, an alkyl, an alkenyl, an alkynyl, $-(CH_2)_m-R_7$, $-(CH_2)_m-OH$, $-(CH_2)_m-O-alkyl$, $-(CH_2)_m-O-alkenyl$, $-(CH_2)_m-O-alkynyl$, $-(CH_2)_m-O-C(=O)-alkyl$, $-(CH_2)_m-O-C(=O)-alkenyl$, $-(CH_2)_m-O-C(=O)-alkynyl$, $-(CH_2)_m-O-C(=O)-(CH_2)_m-R_7$;

$R_3$ and $R_4$ each represent a hydrogen, an alkyl, an alkenyl, an alkynyl, $-(CH_2)_m-R_7$, $-(CH_2)_n-OH$, $-(CH_2)_n-O-alkyl$,$-(CH_2)_n-O-alkenyl$, $-(CH_2)_n-O-alkynyl$, $-(CH_2)_n-O-(CH_2)_m-R_7$,$-(CH_2)_n-SH$, $-(CH_2)_n-S-alkyl$, $-(CH_2)_n-S-alkenyl$, $-(CH_2)_n-S-alkynyl$, $-(CH_2)_n-S-(CH_2)_m-R_7$, an $\alpha$-carbon linked side chain of an amino acid or an amino acid analog,

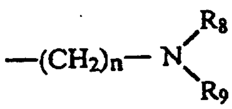, 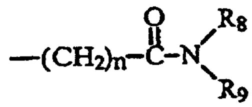, 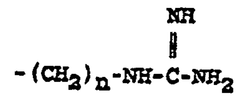, 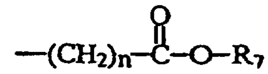

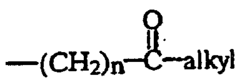, 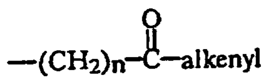, 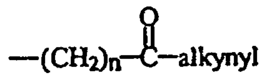, or 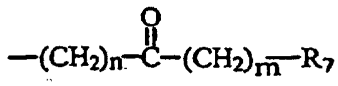

or, $R_2$ and $R_3$ taken together can complete a ring having from 4 to 8 atoms in the ring structure,

or, provided $R_2$ and $R_3$ are not taken together to form a ring, $R_3$ and $R_4$ taken together can complete a ring having from 3 to 8 atoms in the ring structure, and

$R_{11}$, independently for each occurrence, represents hydrogen, a alkyl, or a pharmaceutically acceptable salt, or both $R_{11}$ taken together with the O-B-O atoms to which they are attached complete a heterocyclic ring having from 5 to 8 atoms in the ring structure.

**24.** The method of claim 23, wherein $R_{11}$ represents H in both occurrences.

**25.** The method of claim 23, wherein $R_1$ represents a C-terminally linked amino acid residue or amino acid analog and $R_2$ is H.

**26.** The method of claim 1, wherein the peptide library includes peptides represented by the general Formula III:

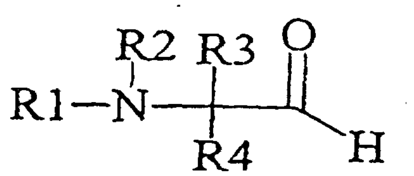

(III)

wherein

$R_1$ represents a C-terminally linked amino acid residue or amino acid analog, or a C terminally linked peptide or peptide analog, or

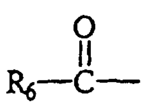, 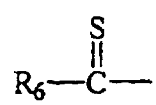, 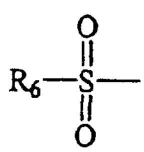

$R_2$ represents hydrogen, an alkyl, an alkenyl, an alkynyl, $-(CH_2)_m-R_7$, $-(CH_2)_m-OH$, $-(CH_2)_m-O-alkyl$, $-(CH_2)_m-O-alkenyl$, $-(CH_2)_m-O-alkynyl$, $-(CH_2)_m-O-C(=O)-alkyl$, $-(CH_2)_m-O-C(=O)-alkenyl$, $-(CH_2)_m-O-C(=O)-alkynyl$ $-(CH_2)_m-O-C(=O)-(CH_2)_m-R_7$; and

$R_3$ and $R_4$ each represent a hydrogen, an alkyl, an alkenyl, an alkynyl, $-(CH_2)_m-R_7$, $-(CH_2)_n-OH$, $-(CH_2)_n-O-alkyl$,$-(CH_2)_n-O-alkenyl$, $-(CH_2)_n-O-alkynyl$, $-(CH_2)_n-O-(CH_2)_m-R_7$,$-(CH_2)_n-SH$, - $(CH_2)_n-S-alkyl$, $-(CH_2)_n-S-alkenyl$, $-(CH_2)_n-S-alkynyl$, $-(CH_2)_n-S-(CH_2)_m-R_7$, an $\alpha$-carbon linked side chain of an amino acid or an amino acid analog,

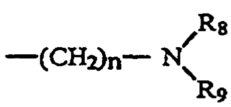 , 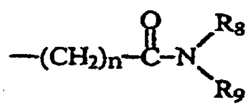 , 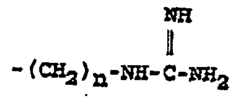 , 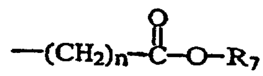

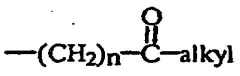 , 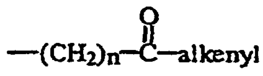 , 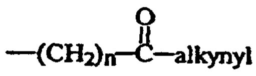 , or 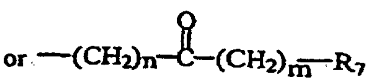

or, $R_2$ and $R_3$ taken together can complete a ring having from 4 to 8 atoms in the ring structure,

or, provided $R_2$ and $R_3$ are not taken together to form a ring, $R_3$ and $R_4$ taken together can complete a ring having from 3 to 8 atoms in the ring structure.

**27.** The method of claim 26, wherein $R_1$ represents a C-terminally linked amino acid residue or amino acid analog and $R_2$ is H.

**28.** The method of claim 1, wherein the peptide library includes peptides represented by the general Formula IIIa:

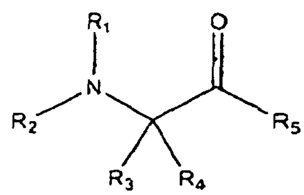

IIIa

wherein

$R_1$ represents a C-terminally linked amino acid residue or amino acid analog, or a C terminally linked peptide or peptide analog, or

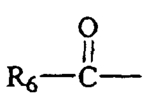 , 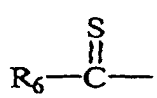 , 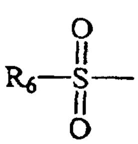

$R_2$ represents hydrogen, an alkyl, an alkenyl, an alkynyl, $-(CH_2)_m-R_7$, $-(CH_2)_m-OH$, $-(CH_2)_m-O-alkyl$, $-(CH_2)_m-O-alkenyl$, $-(CH_2)_m-O-alkynyl$, $-(CH_2)_m-O-C(=O)-alkyl$, $-(CH_2)_m-O-C(=O)-alkenyl$, $-(CH_2)_m-O-C(=O)-alkynyl-(CH_2)_m-O-C(=O)-(CH_2)_m-R_7$;

$R_3$ and $R_4$ each represent a hydrogen, an alkyl, an alkenyl, an alkynyl, $-(CH_2)_m-R_7$, $-(CH_2)_n-OH$, $-(CH_2)_n-O-alkyl$,$-(CH_2)_n-O-alkenyl$, $-(CH_2)_n-O-alkynyl$, $-(CH_2)_n-O-(CH_2)_m-R_7$,$-(CH_2)_n-SH$, $-(CH_2)_n-S-alkyl$, $-(CH_2)_n-S-alkenyl$, $-(CH_2)_n-S-alkynyl$, $-(CH_2)_n-S-(CH_2)_m-R_7$, an α-carbon linked side chain of an amino acid or an amino acid analog,

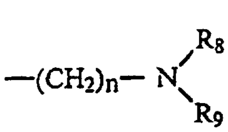 , 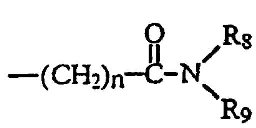 , 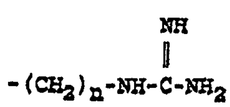 , 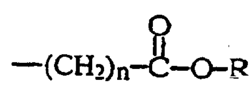

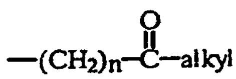, 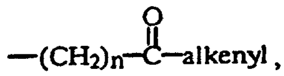, 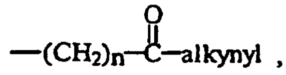, or 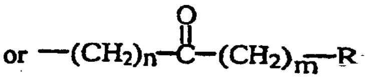

or, $R_2$ and $R_3$ taken together can complete a ring having from 4 to 8 atoms in the ring structure,
or, provided $R_2$ and $R_3$ are not taken together to form a ring, $R_3$ and $R_4$ taken together can complete a ring having from 3 to 8 atoms in the ring structure; and
$R_5$ represents halomethyl, trifluoromethyl, amido, ester, ketone, or carboxyl.

**29.** The method of claim 28, wherein $R_1$ represents a C-terminally linked amino acid residue or amino acid analog and $R_2$ is H.

**30.** The method of claim 1, wherein the peptide library includes compounds represented by the general Formula IV:

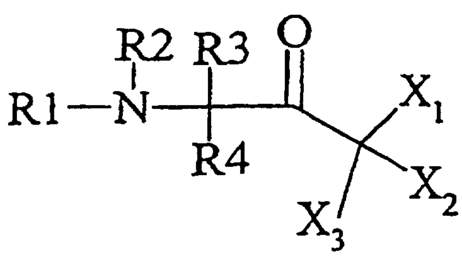

wherein

$R_1$ represents a C-terminally linked amino acid residue or amino acid analog, or a C terminally linked peptide or peptide analog, or

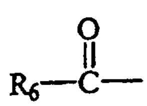, 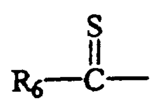, 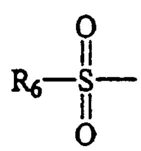

$R_2$ represents hydrogen, an alkyl, an alkenyl, an alkynyl, $-(CH_2)_m-R_7$, $-(CH_2)_m-OH$, $-(CH_2)_m-O-alkyl$, $-(CH_2)_m-O-alkenyl$, $-(CH_2)_m-O-alkynyl$, $-(CH_2)_m-O-C(=O)-alkyl$, $-(CH_2)_m-O-C(=O)-alkenyl$, $-(CH_2)_m-O-C(=O)-alkynyl$ $-(CH_2)_m-O-C(=O)-(CH_2)_m-R_7$;
$R_3$ and $R_4$ each represent a hydrogen, an alkyl, an alkenyl, an alkynyl, $-(CH_2)_m-R_7$, $-(CH_2)_n-OH$, $-(CH_2)_n-O-alkyl$, $-(CH_2)_n-O-alkenyl$, $-(CH_2)_n-O-alkynyl$, $-(CH_2)_n-O-(CH_2)_m-R_7$, $-(CH_2)_n-SH$, $-(CH_2)_n-S-alkyl$, $-(CH_2)_n-S-alkenyl$, $-(CH_2)_n-S-alkynyl$, $-(CH_2)_n-S-(CH_2)_m-R_7$, an $\alpha$-carbon linked side chain of an amino acid or an amino acid analog,

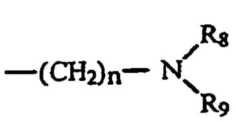, 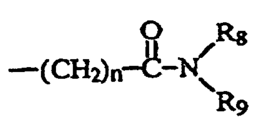, 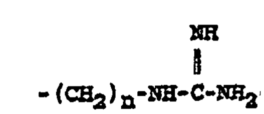, 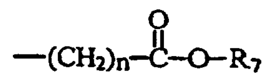

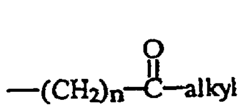, 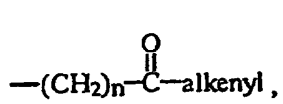, 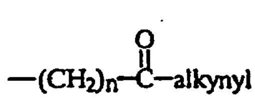, or 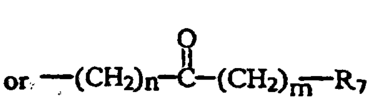

or, $R_2$ and $R_3$ taken together can complete a ring having from 4 to 8 atoms in the ring structure,
or, provided $R_2$ and $R_3$ are not taken together to form a ring, $R_3$ and $R_4$ taken together can complete a ring having from 3 to 8 atoms in the ring structure; and
$X_1$, $X_2$ and $X_3$ each represent a hydrogen or a halogen.

**31.** The method of claim 30, wherein $R_1$ represents a C-terminally linked amino acid residue or amino acid analog and $R_2$ is H.

**32.** The method of claim 30, wherein $X_1$, $X_2$, and $X_3$, independently, each represent H, Cl, Br, or I, or $X_1$, $X_2$, and $X_3$ all represent F.

**33.** The method of claim 1, wherein the library comprises an active subunit represented by the general Formula V:

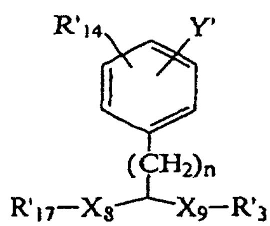

(V)

wherein

Y' represents a substitution at one of the meta, ortho or para positions of the phenyl moiety, Y' being a borono given by the general formula

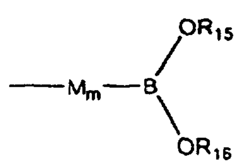

or a phosphono, given by the general formula

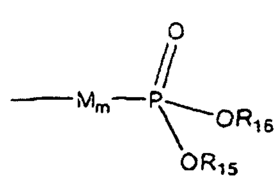

$R_{15}$ and $R_{16}$ each independently represent hydrogen, a lower alkyl, or a pharmaceutically acceptable salt, or $R_{15}$ and $R_{16}$ taken together with the O-B-O or O-P-O atoms to which they are attached complete a heterocyclic ring having from 5 to 8 atoms in the ring structure;
$R'_{14}$ is absent or represents one or more substituents at remaining ring positions, which substituents are selected from halogens, lower alkyls, lower alkoxys, a hydroxyl, amino, nitro, thiol, amines, imines, amides, carbonyls, carboxyls, silyls, ethers, thioethers, sulfonyls, selenoethers, ketones, aldehydes, esters, or $-(CH_2)_m-R_7$, $-CF_3$, -CN, or the like;

M, independently for each occurrence, represents a substituted or unsubstituted methylene group;

$X_8$ and $X_9$ each, independently, represent a methylene, an ethylene, an acetylene, an amine, a carbonyl, a phosphonyl, a sulfur, an oxygen, or a selenium;

$R'_{17}$ is absent, or represents hydrogen, halogens, alkyls, alkenyls, alkynyls, hydroxyl, amino, nitro, thiol, amines, imines, amides, phosphoryls, phosphonates, phosphines, carbonyls, carboxyls, silyls, ethers, thioethers, sulfonyls, selenoethers, ketones, aldehydes, esters, or $-(CH_2)_m-R_7$ or $R'_{17}$ represents an amino acid residue or peptide condensed with $X_8$;

$R'_3$ is absent, or represents hydrogen, halogens, alkyls, alkenyls, alkynyls, hydroxyl, amino, nitro, thiol, amines, imines, amides, phosphoryls, phosphonates, phosphines, carbonyls, carboxyls, silyls, ethers, thioethers, sulfonyls, selenoethers, ketones, aldehydes, esters, or $-(CH_2)_m-R_7$ or $R'_3$ represents an amino acid residue or peptide condensed with $X_9$;

$R_7$ represents an aryl, a cycloalkyl, a cycloalkenyl, a heterocycle or a polycycle;

m is zero or an integer in the range of 1 to 8; and

n is 1, 2 or 3.

**34.** The method of claim 1, wherein the library comprises an active subunit having a residue of Formula VI:

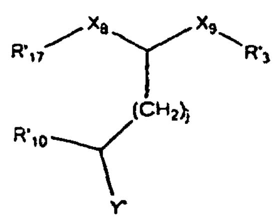

wherein

Y' represents a substitution at one of the meta, ortho or para positions of the phenyl moiety, Y' being a borono given by the general formula

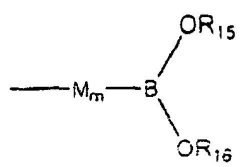

or a phosphono, given by the general formula

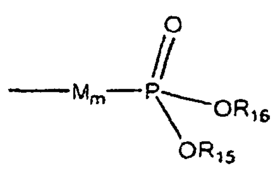

$R_{15}$ and $R_{16}$ each independently represent hydrogen, a lower alkyl, or a pharmaceutically acceptable salt, or $R_{15}$ and $R_{16}$ taken together with the O-B-O or O-P-O atoms to which they are attached complete a heterocyclic ring having from 5 to 8 atoms in the ring structure;

$R'_{14}$ is absent or represents one or more substituents at remaining ring positions, which substituents are selected from halogens, lower alkyls, lower alkoxys, a hydroxyl, amino, nitro, thiol, amines, imines, amides, carbonyls, carboxyls, silyls, ethers, thioethers, sulfonyls, selenoethers, ketones, aldehydes, esters, or $-(CH_2)_m-$

$R_7$,-$CF_3$, -CN, or the like;
M, independently for each occurrence, represents a substituted or unsubstituted methylene group;
$X_8$ and $X_9$ each, independently, represent a methylene, an ethylene, an acetylene, an amine, a carbonyl, a phosphonyl, a sulfur, an oxygen, or a selenium;
$R'_{17}$ is absent, or represents hydrogen, halogens, alkyls, alkenyls, alkynyls, hydroxyl, amino, nitro, thiol, amines, imines, amides, phosphoryls, phosphonates, phosphines, carbonyls, carboxyls, silyls, ethers, thioethers, sulfonyls, selenoethers, ketones, aldehydes, esters, or -$(CH_2)_m$-$R_7$ or $R'_{17}$ represents an amino acid residue or peptide condensed with $X_8$;
$R'_3$ is absent, or represents hydrogen, halogens, alkyls, alkenyls, alkynyls, hydroxyl, amino, nitro, thiol, amines, imines, amides, phosphoryls, phosphonates, phosphines, carbonyls, carboxyls, silyls, ethers, thioethers, sulfonyls, selenoethers, ketones, aldehydes, esters, or -$(CH_2)_m$-$R_7$ or $R'_3$ represents an amino acid residue or peptide condensed with $X_9$;
$R_7$ represents an aryl, a cycloalkyl, a cycloalkenyl, a heterocycle or a polycycle;
m is zero or an integer in the range of 1 to 8;
j is an integer from 0-3, and
$R'_{10}$ is H, OH, $NH_2$, halogen, or lower alkyl, preferably H or Me.

**35.** The method of claim 34, wherein the active subunit binds irreversibly to the enzyme.

**36.** The method of any of claims 1, 4, 6, 9, 12, 16, 22, 25, 27, 29 32 or 33 wherein members of the library are slow-binding inhibitors of the enzyme.

**37.** The method of any of claims 4, 6, 7, 9, or 12, wherein Yaa represents a boronic acid-bearing active subunit.

## Patentansprüche

**1.** Verfahren zur Bestimmung eines bevorzugten Aminosäuresequenz-Motivs für ein aktives Zentrum eines Enzyms, umfassend folgende Stufen:

a) man bringt das Enzym mit einer orientierten, degenerierten Peptidbibliothek, die eine aktive Untereinheit an einer fixierten, nicht-degenerierten Position umfasst, unter Bedingungen in Kontakt, die es der aktiven Untereinheit ermöglichen, mit dem aktiven Zentrum des Enzyms in Wechselwirkung zu treten;
b) man lässt das Enzym Peptide innerhalb der orientierten, degenerierten Peptidbibliothek binden;
c) man trennt die Population von ungebundenen Peptiden von Peptid/Enzym-Komplexen;
d) man setzt die gebundenen Peptide vom Enzym frei;
e) man bestimmt die Aminosäuresequenzen der Population von gebundenen Peptiden; und
f) man bestimmt ein bevorzugtes Aminosäuresequenz-Motiv für ein aktives Zentrum des Enzyms auf der Grundlage der relativen Häufigkeit von verschiedenen Aminosäureresten an jeder degenerierten Position innerhalb der population von gebundenen Peptiden.

**2.** Verfahren nach Anspruch 1, wobei es 'sich bei der orientierten, degenerierten Peptidbibliothek um eine lösliche synthetische Peptidbibliothek handelt.

**3.** Verfahren nach Anspruch 1, wobei es sich bei der orientierten, degenerierten Peptidbibliothek um eine an einen festen Träger gebundene Bibliothek handelt.

**4.** Verfahren nach Anspruch 1, wobei die orientierte, degenerierte Peptidbibliothek Peptide der Formel

$$(Xaa)_n\text{-}Yaa\text{-}(Xaa)_m$$

umfasst, wobei

Yaa eine aktive Untereinheit bedeutet, die zur Bildung eines kovalenten Addukts oder eines fest gebundenen Addukts mit einem Bereich eines aktiven Zentrums eines Enzyms befähigt ist;
Xaa eine Aminosäure oder ein Aminosäureanaloges bedeutet; und
n und m ganze Zahlen mit Werten von 0 bis einschließlich 10 sind, wobei die Summe von m und n mindestens

2 beträgt.

5. Verfahren nach Anspruch 1, wobei die aktive Untereinheit an der fixierten, nicht-degenerierten Position der Peptide der orientierten, degenerierten Peptidbibliothek die einzige aktive Untereinheit in den Peptiden darstellt.

6. Verfahren nach Anspruch 1, wobei es sich bei dem Enzym um eine Protease handelt und die orientierte, degenerierte Peptidbibliothek Peptide der Formel

$$(Xaa)_n\text{-}Yaa\text{-}(Xaa)_m$$

umfasst, wobei

Yaa eine aktive Untereinheit bedeutet, die eine funktionelle Gruppe umfasst, die zur Bildung eines kovalenten Addukts oder eines fest gebundenen Addukts mit einer nukleophilen Substanz befähigt ist;
Xaa eine Aminosäure oder ein Amincsäureanaloges bedeutet; und
n und m ganze Zahlen mit Werten von 0 bis einschließlich 10 sind, wobei die Summe von m und n mindestens 2 beträgt.

7. Verfahren nach Anspruch 6, wobei Yaa eine aus folgender Gruppe ausgewählte funktionelle Gruppe umfasst: funktionelle Aldehyd-, Borsäure-, Halogenmethylketon-, Trifluormethylketon-, alpha-Ketocarbonsäurederivat- und Phosphongruppen.

8. Verfahren nach Anspruch 4, wobei es sich bei dem Enzym um eine Protease handelt.

9. Verfahren nach Anspruch 1, wobei die orientierte, degenerierte Peptidbibliothek Peptide der Formel

$$Z_1\text{-}(Xaa)_n\text{-}Yaa\text{-}(Xaa)_m\text{-}Z_2$$

umfasst, wobei

Yaa eine aktive Untereinheit bedeutet, die zur Bildung eines kovalenten Addukts oder eines fest gebundenen Addukts mit einem Bereich eines aktiven Zentrums eines Enzyms befähigt ist;
Xaa eine Aminosäure oder ein Aminosäureanaloges bedeutet; und
n und m ganze Zahlen mit Werten von 0 bis einschließlich 10 sind, wobei die Summe von n und m mindestens 2 beträgt;
$Z_1$ ein Wasserstoffatom oder ein Peptid der Formel $(Xaa)_a$ bedeutet, wobei Xaa eine beliebige nichtdegenerierte Aminosäure oder Aminosäureanaloges bedeutet und a eine ganze Zahl mit einem Wert von 1 bis einschließlich 15 ist; und
$Z_2$ ein Wasserstoffatom oder ein Peptid der Formel $(Xaa)_b$ bedeutet, wobei Xaa eine beliebige nichtdegenerierte Aminosäure oder Aminosäureanaloges bedeutet und b eine ganze Zahl mit einem Wert von 1 bis einschließlich 15 ist.

10. Verfahren nach Anspruch 9, wobei a und b ganze Zahlen mit einem Wert von 1 bis einschließlich 10 sind.

11. Verfahren nach Anspruch 9, wobei a und b ganze Zahlen mit einem Wert von 1 bis einschließlich 5 sind.

12. Verfahren nach Anspruch 1, wobei die orientierte, degenerierte Peptidbibliothek Peptide der Formel

$$Xaa_1\text{-}[Xaa_1]_n\text{-}Xaa_1\text{-}Yaa$$

umfasst, wobei

$Xaa_1$ (unabhängig für jedes Auftreten) eine beliebige Aminosäure oder Aminosäureanaloges bedeutet;
n eine ganze Zahl mit einem Wert von 0 bis 20 ist; und
Yaa eine aktive Untereinheit bedeutet, die zur Bildung eines kovalenten Addukts oder eines fest gebundenen

Addukts mit einem Bereich eines aktiven Zentrums eines Enzyms befähigt ist.

**13.** Verfahren nach Anspruch 12, wobei n eine ganze Zahl mit einem Wert zwischen 0 und 10 ist.

**14.** Verfahren nach Anspruch 1, wobei die aktive Untereinheit mindestens eine kovalente Bindung oder einen fest gebundenen Komplex mit einem Bereich des aktiven Zentrums des Enzyms bildet.

**15.** Verfahren nach Anspruch 1, wobei es sich beim Enzym um eine Protein-phosphatase handelt.

**16.** Verfahren nach Anspruch 1, wobei das bevorzugte Aminosäuresequenz-Motiv für das aktive Zentrum des Enzyms bestimmt wird, indem man einen Wert für die relative Häufigkeit (RA) für jeden Aminosäurerest (Xaa) an jeder degenerierten Position berechnet, wobei RA durch die folgende Formel bestimmt wird:

$$\text{RA.} = \frac{\text{Menge an Xaa in der Population von ausgewählten Peptiden}}{\text{Menge von Xaa in der orientierten degenerierten Peptidbibliothek}}$$

und Aminosäurereste auswählt, die einen relativen Häufigkeitswert von mehr als 1,0 an einer degenerierten Position aufweisen, zur Einbeziehung an einer Position, die der degenerierten Position im bevorzugten Aminosäuresequenz-Motiv entspricht.

**17.** Verfahren nach Anspruch 1, ferner umfassend das Herstellen eines Peptidomimetikums, das dem ermittelten, bevorzugten Aminosäuresequenz-Motiv entspricht.

**18.** Verfahren nach Anspruch 1, wobei die Bibliothek eine aktive Untereinheit mit einer Struktur der Formel I

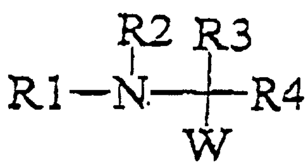

umfasst, wobei

W $BY_1Y_2$, Perhalogenalkyl oder $C(=O)R_5$ bedeutet;
$R_1$ einen C-terminal verknüpften Aminosäurerest oder ein C-terminal verknüpftes Peptid oder Peptidanaloges oder

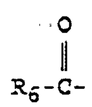 , 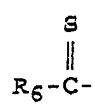 oder 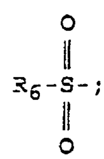 ;

bedeutet;

$R_2$ Wasserstoff, Alkyl, Alkenyl, Alkinyl, $-(CH_2)_m-R_7$,$-(CH_2)m-OH$, $-(CH_2)_m$-O-Alkyl, $-(CH_2)_m$-O-Alkenyl, $-(CH_2)_m$-O-Alkinyl, $-(CH_2)_m$-O-C(=O)-Alkyl, $-(CH_2)_m$-O-C(=O)-Alkenyl oder $-(CH_2)_m$-O-C(=O)-Alkinyl-$(CH_2)_m$-O-C(=O)-$(CH_2)_m-R_7$ bedeutet;
$R_3$ und $R_4$ jeweils Wasserstoff, Alkyl, Alkenyl, Alkinyl, $-(CH_2)_m-R_7$, $-(CH_2)_n-OH$, $-(CH_2)_n$-O-Alkyl, $-(CH_2)_n$-O-Alkenyl, $-(CH_2)_n$-O-Alkinyl, $-(CH_2)_n-O-(CH_2)_m-R_7$, $-(CH_2)_n-SH$, $-(CH_2)_n$-S-Alkyl, $-(CH_2)_n$-S-Alkenyl, $-(CH_2)_n$-S-Alkinyl, $-(CH_2)_n-S-(CH_2)_m-R_7$, eine $\alpha$-Kohlenstoff-verknüpfte Seitenkette einer Aminosäure oder eines Aminosäureanalogen,

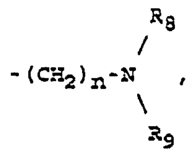 , 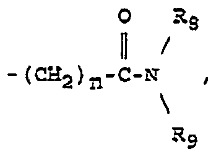 , 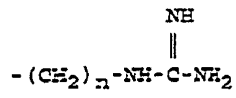 , 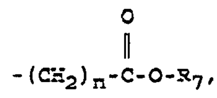

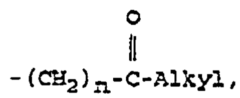 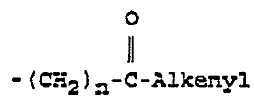 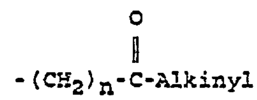

oder

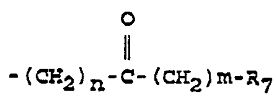

bedeutet oder

$R_2$ und $R_3$ zusammen einen Ring mit 4 bis 8 Atomen in der Ringstruktur vervollständigen können; oder vorausgesetzt, dass $R_2$ und $R_3$ nicht zusammen einen Ring bilden, $R_3$ und $R_4$ zusammen einen Ring mit 3 bis 8 Atomen in der Ringstruktur vervollständigen können;

$Y_1$ und $Y_2$ unabhängig voneinander oder zusammen Hydroxyl oder eine Gruppe, die zu einer Hydroxylgruppe hydrolysiert werden kann, bedeuten, einschließlich cyclische Derivate, wobei $Y_1$ und $Y_2$ über einen Ring mit 5 bis 8 Atomen in der Ringstruktur gebunden sind, oder $Y_1$ und/oder $Y_2$ eine Bindung an eine Aminogruppe eines Aminosäurerestes bedeuten,

$R_5$ H, Halogenmethyl, Trifluormethyl, Amido, Ester, Keton, Carboxyl, Alkyl, Alkenyl, Alkinyl, $-(CH_2)_m-R_7$, $-(CH_2)_n-OH$, $-(CH_2)_n$-O-Alkyl, $-(CH_2)_n$-O-Alkenyl, $-(CH_2)_n$-O-Alkinyl, $-(CH_2)_n-O-(CH_2)_m-R_7$, $-(CH_2)_n-SH$, $-(CH_2)_n$-S-Alkyl, $-(CH_2)_n$-S-Alkenyl, $-(CH_2)_n$-S-Alkinyl, $-(CH_2)_n-S-(CH_2)_m-R_7$ oder $-CH_2O-R_{10}$ bedeutet,

$R_6$ Wasserstoff, Halogen, Alkyl, Alkenyl, Alkinyl, Aryl, $-(CH_2)_m-R_7$, $-(CH_2)_m-OH$, $-(CH_2)_m$-O-Alkyl, $-(CH_2)_m$-O-Alkenyl, $-(CH_2)_m$-O-Alkinyl, $-(CH_2)_m-O-(CH_2)_m-R_7$, $-(CH_2)_m-SH$, $-(CH_2)_m$-S-Alkyl, $-(CH_2)_m$-S-Alkenyl, $-(CH_2)_m$-S-Alkinyl, $-(CH_2)_m-S-(CH_2)_m-R_7$,

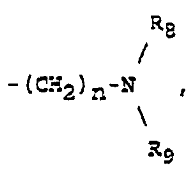 , 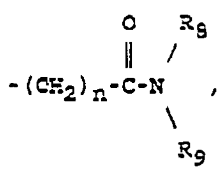 , 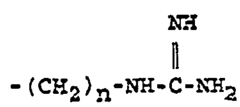 , 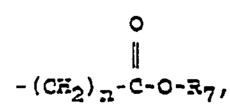

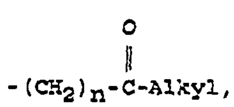 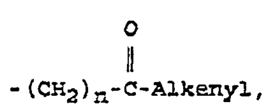 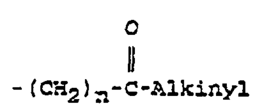

oder

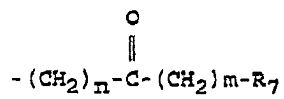

bedeutet;

$R_7$ Aryl, Cycloalkyl, Cycloalkenyl oder Heterocyclyl bedeutet;

$R_8$ und $R_9$ jeweils unabhängig voneinander Wasserstoff, Alkyl, Alkenyl, $-(CH_2)_m-R_7$, -C(=O)-Alkyl, -C(=O)-Alkenyl,-C(=O)-Alkinyl oder $-C(=O)-(CH_2)_m-R_7$ bedeuten

oder $R_8$ und $R_9$ zusammen mit dem N-Atom, an das sie gebunden sind, einen heterocyclischen Ring mit 4 bis 8 Atomen in der Ringstruktur vervollständigen;

$R_{10}$ einen C-terminal verknüpften Aminosäurerest oder Aminosäureanaloges oder ein C-terminal verknüpftes Peptid oder Peptidanaloges oder

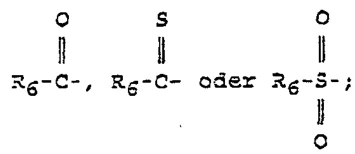

bedeutet;

m 0 oder eine ganze Zahl im Bereich von 1 bis 8 ist; und

n eine ganze Zahl im Bereich von 1 bis 8 ist.

**19.** Verfahren nach Anspruch 18, wobei W eine Boronsäure, ein Aldehyd, ein Perhalogenalkyl oder Perfluoralkyl, ein Trifluormethylketon, ein Halogenmethylketon, einen alpha-Ketoester, ein alpha-Diketon, ein alkpha-Ketoamid oder eine alpha-Ketosäure bedeutet.

**20.** Verfahren nach Anspruch 18, wobei $R_2$ und $R_4$ beide H bedeuten und $R_3$ (unabhängig von jedem Auftreten in der Verbindungsbibliothek) eine natürlich vorkommende Aminosäureseitenkette bedeutet.

**21.** Verfahren nach Anspruch 18, wobei W $BY_1Y_2$ bedeutet und $Y_1$ und $Y_2$ unabhängig voneinander oder zusammen Hydroxyl oder eine Gruppe, die zu einer Hydroxylgruppe hydrolysiert werden kann, bedeuten, einschließlich cyclische Derivate, wobei $Y_1$ und $Y_2$ über einen Ring mit 5 bis 8 Atomen in der Ringstruktur verbunden sind.

**22.** Verfahren nach Anspruch 18, wobei $R_1$ einen C-terminal verknüpften Aminosäurerest oder Aminosäureanaloges bedeutet und $R_2$ H bedeutet.

**23.** Verfahren nach Anspruch 1, wobei die Bibliothek eine Mehrzahl von Peptiden der allgemeinen Formel II

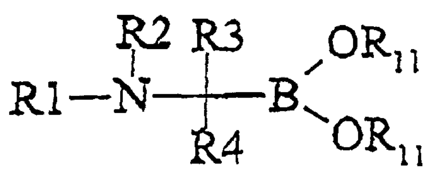

umfasst, wobei

$R_1$ einen C-terminal verknüpften Aminosäurerest oder Aminosäureanaloges oder ein C-terminal verknüpftes Peptid oder Peptidanaloges oder

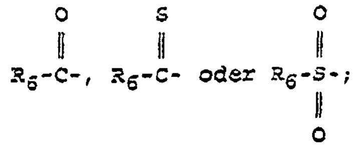

bedeutet;

$R_2$ Wasserstoff, Alkyl, Alkenyl, Alkinyl, $-(CH_2)m-R_7$,$-(CH_2)_m-OH$, $-(CH_2)_m$-O-Alkyl, $-(CH_2)_m$-O-Alkenyl, $-(CH_2)_m$-O-Alkinyl, $-(CH_2)_m-O-C(=O)$-Alkyl, $-(CH_2)_m-O-C(=O)$-Alkenyl oder $-(CH_2)_m-O-C(=O)$-Alkinyl-$(CH_2)_m-O-C(=O)-(CH_2)_m-R_7$ bedeutet;

$R_3$ und $R_4$ jeweils Wasserstoff, Alkyl, Alkenyl, Alkinyl, $-(CH_2)_m-R_7$, $-(CH_2)_n-OH$, $-(CH_2)_n$-O-Alkyl, $-(CH_2)_n$-O-Alkenyl, $-(CH_2)_n$-O-Alkinyl, $-(CH_2)_n-O-(CH_2)_m-R_7$, $-(CH_2)_n-SH$, $-(CH_2)_n$-S-Alkyl, $-(CH_2)_n$-S-Alkenyl, $-(CH_2)_n$-S-Alkinyl, $-(CH_2)_n-S-(CH_2)_m-R_7$, eine $\alpha$-Kohlenstoff-verknüpfte Seitenkette einer Aminosäure oder eines Aminosäureanalogen,

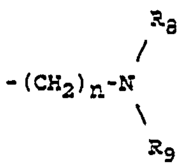 , 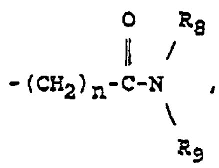 , 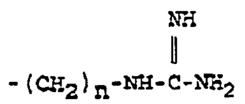 , 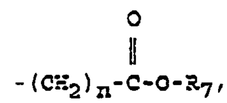

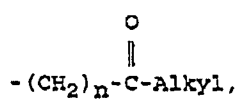 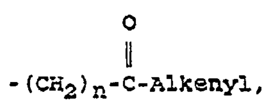 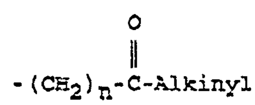

oder

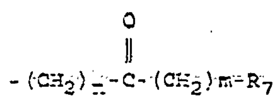

bedeutet oder

$R_2$ und $R_3$ zusammen einen Ring mit 4 bis 8 Atomen in der Ringstruktur vervollständigen können;

oder vorausgesetzt, dass $R_2$ und $R_3$ nicht zusammen einen Ring bilden, $R_3$ und $R_4$ zusammen einen Ring mit 3 bis 8 Atomen in der Ringstruktur vervollständigen können; und

$R_{11}$ (unabhängig für jedes Auftreten) Wasserstoff, Alkyl oder ein pharmazeutisch verträgliches Salz bedeutet, oder beide Reste $R_{11}$ zusammen mit den O-B-O-Atomen, an die sie gebunden sind, einen heterocyclischen Ring mit 5 bis 8 Atomen in der Ringstruktur vervollständigen.

**24.** Verfahren nach Anspruch 23, wobei $R_{11}$ in beiden Fällen H bedeutet.

**25.** Verfahren nach Anspruch 23, wobei $R_1$ einen C-terminal verknüpften Aminosäurerest oder Aminosäureanaloges bedeutet und $R_2$ H bedeutet.

**26.** Verfahren nach Anspruch 1, wobei die Peptidbibliothek Peptide der allgemeinen Formel III

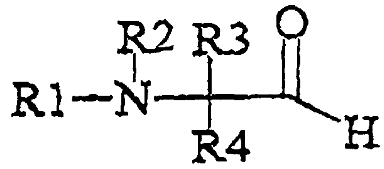

(III)

umfasst, wobei

$R_1$ einen C-terminal verknüpften Aminosäurerest oder ein Aminosäureanaloges oder ein C-terminal verknüpftes Peptid oder Peptidanaloges oder

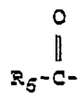, 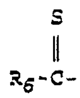 oder 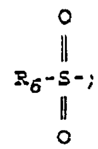;

bedeutet;

$R_2$ Wasserstoff, Alkyl, Alkenyl, Alkinyl, $-(CH_2)_m-R_7$,$-(CH_2)_m-OH$, $-(CH_2)_m-O$-Alkyl, $-(CH_2)_m-O$-Alkenyl, $-(CH_2)_m-O$-Alkinyl, $-(CH_2)_m-O-C(=O)$-Alkyl, $-(CH_2)_m-O-C(=O)$-Alkenyl oder $-(CH_2)_m-O-C(=O)$-Alkinyl$-(CH_2)_m-O-C(=O)-(CH_2)_m-R_7$ bedeutet;

$R_3$ und $R_4$ jeweils Wasserstoff, Alkyl, Alkenyl, Alkinyl, $-(CH_2)_m-R_7$, $-(CH_2)_n-OH$, $-(CH_2)_n-O$-Alkyl, $-(CH_2)_n-O$-Alkenyl, $-(CH_2)_n-O$-Alkinyl, $-(CH_2)_n-O-(CH_2)_m-R_7$, $-(CH_2)_n-SH$, $-(CH_2)_n-S$-Alkyl, $-(CH_2)_n-S$-Alkenyl, $-(CH_2)_n-S$-Alkinyl, $-(CH_2)_n-S-(CH_2)_m-R_7$, eine $\alpha$-Kohlenstoff-verknüpfte Seitenkette einer Aminosäure oder eines Aminosäureanalogen,

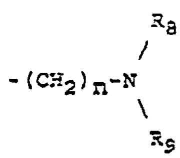, 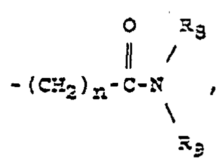, 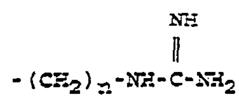, 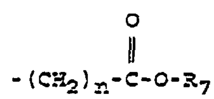,

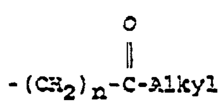, 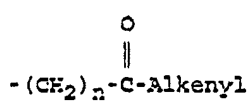, 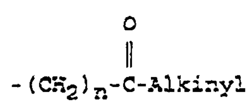

oder

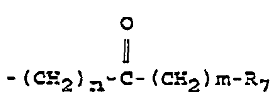

bedeutet; oder

$R_2$ und $R_3$ zusammen einen Ring mit 4 bis 8 Atomen in der Ringstruktur vervollständigen können;

oder vorausgesetzt, dass $R_2$ und $R_3$ nicht zusammen einen Ring bilden, $R_3$ und $R_4$ zusammen einen Ring mit 3 bis 8 Atomen in der Ringstruktur vervollständigen können.

**27.** Verfahren nach Anspruch 26, wobei $R_1$ einen C-terminal verknüpften Aminosäurerest oder Aminosäureanaloges

bedeutet und $R_2$ H bedeutet.

**28.** Verfahren nach Anspruch 1, wobei die Peptidbibliothek Peptide der allgemeinen Formel IIIa

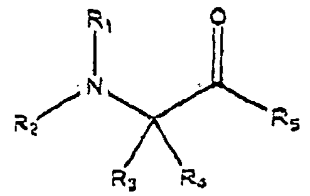

IIIa

umfasst, wobei

$R_1$ einen C-terminal verknüpften Aminosäurerest oder oder Aminosäureanaloges oder ein C-terminal verknüpftes Peptid oder Peptidanaloges oder

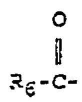, 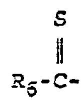 oder 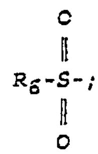;

bedeutet;

$R_2$ Wasserstoff, Alkyl, Alkenyl, Alkinyl, $-(CH_2)_m-R_7$,$-(CH_2)_m-OH$, $-(CH_2)_m-O-Alkyl$, $-(CH_2)_m-O-Alkenyl$, $-(CH_2)_m-O-Alkinyl$, $-(CH_2)_m-O-C(=O)-Alkyl$, $-(CH_2)_m-O-C(=O)-Alkenyl$ oder $-(CH_2)_m-O-C(=O)-Alkinyl-(CH_2)_m-O-C(=O)-(CH_2)_m-R_7$ bedeutet;

$R_3$ und $R_4$ jeweils Wasserstoff, Alkyl, Alkenyl, Alkinyl, $-(CH_2)_m-R_7$, $-(CH_2)_n-OH$, - $(CH_2)_n-O-Alkyl$, $-(CH_2)_n-O-Alkenyl$, $-(CH_2)_n-O-Alkinyl$, $-(CH_2)_n-O-(CH_2)_m-R_7$, $-(CH_2)_n-SH$, $-(CH_2)_n-S-Alkyl$, $-(CH_2)_n-S-Alkenyl$, $-(CH_2)_n-S-Alkinyl$, $-(CH_2)_n-S-(CH_2)_m-R_7$, eine $\alpha$-Kohlenstoff-verknüpfte Seitenkette einer Aminosäure oder eines Aminosäureanalogen,

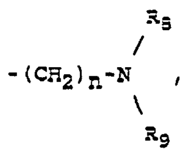, 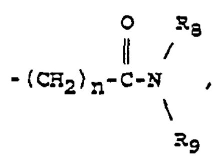, 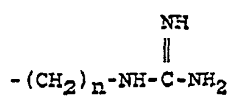, 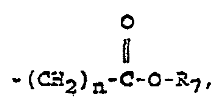,

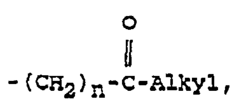, 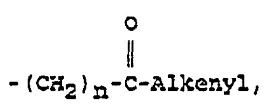, 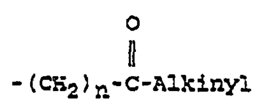

oder

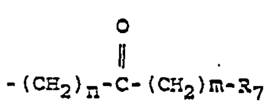

bedeutet; oder

$R_2$ und $R_3$ zusammen einen Ring mit 4 bis 8 Atomen in der Ringstruktur vervollständigen können;

oder vorausgesetzt, dass $R_2$ und $R_3$ nicht zusammen einen Ring bilden, $R_3$ und $R_4$ zusammen einen Ring mit 3 bis 8 Atomen in der Ringstruktur vervollständigen können; und
$R_5$ Halogenmethyl, Trifluormethyl, Amido, Ester, Keton oder Carboxyl bedeutet.

**29.** Verfahren nach Anspruch 28, wobei $R_1$ einen C-terminal verknüpften Aminosäurerest oder Aminosäureanaloges bedeutet und $R_2$ H bedeutet.

**30.** Verfahren nach Anspruch 1, wobei die Peptidbibliothek Verbindungen der allgemeinen Formel IV

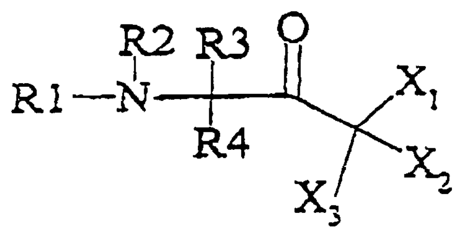

umfasst, wobei

$R_1$ einen C-terminal verknüpften Aminosäurerest oder Aminosäureanaloges oder ein C-terminal verknüpftes Peptid oder Peptidanaloges oder

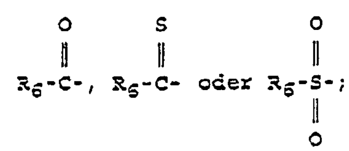

bedeutet;
$R_2$ Wasserstoff, Alkyl, Alkenyl, Alkinyl, $-(CH_2)_m-R_7$,$-(CH_2)_m-OH$, $-(CH_2)_m-O-$Alkyl, $-(CH_2)_m-O-$Alkenyl, $-(CH_2)_m-O-$Alkinyl, $-(CH_2)_m-O-C(=O)-$Alkyl, $-(CH_2)_m-O-C(=O)-$Alkenyl oder $-(CH_2)_m-O-C(=O)-$Alkinyl$-(CH_2)_m-O-C(=O)-(CH_2)_m-R_7$ bedeutet;
$R_3$ und $R_4$ jeweils Wasserstoff, Alkyl, Alkenyl, Alkinyl, $-(CH_2)_m-R_7$, $-(CH_2)_n-OH$, $-(CH_2)_n-O-$Alkyl, $-(CH_2)_n-O-$Alkenyl, $-(CH_2)_n-O-$Alkinyl, $-(CH_2)_n-O-(CH_2)_m-R_7$, $-(CH_2)_n-SH$, $-(CH_2)_n-S-$Alkyl, $-(CH_2)_n-S-$Alkenyl, $-(CH_2)_n-S-$Alkinyl, $-(CH_2)_n-S-(CH_2)_m-R_7$, eine $\alpha$-Kohlenstoff-verknüpfte Seitenkette einer Aminosäure oder eines Aminosäureanalogen,

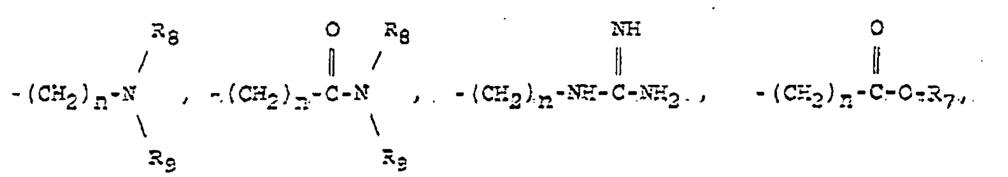

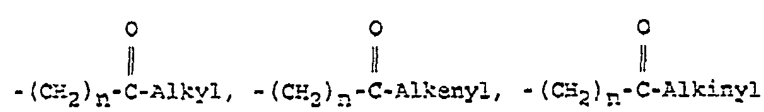

oder

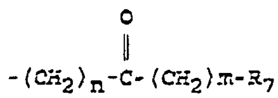

bedeutet; oder

$R_2$ und $R_3$ zusammen einen Ring mit 4 bis 8 Atomen in der Ringstruktur vervollständigen können; oder vorausgesetzt, dass $R_2$ und $R_3$ nicht zusammen einen Ring bilden, $R_3$ und $R_4$ zusammen einen Ring mit 3 bis 8 Atomen in der Ringstruktur vervollständigen können; und

$X_2$, $X_2$ und $X_3$ jeweils Wasserstoff oder Halogen bedeuten.

**31.** Verfahren nach Anspruch 30, wobei $R_1$ einen C-terminal verknüpften Aminosäurerest oder Aminosäureanaloges bedeutet und $R_2$ H bedeutet.

**32.** Verfahren nach Anspruch 30, wobei $X_1$, $X_2$ und $X_3$ jeweils unabhängig voneinander H, Cl, Br oder I bedeuten oder $X_1$, $X_2$ und $X_3$ alle F bedeuten.

**33.** Verfahren nach Anspruch 1, wobei die Bibliothek eine aktive Untereinheit der allgemeinen Formel V

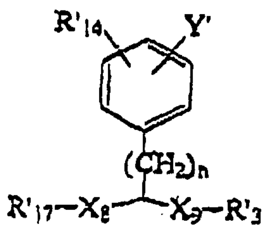

(V)

umfasst, wobei Y' eine Substitution an einer der meta, ortho- oder para-Positionen des Phenylrestes bedeutet, wobei Y' eine Boronogruppe der allgemeinen Formel

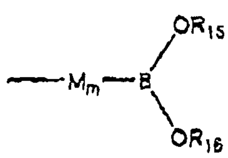

oder eine Phosphonogruppe der allgemeinen Formel

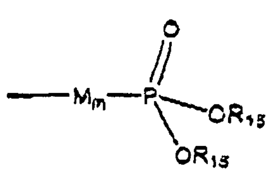

bedeutet,

wobei $R_{15}$ und $R_{16}$ jeweils unabhängig voneinander Wasserstoff, Niederalkyl oder ein pharmazeutisch verträgliches Salz bedeuten oder $R_{15}$ und $R_{16}$ zusammen mit den O-B-O- oder O-P-O-Atomen, an die sie gebunden sind, einen heterocyclischen Ring mit 5 bis 8 Atomen in der Ringstruktur vervollständigen;

$R'_{14}$ abwesend ist oder einen oder mehrere Substituenten an den restlichen Ringpositionen bedeutet, wobei die Substituenten unter Halogen, Niederalkyl, Niederalkoxy, Hydroxyl, Amino, Nitro, Thiol, Amin, Imin, Amid, Carbonyl, Carboxyl, Silyl, Ether, Thioether, Sulfonyl, Selenoether, Keton, Aldehyd, Ester oder $-(CH_2)_m-R_7$, $-CF_3$, -CN oder dergleichen ausgewählt sind;

M (unabhängig für jedes Auftreten) eine substituierte oder unsubstituierte Methylengruppe bedeutet;

$X_8$ und $X_9$ jeweils unabhängig voneinander Methylen, Ethylen, Acetylen, Amin, Carbonyl, Phosphonyl, Schwefel, Sauerstoff oder Selen bedeuten;

$R'_{17}$ abwesend ist oder Wasserstoff, Halogen, Alkyl, Alkenyl, Alkinyl, Hydroxyl, Amino, Nitro, Thiol, Amin, Imin, Amid, Phosphoryl, Phosphonat, Phosphin, Carbonyl, Carboxyl, Silyl, Ether, Thioether, Sulfonyl, Selenoether, Keton, Aldehyd, Ester oder $-(CH_2)_m-R_7$ bedeutet oder $R'_{17}$ einen mit $X_8$ kondensierten Aminosäurerest oder Peptid bedeutet;

$R'_3$ abwesend ist oder Wasserstoff, Halogen, Alkyl, Alkenyl, Alkinyl, Hydroxyl, Amino, Nitro, Thiol, Amin, Imin, Amid, Phosphoryl, Phosphonat, Phosphin, Carbonyl, Carboxyl, Silyl, Ether, Thioether, Sulfonyl, Selenoether, Keton, Aldehyd, Ester oder $-(CH_2)_m-R_7$ bedeutet oder $-R'_3$ einen mit $X_9$ kondensierten Aminosäurerest oder Peptid bedeutet;

$R_7$ Aryl, Cycloalkyl, Cycloalkenyl, Heterocyclyl oder Polycyclyl bedeutet;

m 0 oder eine ganze Zahl im Bereich von 1 bis 8 bedeutet; und

n 1, 2 oder 3 ist.

**34.** Verfahren nach Anspruch 1, wobei die Bibliothek eine aktive Untereinheit mit einem Rest der Formel VI

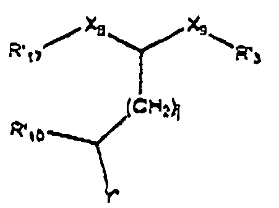

umfasst, wobei Y' eine Substitution an einer der meta-, ortho- oder para-Positionen des Phenylrestes bedeutet, wobei Y' eine Boronogruppe der allgemeinen Formel

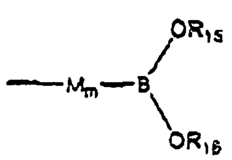

oder eine Phosphonogruppe der allgemeinen Formel

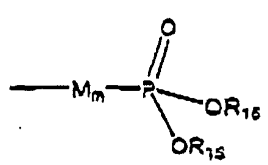

bedeutet,

wobei $R_{15}$ und $R_{16}$ jeweils unabhängig voneinander Wasserstoff, Niederalkyl oder ein pharmazeutisch verträgliches Salz bedeuten oder $R_{15}$ und $R_{16}$ zusammen mit den O-B-O- oder O-P-O-Atomen, an die sie gebunden sind, einen heterocyclischen Ring mit 5 bis 8 Atomen in der Ringstruktur vervollständigen;

$R'_{14}$ abwesend ist oder einen oder mehrere Substituenten an den restlichen Ringpositionen bedeutet, wobei die Substituenten unter Halogen, Niederalkyl, Niederalkoxy, Hydroxyl, Amino, Nitro, Thiol, Amin, Imin, Amid, Carbonyl, Carboxyl, Silyl, Ether, Thioether, Sulfonyl, Selenoether, Keton, Aldehyd, Ester oder $-(CH_2)_m-R_7$, $-CF_3$,

-CN oder dergleichen ausgewählt sind;

M (unabhängig für jedes Auftreten) eine substituierte oder unsubstituierte Methylengruppe bedeutet;

$X_8$ und $X_9$ jeweils unabhängig voneinander Methylen, Ethylen, Acetylen, Amin, Carbonyl, Phosphonyl, Schwefel, Sauerstoff oder Selen bedeuten;

$R'_{17}$ abwesend ist oder Wasserstoff, Halogen, Alkyl, Alkenyl, Alkinyl, Hydroxyl, Amino, Nitro, Thiol, Amin, Imin, Amid, Phosphoryl, Phosphonat, Phosphin, Carbonyl, Carboxyl, Silyl, Ether, Thioether, Sulfonyl, Selenoether, Keton, Aldehyd, Ester oder $-(CH_2)_m-R_7$ bedeutet oder $R'_{17}$ einen mit $X_8$ kondensierten Aminosäurerest oder Peptid bedeutet;

$R'_3$ abwesend ist oder Wasserstoff, Halogen, Alkyl, Alkenyl, Alkinyl, Hydroxyl, Amino, Nitro, Thiol, Amin, Imin, Amid, Phosphoryl, Phosphonat, Phosphin, Carbonyl, Carboxyl, Silyl, Ether, Thioether, Sulfonyl, Selenoether, Keton, Aldehyd, Ester oder $-(CH_2)m-R_7$ oder $R'_3$ einen mit $X_9$ kondensierten Aminosäurerest oder Peptid bedeutet;

$R_7$ Aryl, Cycloalkyl, Cycloalkenyl, Heterocyclyl oder Polycyclyl bedeutet;

m 0 oder eine ganze Zahl im Bereich von 1 bis 8 ist;

j eine ganze Zahl mit einem Wert von 0 bis 3 bedeutet; und

$R'_{10}$ H, OH, $NH_2$, Halogen oder Niederalkyl und vorzugsweise H oder Me bedeutet.

**35.** Verfahren nach Anspruch 34, wobei die aktive Untereinheit irreversibel an das Enzym bindet.

**36.** Verfahren nach einem der Ansprüche 1, 4, 6, 9, 12, 16, 22, 25, 27, 29, 32 oder 33, wobei es sich bei den Mitgliedern der Bibliothek um langsam bildende Inhibitoren des Enzyms handelt.

**37.** Verfahren nach einem der Ansprüche 4, 6, 7, 9 oder 12, wobei Yaa eine eine Boronsäure aufweisende aktive Untereinheit bedeutet.

## Revendications

**1.** Procédé pour la détermination d'un motif préféré de séquence d'acides aminés pour un site actif d'une enzyme, comprenant :

(a) la mise en contact de l'enzyme avec une bibliothèque peptidique dégénérée orientée comprenant une sous-unité active à une position non dégénérée fixée dans des conditions qui permettent à la sous-unité active d'interagir avec le site actif de l'enzyme ;
(b) la réalisation de la liaison de l'enzyme aux peptides à l'intérieur de la bibliothèque peptidique dégénérée orientée ;
(c) la séparation de la population de peptides non liés des complexes peptide/enzyme ;
(d) la libération des peptides liés et de l'enzyme ;
(e) la détermination des séquences d'acides aminés de la population de peptides liés ; et
(f) la détermination d'un motif préféré de séquence d'acides aminés pour un site actif de l'enzyme sur la base de l'abondance relative des différents résidus d'acide aminé pour chaque position dégénérée à l'intérieur de la population de peptides liés.

**2.** Procédé selon la revendication 1, dans lequel la bibliothèque peptidique dégénérée orientée est une bibliothèque peptidique synthétique soluble.

**3.** Procédé selon la revendication 1, dans lequel la bibliothèque peptidique dégénérée orientée est une bibliothèque liée sur un support solide.

**4.** Procédé selon la revendication 1, dans lequel la bibliothèque peptidique dégénérée orientée comprend des peptides comprenant une formule :

$$(Xaa)_n\text{-}Yaa\text{-}(Xaa)_m$$

dans laquelle

Yaa est une sous-unité active capable de former l'un d'un produit d'addition par covalence ou d'un produit d'addition lié étroitement avec une partie d'un site actif d'une enzyme ;

Xaa est un quelconque acide aminé ou analogue d'acide aminé ; et
n et m sont des entiers de 0 à 10 inclus, tels que la somme de m et de n soit d'au moins deux.

**5.** Procédé selon la revendication 1, dans lequel la sous-unité active à la position non dégénérée fixée des peptides de la bibliothèque peptidique dégénérée orientée est l'unique sous-unité active dans les peptides.

**6.** Procédé selon la revendication 1, dans lequel l'enzyme est une protéase et la bibliothèque peptidique dégénérée orientée comprend des peptides comprenant une formule :

$$(Xaa)_n\text{-}Yaa\text{-}(Xaa)_m$$

dans laquelle

Yaa est une sous-unité active comprenant un groupe fonctionnel capable de former l'un d'un produit d'addition par covalence
ou d'un produit d'addition lié étroitement avec un nucléophile ;
Xaa est un quelconque acide aminé ou analogue d'acide aminé ; et
n et m sont des entiers de 0 à 10 inclus, tels que la somme de m et de n soit d'au moins deux.

**7.** Procédé selon la revendication 6, dans lequel Yaa comprend un groupe fonctionnel choisi dans le groupe consistant en groupes fonctionnels aldéhyde, acide boronique, halométhyl cétone, trifluorométhyl cétone, dérivé d'acide alpha-céto carboxylique, et phosphonique.

**8.** Procédé selon la revendication 4, dans lequel l'enzyme est une protéase.

**9.** Procédé selon la revendication 1, dans lequel la bibliothèque peptidique dégénérée orientée comprend des peptides comprenant une formule :

$$Z_1\text{-}(Xaa)p\text{-}Yaa\text{-}(Xaa)ra\text{-}Z_2$$

dans laquelle :

Yaa est une sous-unité active capable de former l'un d'un produit d'addition par covalence ou d'un produit d'addition lié étroitement avec une partie d'un site actif d'une enzyme ;
Xaa est un quelconque acide aminé ou analogue d'acide aminé ;
n et m sont des entiers de 0 à 10 inclus, tels que la somme de m et de n soit d'au moins 2 ;
$Z_1$ est un atome d'hydrogène ou un peptide ayant une formule $(Xaa)_a$ dans laquelle Xaa est un quelconque acide aminé ou analogue d'acide aminé non dégénéré et a est un entier de 1 à 15 inclus ; et
$Z_2$ est un atome d'hydrogène ou un peptide ayant une formule $(Xaa)_b$ dans laquelle Xaa est un quelconque acide aminé ou analogue d'acide aminé non dégénéré et b est un entier de 1 à 15 inclus.

**10.** Procédé selon la revendication 9, dans lequel a et b sont des entiers de 1 à 10 inclus.

**11.** Procédé selon la revendication 9, dans lequel a et b sont des entiers de 1 à 5 inclus.

**12.** Procédé selon la revendication 1, dans lequel la bibliothèque peptidique dégénérée orientée comprend des peptides comprenant une formule :

$$Xaa_1\text{-}[Xaa_1]_n\text{-}Xaa_1\text{-}Yaa$$

dans laquelle :

$Xaa_1$ de façon indépendante pour chaque occurrence, représente un quelconque acide aminé ou analogue d'acide aminé ;
n est un entier de 0 à 20 ; et

Yaa est une sous-unité active capable de former l'un d'un produit d'addition par covalence ou d'un produit d'addition lié étroitement avec une partie d'un site actif d'une enzyme.

**13.** Procédé selon la revendication 12, dans lequel n est un entier compris entre 0 et 10.

**14.** Procédé selon la revendication 1, dans lequel la sous-unité active forme au moins l'un d'une liaison covalente ou d'un complexe lié étroitement avec une partie du site actif de l'enzyme.

**15.** Procédé selon la revendication 1, dans lequel l'enzyme est protéine phosphatase.

**16.** Procédé selon la revendication 1, dans lequel le motif préféré de séquence d'acides aminés pour le site actif de l'enzyme est déterminé par le calcul d'une valeur d'abondance relative (RA) pour chaque résidu d'acide aminé (Xaa) à chaque position dégénérée, où RA est déterminé par la formule :

$$RA = \frac{\text{quantité de Xaa dans la population de peptides sélectionnés /}}{\text{quantité de Xaa dans la bibliothèque peptidique dégénérée orientée}}$$

et la sélection des résidus d'acide aminé qui ont une valeur d'abondance relative supérieure à 1,0 à une position dégénérée pour être inclus en une position correspondant à la position dégénérée dans le motif préféré de séquence d'acides aminés.

**17.** Procédé selon la revendication 1, comprenant de plus la préparation d'un peptidomimétique correspondant audit motif préféré de séquence d'acides aminés déterminé.

**18.** Procédé selon la revendication 1, dans lequel la bibliothèque comprend une sous-unité active ayant une structure de formule I :

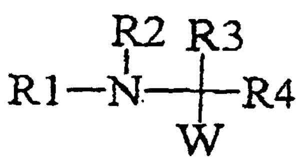

dans laquelle :

W représente un groupe $BY_1Y_2$, perhaloalkyle ou $C(=O)R_5$ ;

$R_1$ représente un résidu d'acide aminé relié par le C-terminal, ou un peptide ou analogue de peptide relié par le C-terminal, ou bien

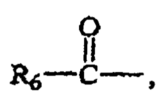, 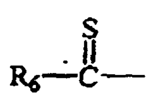, 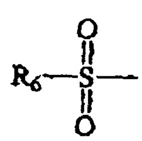 ;

$R_2$ représente un atome d'hydrogène, un groupe alkyle, alcényle, alcynyle, $-(CH_2)_m-R_7$, $-(CH_2)_m-OH$, $-(CH_2)_m$-O-alkyle, $-(CH_2)_m$-O-alcényle, $-(CH_2)_m$-O-alcynyle, $-(CH_2)_m$-O-C(=O)-alkyle, $-(CH_2)_m$-O-C(=O)-alcényle, $-(CH_2)_m$-O-C(=O)-alcynyle, $-(CH_2)_m-O-C(=O)-(CH_2)_m-R_7$ ;

$R_3$ et $R_4$ représentent chacun un atome d'hydrogène, un groupe alkyle, alcényle, alcynyle, $-(CH_2)_m-R_7$, $-(CH_2)_n-OH$, $-(CH_2)_n$-O-alkyle, $-(CH_2)_n$-O-alcényle, $-(CH_2)_n$-O-alcynyle, $-(CH_2)_n-O-(CH_2)_m-R_7$, $-(CH_2)_n-SH$, $-(CH_2)_n$-S-alkyle, $-(CH_2)_n$-S-alcényle, $-(CH_2)_n$-S-alcynyle, $-(CH_2)_n-S-(CH_2)_m-R_7$, une chaîne latérale liée à l'atome de carbone en $\alpha$ d'un acide aminé ou d'un analogue d'acide aminé,

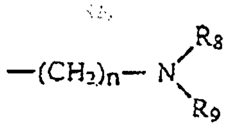 , 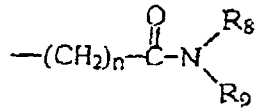 , 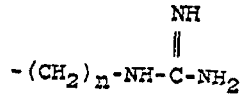 , 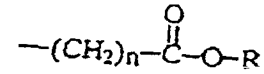

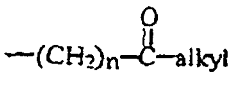 , 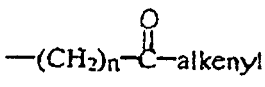 , 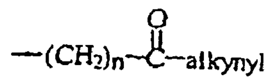 , or 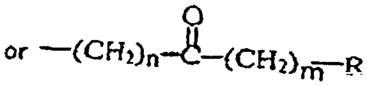

ou, $R_2$ et $R_3$ pris ensemble peuvent compléter un cycle ayant de 4 à 8 atomes dans la structure cyclique,

ou, à condition que $R_2$ et $R_3$ ne soient pas pris ensemble pour former un cycle, $R_3$ et $R_4$ pris ensemble peuvent compléter un cycle ayant de 3 à 8 atomes dans la structure cyclique,

$Y_1$ et $Y_2$ indépendamment ou ensemble sont un groupe hydroxy ou un groupe capable d'être hydrolysé en un groupe hydroxy, y compris des dérivés cycliques dans lesquels $Y_1$ et $Y_2$ sont reliés par l'intermédiaire d'un cycle ayant de 5 à 8 atomes dans la structure cyclique, ou bien l'un de $Y_1$ et $Y_2$ représente une liaison à un groupe amino d'un résidu d'acide aminé,

$R_5$ représente un atome d'hydrogène, un groupe halométhyle, trifluorométhyle, amido, ester, cétone, carboxyle, alkyle, alcényle, alcynyle, $-(CH_2)_m-R_7$, $-(CH_2)_n-OH$, $-(CH_2)_n$-O-alkyle, $-(CH_2)_n$-O-alcényle, $-(CH_2)_n$-O-alcynyle, $-(CH_2)_n-O-(CH_2)_m-R_7$, $-(CH_2)_n-SH$, $-(CH_2)_n$-S-alkyle, $-(CH_2)_n$-S-alcényle, $-(CH_2)_n$-S-alcynyle, $-(CH_2)_n-S-(CH_2)_m-R_7$,$-CH_2O-R_{10}$,

$R_6$ représente un atome d'hydrogène, d'halogène, un groupe alkyle, alcényle, alcynyle, aryle, $-(CH_2)_m-R_7$, $-(CH_2)_m-OH$, $-(CH_2)_m$-O-alkyle, $-(CH_2)_m$-O-alcényle, $-(CH_2)_m$-O-alcynyle, $-(CH_2)_m-O-(CH_2)_m-R_7$, $-(CH_2)_m-SH$, $-(CH_2)_m$-S-alkyle, $-(CH_2)_m$-S-alcényle, $-(CH_2)_m$-S-alcynyle, $-(CH_2)_m-S-(CH_2)_m-R_7$

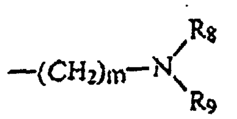 , 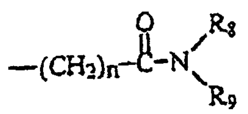 , 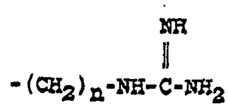 , 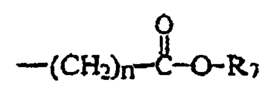

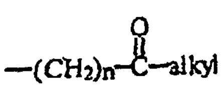 , 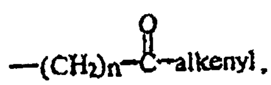 , 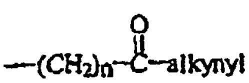 , or 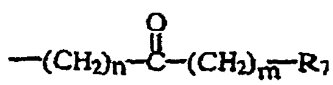

$R_7$ représente un groupe aryle, cycloalkyle, cycloalcényle ou un hétérocycle ;

$R_8$ et $R_9$ représentent chacun, indépendamment un atome d'hydrogène, un groupe alkyle, alcényle, $-(CH_2)_m-R_7$, -C(=O)-alkyle, -C(=O)-alcényle, -C(=O)-alcynyle, $-C(=O)-(CH_2)_m-R_7$,

ou $R_8$ et $R_9$ pris ensemble avec l'atome d'azote auquel ils sont attachés complètent un noyau hétérocyclique ayant de 4 à 8 atomes dans la structure cyclique ;

$R_{10}$ représente un résidu d'acide aminé ou un analogue d'acide aminé relié par le C terminal, ou un peptide ou un analogue de peptide relié par le C terminal, ou :

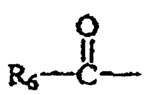 , 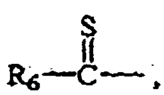 , 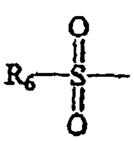

m est zéro ou un entier compris dans la gamme de 1 à 8 ; et

n est un entier compris dans la gamme de 1 à 8.

**19.** Procédé selon la revendication 18, dans lequel W représente un acide boronique, un aldéhyde, un perhaloalkyle ou un perfluoroalkyle, une trifluorométhylcétone, une halométhylcétone, un alpha-cétoester, une alpha-dicétone, un alpha-cétoamide ou un alpha-cétoacide.

**20.** Procédé selon la revendication 18, dans lequel $R_2$ et $R_4$ sont tous deux un atome d'hydrogène, et $R_3$ de façon indépendante pour chaque occurrence dans la bibliothèque de composés, représente une chaîne latérale d'acide aminé d'origine naturelle.

**21.** Procédé selon la revendication 18, dans lequel W représente $BY_1Y_2$ et $Y_1$ et $Y_2$ indépendamment ou ensemble sont un groupe hydroxyle ou un groupe capable d'être hydrolysé en un groupe hydroxyle, comprenant des dérivés cycliques dans lesquels $Y_1$ et $Y_2$ sont reliés par l'intermédiaire d'un cycle ayant de 5 à 8 atomes dans la structure cyclique.

**22.** Procédé selon la revendication 18, dans lequel $R_1$ représente un résidu d'acide aminé ou un analogue d'acide aminé relié par un C-terminal, et $R_2$ représente un atome d'hydrogène.

**23.** Procédé selon la revendication 1, dans lequel la bibliothèque comprend une pluralité de peptides représentés par la formule générale II :

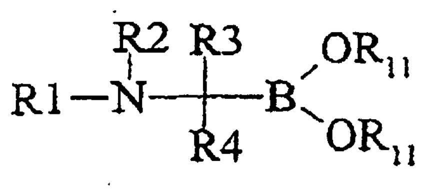

dans laquelle :

$R_1$ représente un résidu d'acide aminé ou un analogue d'acide aminé relié par le C-terminal, ou un peptide ou un analogue de peptide relié par le C-terminal, ou

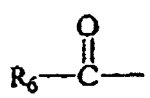 , 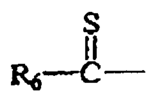 , 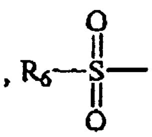

$R_2$ représente un atome d'hydrogène, un groupe alkyle, alcényle, alcynyle, $-(CH_2)_m-R_7$, $-(CH_2)_m-OH$, $-(CH_2)_m-O-alkyle$, $-(CH_2)_m-O-alcényle$, $-(CH_2)_m-O-alcynyle$, $-(CH_2)_m-O-C(=O)-alkyle$, $-(CH_2)_m-O-C(=O)-alcényle$, $-(CH_2)_m-O-C(=O)-alcynyle$, $-(CH_2)_m-O-C(=O)-(CH_2)_m-R_7$ ;

$R_3$ et $R_4$ représentent chacun un atome d'hydrogène, un groupe alkyle, alcényle, alcynyle, $-(CH_2)_m-R_7$, $-(CH_2)_n-OH$, $-(CH_2)_n-O-alkyle$, $-(CH_2)_n-O-alcényle$, $-(CH_2)_n-O-alcynyle$, $-(CH_2)_n-O-(CH_2)_m-R_7$, $-(CH_2)_n-SH$, $-(CH_2)_n-S-alkyle$, $-(CH_2)_n-S-alcényle$, $-(CH_2)_n-S-alcynyle$, $-(CH_2)_n-S-(CH_2)_m-R_7$, une chaîne latérale liée à l'atome de carbone en α d'un acide aminé ou d'un analogue d'acide aminé,

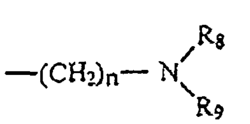 , 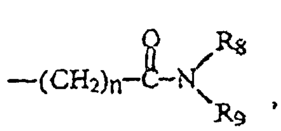 , 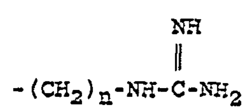 , 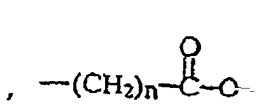

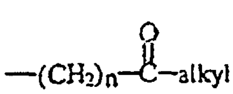 , 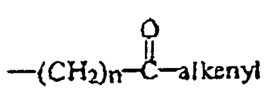 , 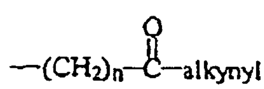 , or 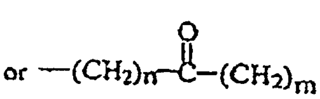

ou, $R_2$ et $R_3$ pris ensemble peuvent compléter un cycle ayant de 4 à 8 atomes dans la structure cyclique, ou, à condition que $R_2$ et $R_3$ ne soient pas pris ensemble pour former un cycle, $R_3$ et $R_4$ pris ensemble peuvent compléter un cycle ayant de 3 à 8 atomes dans la structure cyclique, et

$R_{11}$ de façon indépendante pour chaque occurrence, représente un atome d'hydrogène, un groupe alkyle, ou un sel acceptable du point de vue pharmaceutique,

ou les deux $R_{11}$ pris ensemble avec les atomes O-B-O auxquels ils sont attachés, complètent un noyau hétérocyclique ayant 5 à 8 atomes dans la structure cyclique.

**24.** Procédé selon la revendication 23, dans lequel $R_{11}$ représente un atome d'hydrogène dans les deux occurrences.

**25.** Procédé selon la revendication 23, dans lequel $R_1$ représente un résidu d'acide aminé ou un analogue d'acide aminé relié par le C-terminal et $R_2$ est un atome d'hydrogène.

**26.** Procédé selon la revendication 1, dans lequel la bibliothèque peptidique comprend des peptides représentés par la formule générale III :

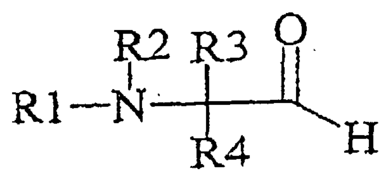

(III)

dans laquelle :

$R_1$ représente un résidu d'acide aminé ou un analogue d'acide aminé relié par le C-terminal, ou un peptide ou un analogue de peptide relié par le C-terminal, ou

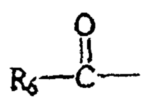, 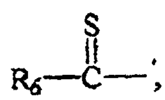; 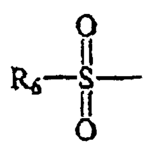

$R_2$ représente un atome d'hydrogène, un groupe alkyle, alcényle, alcynyle, $-(CH_2)_m-R_7$, $-(CH_2)_m-OH$, $-(CH_2)_m$-O-alkyle, $-(CH_2)_m$-O-alcényle, $-(CH_2)_m$-O-alcynyle, $-(CH_2)_m$-O-C(=O)-alkyle, $-(CH_2)_m$-O-C(=O)-alcényle, $-(CH_2)_m$-O-C(=O)-alcynyle, $-(CH_2)_m-O-C(=O)-(CH_2)_m-R_7$ ; et

$R_3$ et $R_4$ représentent chacun un atome d'hydrogène, un groupe alkyle, alcényle, alcynyle, $-(CH_2)_m-R_7$, $-(CH_2)_n-OH$, $-(CH_2)_n$-O-alkyle, $-(CH_2)_n$-O-alcényle, $-(CH_2)_n$-O-alcynyle, $-(CH_2)_n-O-(CH_2)_m-R_7$, $-(CH_2)_n-SH$, $-(CH_2)_n$-S-alkyle, $-(CH_2)_n$-S-alcényle, $-(CH_2)_n$-S-alcynyle, $-(CH_2)_n-S-(CH_2)_m-R_7$, une chaîne latérale liée à l'atome de carbone en α d'un acide aminé ou d'un analogue d'acide aminé,

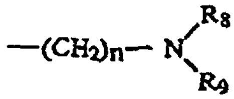, 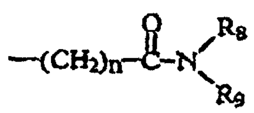, 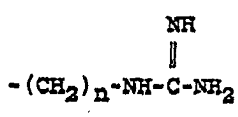, 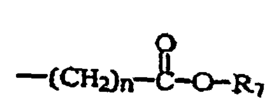

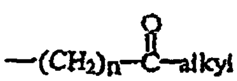, 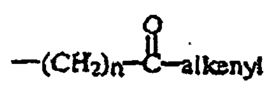, 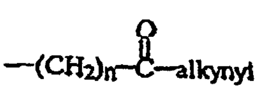, or 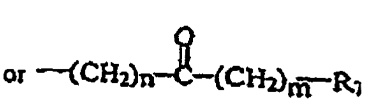

ou, $R_2$ et $R_3$ pris ensemble peuvent compléter un cycle ayant de 4 à 8 atomes dans la structure cyclique, ou, à condition que $R_2$ et $R_3$ ne soient pas pris ensemble pour former un cycle, $R_3$ et $R_4$ pris ensemble peuvent compléter un cycle ayant de 3 à 8 atomes dans la structure cyclique.

**27.** Procédé selon la revendication 26, dans lequel $R_1$ représente un résidu d'acide aminé ou un analogue d'acide aminé relié par le C-terminal et $R_2$ est un atome d'hydrogène.

**28.** Procédé selon la revendication 1, dans lequel la bibliothèque peptidique comprend des peptides représentés par la formule générale IIIa :

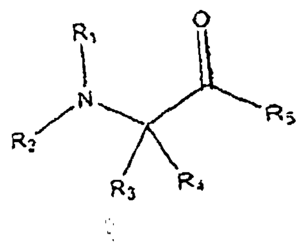

IIIa

dans laquelle:

$R_1$ représente un résidu d'acide aminé ou un analogue d'acide aminé relié par le C-terminal, ou un peptide ou un analogue de peptide relié par le C-terminal, ou

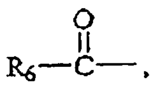, 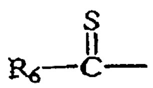, 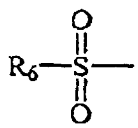

$R_2$ représente un atome d'hydrogène, un groupe alkyle, alcényle, alcynyle, $-(CH_2)_m-R_7$, $-(CH_2)_m-OH$, $-(CH_2)_m-O-$alkyle, $-(CH_2)_m-O-$alcényle, $-(CH_2)_m-O-$alcynyle, $-(CH_2)_m-O-C(=O)-$alkyle, $-(CH_2)_m-O-C(=O)-$alcényle, $-(CH_2)_m-O-C(=O)-$alcynyle, $-(CH_2)_m-O-C(=O)-(CH_2)_m-R_7$ ;

$R_3$ et $R_4$ représentent chacun un atome d'hydrogène, un groupe alkyle, alcényle, alcynyle, $-(CH_2)_m-R_7$, $-(CH_2)_n-OH$, $-(CH_2)_n-O-$alkyle, $-(CH_2)_n-O-$alcényle, $-(CH_2)_n-O-$alcynyle, $-(CH_2)_n-O-(CH_2)_m-R_7$, $-(CH_2)_n-SH$, $-(CH_2)_n-S-$alkyle, $-(CH_2)_n-S-$alcényle, $-(CH_2)_n-S-$alcynyle, $-(CH_2)_n-S-(CH_2)_m-R_7$, une chaîne latérale liée à l'atome de carbone en $\alpha$ d'un acide aminé ou d'un analogue d'acide aminé,

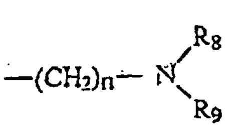, 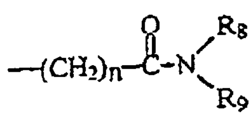, 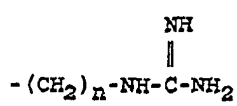, 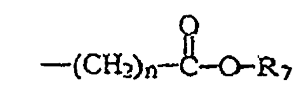

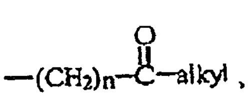, 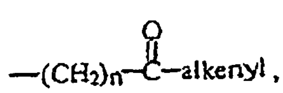, 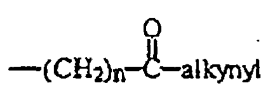, or 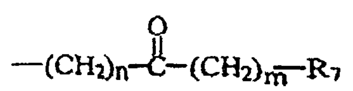

ou, $R_2$ et $R_3$ pris ensemble peuvent compléter un cycle ayant de 4 à 8 atomes dans la structure cyclique,

ou, à condition que $R_2$ et $R_3$ ne soient pas pris ensemble pour former un cycle, $R_3$ et $R_4$ pris ensemble peuvent compléter un cycle ayant de 3 à 8 atomes dans la structure cyclique ; et

$R_5$ représente un groupe halométhyle, trifluorométhyle, amido, ester, cétone ou carboxyle.

**29.** Procédé selon la revendication 28, dans lequel $R_1$ représente un résidu d'acide aminé ou un analogue d'acide aminé relié par le C-terminal et $R_2$ est un atome d'hydrogène.

**30.** Procédé selon la revendication 1, dans lequel la bibliothèque peptidique comprend des peptides représentés par la formule générale IV :

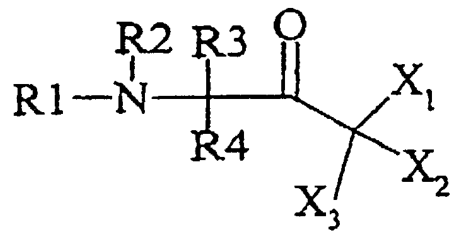

dans laquelle :

$R_1$ représente un résidu d'acide aminé ou un analogue d'acide aminé relié par le C-terminal, ou un peptide ou un analogue de peptide relié par le C-terminal, ou

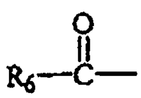 , 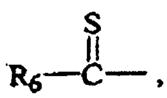 , 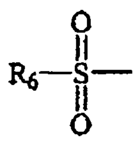

$R_2$ représente un atome d'hydrogène, un groupe alkyle, alcényle, alcynyle, $-(CH_2)_m-R_7$, $-(CH_2)_m-OH$, $-(CH_2)_m$-O-alkyle, $-(CH_2)_m$-O-alcényle, $-(CH_2)_m$-O-alcynyle, $-(CH_2)_m$-O-C(=O)-alkyle, $-(CH_2)_m$-O-C(=O)-alcényle, $-(CH_2)_m$-O-C(=O)-alcynyle, $-(CH_2)_m-O-C(=O)-(CH_2)_m-R_7$ ;

$R_3$ et $R_4$ représentent chacun un atome d'hydrogène, un groupe alkyle, alcényle, alcynyle, $-(CH_2)_m-R_7$, $-(CH_2)_n-OH$, $-(CH_2)_n$-O-alkyle, $-(CH_2)_n$-O-alcényle, $-(CH_2)_n$-O-alcynyle, $-(CH_2)_n-O-(CH_2)_m-R_7$, $-(CH_2)_n-SH$, $-(CH_2)_n$-S-alkyle, $-(CH_2)_n$-S-alcényle, $-(CH_2)_n$-S-alcynyle, $-(CH_2)_n-S-(CH_2)_m-R_7$, une chaîne latérale liée à l'atome de carbone en α d'un acide aminé ou d'un analogue d'acide aminé,

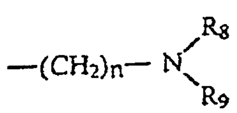 , 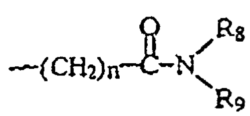 , 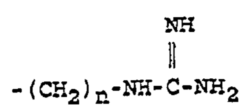 , 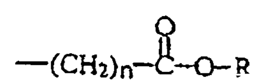

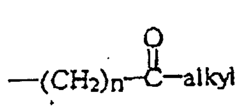 , 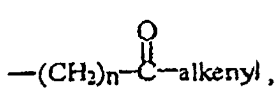 , 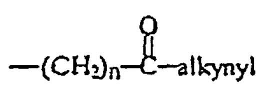 , or 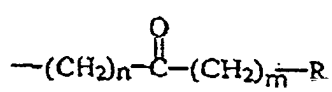

ou, $R_2$ et $R_3$ pris ensemble peuvent compléter un cycle ayant de 4 à 8 atomes dans la structure cyclique,

ou, à condition que $R_2$ et $R_3$ ne soient pas pris ensemble pour former un cycle, $R_3$ et $R_4$ pris ensemble peuvent compléter un cycle ayant de 3 à 8 atomes dans la structure cyclique ; et

$X_1$, $X_2$ et $X_3$ représentent chacun un atome d'hydrogène ou d'halogène.

**31.** Procédé selon la revendication 30, aans lequel $R_1$ représente un résidu d'acide aminé ou un analogue d'acide aminé relié par le C-terminal et $R_2$ est un atome d'hydrogène.

**32.** Procédé selon la revendication 30, dans lequel $X_1$, $X_2$ et $X_3$ indépendamment, représentent chacun H, C1, Br ou I ou bien $X_1$, $X_2$ et $X_3$ représentent tous un atome de fluor.

**33.** Procédé selon la revendication 1, dans lequel la bibliothèque comprend une sous-unité active représentée par la formule générale V :

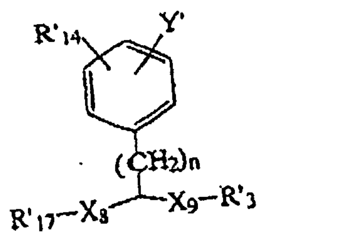

(V)

dans laquelle :

Y' représente une substitution à l'une des positions méta, ortho ou para de la partie phényle, Y' étant un groupe borono représenté par la formule générale :

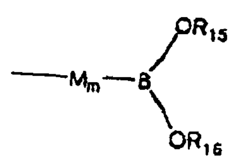

ou un groupe phosphono, représenté par la formule générale :

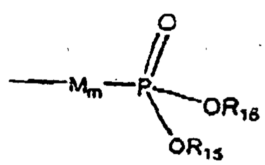

$R_{15}$ et $R_{16}$ représentent chacun indépendamment un atome d'hydrogène, un groupe alkyle inférieur ou un sel acceptable du point de vue pharmaceutique, ou bien $R_{15}$ et $R_{16}$ pris ensemble avec les atomes O-B-O ou O-P-O auxquels ils sont attachés, complètent un noyau hétérocyclique ayant de 5 à 8 atomes dans la structure cyclique ;

$R'_{14}$ est absent ou représente un ou plusieurs substituants aux positions restantes sur le cycle, lesquels substituants sont choisis parmi des atomes d'halogène, des groupes alkyle inférieur, alcoxy inférieur, hydroxyle, amino, nitro, thiol, amines, imines, amides, carbonyle, carboxyles, silyles, éthers, thioéthers, sulfonyles, sélénoéthers, cétones, aldéhydes, esters ou $-(CH_2)_m-R_7$, $-CF_3$, -CN ou similaires ;

M de façon indépendante pour chaque occurrence, représente un groupe méthylène substitué ou non substitué ;

$X_8$ et $X_9$ représentent chacun, indépendamment, un groupe méthylène, éthylène, acétylène, amine, carbonyle, phosphonyle, un atome de soufre, d'oxygène ou de sélénium ;

$R'_{17}$ est absent ou représente un atome d'hydrogène, d'halogène, des groupes alkyle, alcényle, alcynyle, hydroxyle, amino, nitro, thiol, amines, imines, amides, phosphoryles, phosphonates, phosphines, carbonyles, carboxyles, silyles, éthers, thioéthers, sulfonyles, sélénoéthers, cétones, aldéhydes, esters ou - $(CH_2)_m-R_7$, ou bien $R'_{17}$ représente un résidu d'acide aminé ou un peptide condensé avec $X_8$ ;

$R'_3$ est absent ou représente un atome d'hydrogène, d'halogène, des groupes alkyle, alcényle, alcynyle, hydroxyle, amino, nitro, thiol, amines, imines, amides, phosphoryle, phosphonates, phosphines, carbonyles, carboxyles, silyles, éthers, thioéthers, sulfonyles, sélénoéthers, cétones, aldéhydes, esters ou - $(CH_2)_m$-$R_7$, ou bien $R'_3$ représente un résidu d'acide aminé ou un peptide condensé avec $X_9$ ;

$R_7$ représente un groupe aryle, cycloalkyle, cycloalcényle, un hétérocycle ou un polycycle ;

m est zéro ou un entier compris dans la gamme de 1 à 8 ; et

n est 1, 2 ou 3.

**34.** Procédé selon la revendication 1, dans lequel la bibliothèque comprend une sous-unité active ayant un résidu de formule VI :

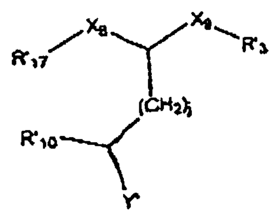

dans laquelle :

Y' représente une substitution à l'une des positions méta, ortho ou para de la partie phényle, Y' étant un groupe borono représenté par la formule générale :'

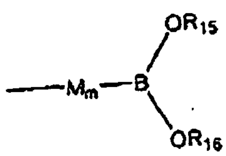

ou un groupe phosphono, représenté par la formule générale :

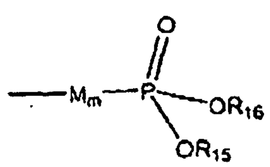

$R_{15}$ et $R_{16}$ représentent chacun, indépendamment, un atome d'hydrogène, un groupe alkyle inférieur ou un sel acceptable du point de vue pharmaceutique, ou bien $R_{15}$ et $R_{16}$ pris ensemble avec les atomes O-B-O ou O-P-O auxquels ils sont attachés complètent un noyau hétérocyclique ayant de 5 à 8 atomes dans la structure cyclique ;

$R'_{14}$ est absent ou représente un ou plusieurs substituants aux positions restantes sur le cycle, lesquels substituants sont choisis parmi des atomes d'halogène, des groupes alkyle inférieur, alcoxy inférieur, hydroxyle, amino, nitro, thiol, amines, imines, amides, carbonyles, carboxyles, silyles, éthers, thioéthers, sulfonyles, sélénoéthers, cétones, aldéhydes, esters ou -$(CH_2)_m$-$R_7$, -$CF_3$, -CN ou similaires ;

M de façon indépendante pour chaque occurrence, représente un groupe méthylène substitué ou non substitué ;

$X_8$ et $X_9$ représentent chacun, indépendamment, un groupe méthylène, éthylène, acétylène, amine, carbonyle, phosphonyle, un atome de soufre, d'oxygène ou de sélénium ;

$R'_{17}$ est absent ou représente un atome d'hydrogène, d'halogène, des groupes alkyle, alcényle, alcynyle, hydroxyle, amino, nitro, thiol, amines, imines, amides, phosphoryles, phosphonates, phosphines, carbonyles, carboxyles, silyles, éthers, thioéthers, sulfonyles, sélénoéthers, cétones, aldéhydes, esters ou - $(CH_2)_m$-$R_7$, ou $R'_{17}$ représente un résidu d'acide aminé ou un peptide condensé avec $X_8$ ;

| | |
|---|---|
| $R'_3$ | est absent ou représente un atome d'hydrogène, d'halogène, des groupes alkyle, alcényle, alcynyle, hydroxyle, amino, nitro, thiol, amines, imines, amides, phosphoryles, phosphonates, phosphines, carbonyles, carboxyles, silyles, éthers, thioéther, sulfonyles, sélénoéthers, cétones, aldéhydes, esters ou $-(CH_2)_m-R_7$, ou bien $R'_3$ représente un résidu d'acide aminé ou un peptide condensé avec $X_9$ ; |
| $R_7$ | représente un groupe aryle, cycloalkyle, cycloalcényle, un hétérocycle ou un polycycle ; |
| m | est zéro ou un entier compris dans la gamme de 1 à 8 ; |
| j | est un entier de 0 à 3, et |
| $R'_{10}$ | est H, OH, $NH_2$, halogène ou alkyle inférieur, de préférence H ou Me. |

**35.** Procédé selon la revendication 34, dans lequel la sous-unité active se lie de façon irréversible à l'enzyme.

**36.** Procédé selon l'une quelconque des revendications 1, 4, 6, 9, 12, 16, 22, 25, 27, 29, 32 ou 33, dans lequel des membres de la bibliothèque sont des inhibiteurs à liaison lente de l'enzyme.

**37.** Procédé selon l'une quelconque des revendications 4, 6, 7, 9 ou 12, dans lequel Yaa représente une sous-unité active portant un acide boronique.

*Figure 1* .